# EUROPEAN PATENT APPLICATION

(11) **EP 4 186 917 A1**
(43) Date of publication of application: **31.05.2023**
(21) Application number: 21211038.1
(22) Date of filing: 29.11.2021
(51) Int. Cl.: C07K 14/415, C12N 15/82

(54) **TOBAMOVIRUS RESISTANT PLANTS**

(71) Applicant: Keygene N.V., 6700 AE Wageningen (NL)
(72) Inventor: DIERGAARDE, Paul Johan, 6700 AE Wageningen (NL); EVRARD, Alexandre, 6700 AE Wageningen (NL); LEDERER, Julie, 6700 AE Wageningen (NL); PRINS, Marinus Willem, 6700 AE Wageningen (NL); DE VOS, Martin, 6700 AE Wageningen (NL)
(74) Representative: Nederlandsch Octrooibureau

(57) **Abstract**

The invention is in the field of agriculture, in particular in the field of crop protection, more particularly in the field of providing tobamovirus resistance to plants. Provided is a nucleic acid and protein A that are capable of converting tobamovirus resistance to a plant, the use of such nucleic acid and protein in converting tobamovirus rsistance, a method for producing a plant having improved tobamovirus resistance, as well as a plant produced by such method.

## Description

### FIELD OF THE INVENTION

The invention is in the field of agriculture, in particular in the field of crop protection, more particularly in the field of providing tobamovirus resistance, preferably ToBRFV resistance, to plants.

### BACKGROUND OF THE INVENTION

The genus *Tobamovirus* includes over 30 recognized species. Many tobamoviruses are considered to be severe pathogens with often worldwide distributions. Examples of these are tobacco mosaic virus (TMV), tomato mosaic virus (ToMV) and tomato mottle mosaic virus (ToMMV), pepper mild mottle virus (PMMoV) in Solanaceous plants like tobacco, tomato and pepper, whereas cucumber green mottle mosaic virus (CGMMV) is an effective tobamovirus pathogen of cucurbits plants such cucumber, melon, zucchini, etc.

Tobamoviruses are extremely stable viruses and highly effectively transmitted by touch (i.e. mechanically), through humans, animals and insects. These viruses are known to cause up to 30% yield losses in pepper, tobacco, tomato and cucumber. Yield losses in tomato attributed to tobamoviruses have decreased dramatically since the 1960s due to the discovery and deployment of three genetic sources from wild species: the *Tm-1, Tm-2,* and *Tm-2²* R genes, which have effectively controlled resistance to TMV and ToMV infection for many decades. These R genes are considered unicorns in the R gene resistance breeding field, since most genetic resistances of this R gene type are (far) shorter lasting due to rapid virus adaptation due to mutation.

In September 2014, a new tobamovirus was discovered in Israel that was able to break *Tm-2²*-mediated resistance in tomato that had served growers well for over 50 years. The virus was isolated, and sequencing of its genome showed it to be an isolate of tomato brown rugose fruit virus (ToBRFV), a new tobamovirus that had been identified in Jordan in the early 2010s. Previous studies on mutant viruses that cause resistance breaking, including Tm-2-mediated resistance, demonstrated that resistance breaking had resulted from only a few mutations. RNA viruses like Tobamoviruses are notorious for rapid genetic drift, i.e. creating and evolutionary selecting point mutations beyond recognition of 'classical' (R type) dominant resistance genes. The discovery of a novel tobamovirus that appears to effectively break *Tm-2* resistance in practice means that, due to the extremely infectious nature of Tobamoviruses, the once reliable source of resistance to tobamoviruses in tomato must be considered lost for the multibillion tomato growing industry at large.

It is therefore of utmost importance for tomato producers that novel sources of resistance are rapidly identified and introduced into tomato germplasm. Two complementary approaches are typically being explored and exploited by the tomato seed breeding industry: (1) uncover hitherto undescribed R genes potentially resembling the R genes of the *Tm-2* clade (or other clades) that are capable of recognizing particular features of the new ToBRFV virus isolate and consequently lead to hypersensitive response (HR) based resistance; and (2) develop new types of resistance based on loss-of-susceptibility of mutant populations.

Comparative genomic analysis identified twelve potential resistance-breaking mutations in the viral movement protein (MP), the primary target of the related *Tm-2* resistance, and nine in its replicase (Maayan et al. Archives of Virology (2018) 163:1863-1875). This indicates the genomic flexibility of this virus in the MP area, suggesting that the durability of possible new *Tm-2²* variants is difficult to predict and by no means it can be stated that such new sources of resistance will last as long as *Tm-2²* did. Additional sources of plant protection against these new Tobamoviruses are therefore required.

For this purpose a different strategy can be chosen which is based on the discovery of so-called plant susceptibility factors. Such S factors are plant-expressed proteins or RNAs that are exploited by the virus to complete its infection cycle in given plants. S gene products result in a susceptible plant often by interacting with viral proteins and thus being high-jacked for the viral benefit. Specific alterations in a given S gene product leading to altered structure and function or a loss of expression of the S gene all together, which then results in a loss of susceptibility and plants having an increased virus resistance or virus resistant plants. Though less frequent than R genes, such functionally altered examples have been described in natural genetic variation (e.g. Ruffel et al. Journal of General Virology (2006), 87: 2089-2098). Combining S gene based resistance with R gene based resistance furthermore provides the opportunity to create more durable resistance complexes.

Thus, there remains a need in the art to uncover new molecular target(s) (e.g. genes) and their related protein(s), which can be manipulated or altered in a plant to prevent or reduce at least one of the successful invasion, establishment and spread of a Tobamovirus, preferably ToBRFV, in said plant. There is also a need for an alternative or an improved method for conferring Tobamovirus, preferably ToBRFV, resistance to a plant, preferably a method that allows for the efficient generation of plants resistant to Tobamovirus or having an improved Tobamovirus resistance as compared to a control plant, *e.g.,* a wild-type plant.

### SUMMARY OF THE INVENTION

The present invention describes the discovery of one or more S genes of which the products were shown to interact with an essential viral protein: movement protein. Specific alleles of these S genes lead to increased resistance against ToBRFV in tomato and pepper in an R gene independent manner, allowing for crop protection against tobamoviruses, wherein said tobamoviruses preferably are ToBRFV and ToMV, in *Solanaceae* crops such as tomato and pepper and moreover are likely to generate resistance that is longer lasting than resistance based on an R gene alone.

In a first embodiment, the invention provides for a method for producing a plant having improved tobamovirus resistance as compared to a control plant, comprising the step of impairing expression and/or activity of at least one of:
- a DnaA initiator-associating dia A protein, preferably having an amino acid sequence of SEQ ID NO: 1 or a homologue or orthologue thereof;
- a TAF-2 protein, preferably having an amino acid sequence of SEQ ID NO: 2 or a homologue or orthologue thereof;
- an Adenylyl cyclase-associated protein, preferably having an amino acid sequence of SEQ ID NO: 3 or a homologue or orthologue thereof;
- a Phosphoethanolamine N-methyltransferase (PMT) protein, preferably having an amino acid sequence of SEQ ID NO: 4 or a homologue or orthologue thereof;
- a DnaJ domain-containing protein, preferably having an amino acid sequence of SEQ ID NO: 5 or a homologue or orthologue thereof;
- an Elongation factor 1-gamma 1 (eEF1B) protein, preferably having an amino acid sequence of SEQ ID NO: 6 or a homologue or orthologue thereof;
- a Tetratricopeptide repeat (TPR)-like superfamily protein, preferably having an amino acid sequence of SEQ ID NO: 7 or a homologue or orthologue thereof; and
- an XRl1-like protein, preferably having an amino acid sequence of SEQ ID NO: 8 or a homologue or orthologue thereof.

Preferably expression and/or activity of said protein is impaired by modifying an endogenous gene encoding said protein.

The step of impairing expression may comprise the insertion, deletion and/or substitution of at least one nucleotide in the sequence encoding the endogenous protein, resulting in a sequence encoding a modified protein with reduced function.

In addition or alternatively, the step of impairing expression may comprise the insertion, deletion and/or substitution of at least one nucleotide in a transcription regulatory sequence of the gene encoding the endogenous protein, resulting in decreased protein expression.

Preferably, said tobamovirus is ToBRFV.

The method may further comprise the step of regenerating said plant.

Preferably, expression of the protein is impaired at least in the leaves and/or fruits of said plant.

In a further embodiment, the invention provides for a nucleic acid comprising a gene encoding a protein, wherein said gene comprises one or more modifications resulting in impaired expression and/or activity of the encoded protein selected from the group consisting of:
- a DnaA initiator-associating dia A protein, preferably having an amino acid sequence of SEQ ID NO: 1 or a homologue or orthologue thereof;
- a TAF-2 protein, preferably having an amino acid sequence of SEQ ID NO: 2 or a homologue or orthologue thereof;
- an Adenylyl cyclase-associated protein, preferably having an amino acid sequence of SEQ ID NO: 3 or a homologue or orthologue thereof;
- a Phosphoethanolamine N-methyltransferase (PMT) protein, preferably having an amino acid sequence of SEQ ID NO: 4 or a homologue or orthologue thereof;
- a DnaJ domain-containing protein, preferably having an amino acid sequence of SEQ ID NO: 5 or a homologue or orthologue thereof;
- an Elongation factor 1-gamma 1 (eEF1B) protein, preferably having an amino acid sequence of SEQ ID NO: 6 or a homologue or orthologue thereof;
- a Tetratricopeptide repeat (TPR)-like superfamily protein, preferably having an amino acid sequence of SEQ ID NO: 7 or a homologue or orthologue thereof; and
- an XRl1-like protein, preferably having an amino acid sequence of SEQ ID NO: 8 or a homologue or orthologue thereof.

In a further embodiment, the invention provides for a construct, vector or host cell comprising the nucleic acid of the previous embodiment.

In a further embodiment, the invention provides for a protein encoded by a nucleic acid of the invention having one or more modifications in the coding sequence of the gene encompassed in the nucleic acid, wherein said protein is capable of improving tobamovirus resistance when expressed in a plant.

In a further embodiment, the invention provides for a plant obtainable by a method of the first embodiment.

Preferably the plant of the invention has improved tobamovirus resistance as compared to a control plant, wherein said plant shows impaired expression and/or activity of at least one protein selected from the group consisting of:
- a DnaA initiator-associating dia A protein, preferably having an amino acid sequence of SEQ ID NO: 1 or a homologue or orthologue thereof;
- a TAF-2 protein, preferably having an amino acid sequence of SEQ ID NO: 2 or a homologue or orthologue thereof;
- an Adenylyl cyclase-associated protein, preferably having an amino acid sequence of SEQ ID NO: 3 or a homologue or orthologue thereof;
- a Phosphoethanolamine N-methyltransferase (PMT) protein, preferably having an amino acid sequence of SEQ ID NO: 4 or a homologue or orthologue thereof;
- a DnaJ domain-containing protein, preferably having an amino acid sequence of SEQ ID NO: 5 or a homologue or orthologue thereof;
- an Elongation factor 1-gamma 1 (eEF1B) protein, preferably having an amino acid sequence of SEQ ID NO: 6 or a homologue or orthologue thereof;
- a Tetratricopeptide repeat (TPR)-like superfamily protein, preferably having an amino acid sequence of SEQ ID NO: 7 or a homologue or orthologue thereof; and
- an XRl1-like protein, preferably having an amino acid sequence of SEQ ID NO: 8 or a homologue or orthologue thereof.

Preferably, the plant of the invention, or progeny thereof, comprises a nucleic acid, construct, vector or host cell of the invention, or expresses a protein of the invention.

In a further embodiment, the invention provides for a use of a nucleic acid, construct vector and/or host cell of the invention for improving tobamovirus resistance in a plant.

In a further embodiment, the invention provides for a method of screening a plant for tobamovirus resistance, wherein said method comprises the steps of:
- assessing the presence of the nucleic acid, a construct, vector, host cell, or a protein of the invention in said plant; and
- optionally selecting said plant.

### Definitions

Various terms relating to the methods, compositions, uses and other aspects of the present invention are used throughout the specification and claims. Such terms are to be given their ordinary meaning in the art to which the invention pertains, unless otherwise indicated. Other specifically defined terms are to be construed in a manner consistent with the definition provided herein.

It is clear for the skilled person that any methods and materials similar or equivalent to those described herein can be used for practicing the present invention.

Methods of carrying out the conventional techniques used in methods of the invention will be evident to the skilled worker. The practice of conventional techniques in molecular biology, biochemistry, computational chemistry, cell culture, recombinant DNA, bioinformatics, genomics, sequencing and related fields are well-known to those of skill in the art and are discussed, for example, in the following literature references: Sambrook et al. Molecular Cloning. A Laboratory Manual, 4th Edition, Cold Spring Harbor Laboratory Press, Cold Spring Harbor, N. Y., 2012; Ausubel et al.. Current Protocols in Molecular Biology, John Wiley & Sons, New York, 1987 and periodic updates; the series Methods in Enzymology, Academic Press, San Diego and JM Walker, the series Methods in Molecular Biology, Springer Protocols.

The singular terms "a," "an," and "the" include plural referents unless the context clearly dictates otherwise. Thus, for example, reference to "a cell" includes a combination of two or more cells, and the like. The indefinite article "a" or "an" thus usually means "at least one".

"Analogous to" in respect of a domain, sequence or position of a protein, in relation to an indicated domain, sequence or position of a reference protein, is to be understood herein as a domain, sequence or position that aligns to the indicated domain, sequence or position of the reference protein upon alignment of the protein to the reference nucleic acid using alignment algorithms as described herein, such as Needleman Wunsch. "Analogous to" in respect of a domain, sequence or position of a nucleic acid, in relation to an indicated domain, sequence or position of a reference nucleic acid, is to be understood herein as a domain, sequence or position that aligns to the indicated domain, sequence or position of the reference nucleic acid upon alignment of the nucleic acid to the reference nucleic acid using alignment algorithms as described herein, such as Needleman Wunsch.

The term "and/or" refers to a situation wherein one or more of the stated cases may occur, alone or in combination with at least one of the stated cases, up to with all of the stated cases.

As used herein, the term "about" is used to describe and account for small variations. For example, the term can refer to less than or equal to ± (+ or -) 10%, such as less than or equal to ±5%, less than or equal to ±4%, less than or equal to ±3%, less than or equal to ±2%, less than or equal to ±1%, less than or equal to ±0.5%, less than or equal to ±0.1%, or less than or equal to ±0.05%. Additionally, amounts, ratios, and other numerical values are sometimes presented herein in a range format. It is to be understood that such range format is used for convenience and brevity and should be understood flexibly to include numerical values explicitly specified as limits of a range, but also to include all individual numerical values or sub-ranges encompassed within that range as if each numerical value and subrange is explicitly specified. For example, a ratio in the range of about 1 to about 200 should be understood to include the explicitly recited limits of about 1 and about 200, but also to include individual ratios such as about 2, about 3, and about 4, and sub-ranges such as about 10 to about 50, about 20 to about 100, and so forth.

The term "comprising" is construed as being inclusive and open ended, and not exclusive. Specifically, the term and variations thereof mean the specified features, steps or components are included. These terms are not to be interpreted to exclude the presence of other features, steps or components.

The terms "protein" or "polypeptide" are used interchangeably and refer to molecules consisting of a chain of amino acids, without reference to a specific mode of action, size, 3 dimensional structure or origin. A "fragment" or "portion" of a protein may thus still be referred to as a "protein". An "isolated protein" is used to refer to a protein which is no longer in its natural environment, for example *in vitro* or in a recombinant bacterial or plant cell. The protein of the invention may be at least one of a recombinant, synthetic or artificial protein.

"Plant" refers to either the whole plant or to parts of a plant, such as cells, protoplasts, calli, tissue, organs (e.g. embryos pollen, ovules, seeds, gametes, roots, leaves, flowers, flower buds, anthers, fruit, etc.) obtainable from the plant, as well as derivatives of any of these and progeny derived from such a plant by selfing or crossing. Non-limiting examples of plants include crop plants and cultivated plants, such as Affrican eggplant, alliums, artichoke, asparagus, barley, beet, bell pepper, bitter gourd, bladder cherry, bottle gourd, cabbage, canola, carrot, cassava, cauliflower, celery, chicory, common bean, corn salad, cotton, cucumber, eggplant, endive, fennel, gherkin, grape, hot pepper, lettuce, maize, melon, oilseed rape, okra, parsley, parsnip, pepino, pepper, potato, pumpkin, radish, rice, ridge gourd, rocket, rye, snake gourd, sorghum, spinach, sponge gourd, squash, sugar beet, sugar cane, sunflower, tomatillo, tomato, tomato scion, vegetable Brassica, watermelon, wax gourd, wheat and zucchini.

"Plant cell(s)" include protoplasts, gametes, suspension cultures, microspores, pollen grains, etc., either in isolation or within a tissue, organ or organism. The plant cell can e.g. be part of a multicellular structure, such as a callus, meristem, plant organ or an explant.

"Similar conditions" for culturing the plant / plant cells means among other things the use of a similar temperature, humidity, nutrition and light conditions, and similar irrigation and day/night rhythm.

"Sequence identity" is herein defined as a relationship between two or more amino acid (polypeptide or protein) sequences or two or more nucleotide (polynucleotide) sequences, as determined by comparing the sequences. In the art, "identity" also means the degree of sequence relatedness between amino acid or nucleic acid sequences, as the case may be, as determined by the match between strings of such sequences. "Similarity" between two amino acid sequences is determined by comparing the amino acid sequence and its conserved amino acid substitutes of one polypeptide to the sequence of a second polypeptide. "Identity" and "similarity" can be readily calculated by known methods. The percentage sequence identity / similarity can be determined over the full length of the sequence.

"Sequence identity" and "sequence similarity" can be determined by alignment of two amino acid or two nucleotide sequences using global or local alignment algorithms, depending on the length of the two sequences. Sequences of similar lengths are preferably aligned using a global alignment algorithms (e.g. Needleman Wunsch) which aligns the sequences optimally over the entire length, while sequences of substantially different lengths are preferably aligned using a local alignment algorithm (e.g. Smith Waterman). Sequences may then be referred to as "substantially identical" or "essentially similar" when they (when optimally aligned by for example the programs GAP or BESTFIT using default parameters) share at least a certain minimal percentage of sequence identity (as defined below). GAP uses the Needleman and Wunsch global alignment algorithm to align two sequences over their entire length (full length), maximizing the number of matches and minimizing the number of gaps. A global alignment is suitably used to determine sequence identity when the two sequences have similar lengths. Generally, the GAP default parameters are used, with a gap creation penalty = 50 (nucleotides) / 8 (proteins) and gap extension penalty = 3 (nucleotides) / 2 (proteins). For nucleotides the default scoring matrix used is nwsgapdna and for proteins the default scoring matrix is Blosum62 (Henikoff & Henikoff, 1992, PNAS 89, 915-919). Sequence alignments and scores for percentage sequence identity may be determined using computer programs, such as the GCG Wisconsin Package, Version 10.3, available from Accelrys Inc., 9685 Scranton Road, San Diego, CA 92121-3752 USA, or using open source software, such as the program "needle" (using the global Needleman Wunsch algorithm) or "water" (using the local Smith Waterman algorithm) in EmbossWIN version 2.10.0, using the same parameters as for GAP above, or using the default settings (both for 'needle' and for 'water' and both for protein and for DNA alignments, the default Gap opening penalty is 10.0 and the default gap extension penalty is 0.5; default scoring matrices are Blossum62 for proteins and DNAFull for DNA). When sequences have a substantially different overall lengths, local alignments, such as those using the Smith Waterman algorithm, are preferred.

Alternatively percentage similarity or identity may be determined by searching against public databases, using algorithms such as FASTA, BLAST, etc. Thus, the nucleic acid and protein sequences of the present invention can further be used as a "query sequence" to perform a search against public databases to, for example, identify other family members or related sequences. Such searches can be performed using the BLASTn and BLASTx programs (version 2.0) of Altschul, et al. (1990) J. Mol. Biol. 215:403-10. BLAST nucleotide searches can be performed with the NBLAST program, score = 100, wordlength = 12 to obtain nucleotide sequences homologous to nucleic acid molecules of the invention. BLAST protein searches can be performed with the BLASTx program, score = 50, wordlength = 3 to obtain amino acid sequences homologous to protein molecules of the invention. To obtain gapped alignments for comparison purposes, Gapped BLAST can be utilized as described in Altschul et al., (1997) Nucleic Acids Res. 25(17): 3389-3402. When utilizing BLAST and Gapped BLAST programs, the default parameters of the respective programs (e.g., BLASTx and BLASTn) can be used. See the homepage of the National Center for Biotechnology Information at http://www.ncbi.nlm.nih.gov/.

A "nucleic acid" or "polynucleotide" according to the present invention may include any polymer or oligomer of pyrimidine and purine bases, preferably cytosine, thymine, and uracil, and adenine and guanine, respectively (See Albert L. Lehninger, Principles of Biochemistry, at 793-800 (Worth Pub. 1982) which is herein incorporated by reference in its entirety for all purposes). The present invention contemplates any deoxyribonucleotide, ribonucleotide or nucleic acid component, and any chemical variants thereof, such as methylated, hydroxymethylated or glycosylated forms of these bases, and the like. The polymers or oligomers may be heterogeneous or homogenous in composition, and may be isolated from naturally occurring sources or may be artificially or synthetically produced. In addition, the nucleic acids may be DNA (optionally cDNA) or RNA, or a mixture thereof, and may exist permanently or transitionally in single-stranded or double-stranded form, including homoduplex, heteroduplex, and hybrid states. An "isolated nucleic acid" is used to refer to a nucleic acid which is no longer in its natural environment, for example *in vitro* or in a recombinant bacterial or plant cell. The nucleic acid of the invention may be at least one of a recombinant, synthetic or artificial nucleic acid.

The terms "nucleic acid construct", "nucleic acid vector", "vector" and "expression construct" are used interchangeably herein and is herein defined as a man-made nucleic acid molecule resulting from the use of recombinant DNA technology. The terms "nucleic acid construct" and "nucleic acid vector" therefore does not include naturally occurring nucleic acid molecules although a nucleic acid construct may comprise (parts of) naturally occurring nucleic acid molecules. The vector backbone may for example be a binary or superbinary vector (see e.g. U.S. Pat. No. 5,591,616, US 2002138879 and WO 95/06722), a co-integrate vector or a T-DNA vector, as known in the art and as described elsewhere herein, into which a chimeric gene is integrated or, if a suitable transcription regulatory sequence is already present, only a desired nucleic acid (e.g. comprising a coding sequence, an antisense or an inverted repeat sequence) is integrated downstream of the transcription regulatory sequence. Vectors can comprise further genetic elements to facilitate their use in molecular cloning, such as e.g. selectable markers, multiple cloning sites and the like.

The term "gene" means a DNA fragment comprising a region (transcribed region), which is transcribed into an RNA molecule (e.g. an mRNA) in a cell, operably linked to suitable regulatory regions (e.g. a promoter). A gene will usually comprise several operably linked fragments, such as a promoter, a 5' leader sequence, a coding region and a 3' non-translated sequence (3' end) comprising a polyadenylation site.

"Expression of a gene" refers to the process wherein a DNA region which is operably linked to appropriate regulatory regions, particularly a promoter, is transcribed into an RNA, which is biologically active, e.g. a regulatory non-coding RNA or an RNA which is capable of being translated into a biologically active protein or peptide. Expression in relation to a protein or peptide is to be understood herein as the process of gene expression resulting in production of said protein or peptide.

The term "operably linked" refers to a linkage of polynucleotide elements in a functional relationship. A nucleic acid region is "operably linked" when it is placed into a functional relationship with another nucleic acid region. For instance, a promoter, or rather a transcription regulatory sequence, is operably linked to a coding sequence if it affects the transcription of the coding sequence. Operably linked may mean that the DNA sequences being linked are contiguous.

"Promoter" refers to a nucleic acid fragment that functions to control the transcription of one or more nucleic acids. A promoter fragment is preferably located upstream (5') with respect to the direction of transcription of the transcription initiation site of the gene, and is structurally identified by the presence of a binding site for DNA-dependent RNA polymerase, transcription initiation site(s) and can further comprise any other DNA sequences, including, but not limited to transcription factor binding sites, repressor and activator protein binding sites, and any other sequences of nucleotides known to one of skill in the art to act directly or indirectly to regulate the amount of transcription from the promoter.

Optionally, the term "promoter" may also include the 5' UTR region (5' Untranslated Region) (e.g. the promoter may herein include one or more parts upstream of the translation initiation codon of transcribed region, as this region may have a role in regulating transcription and/or translation). A "constitutive" promoter is a promoter that is active in most tissues under most physiological and developmental conditions. An "inducible" promoter is a promoter that is physiologically (e.g. by external application of certain compounds) or developmentally regulated. A "tissue specific" promoter is only active in specific types of tissues or cells.

A "3' UTR" or "3' non-translated sequence" (also often referred to as 3' untranslated region, or 3'end) refers to the nucleic acid sequence found downstream of the coding sequence of a gene, which comprises for example a transcription termination site and (in most, but not all eukaryotic mRNAs) a polyadenylation signal (such as e.g. AAUAAA or variants thereof). After termination of transcription, the mRNA transcript may be cleaved downstream of the polyadenylation signal and a poly(A) tail may be added, which is involved in the transport of the mRNA to the cytoplasm (where translation takes place).

The term "cDNA" means complementary DNA. Complementary DNA is made by reverse transcribing RNA into a complementary DNA sequence. cDNA sequences thus correspond to RNA sequences that are expressed from genes. As mRNA sequences when expressed from the genome can undergo splicing, i.e. introns are spliced out of the mRNA and exons are joined together, before being translated in the cytoplasm into proteins, it is understood that expression of a cDNA means expression of the mRNA that encodes for the cDNA. The cDNA sequence thus may not be identical to the genomic DNA sequence to which it corresponds as cDNA may comprise only the complete open reading frame, consisting of the joined exons, for a protein, whereas the genomic DNA may comprise exons interspersed by intron sequences. Impairment of expression a protein by genetic modification of a gene encoding the protein may thus not only relate to modifying the sequences encoding the protein, but may also involve mutating intronic sequences of the genomic DNA and/or other gene regulatory sequences of that gene, as long as it results in the impairment of gene expression.

The term "regeneration" is herein defined as the formation of a new tissue and/or a new organ from a single plant cell, a callus, an explant, a tissue or from an organ. The regeneration pathway can be somatic embryogenesis or organogenesis Somatic embryogenesis is understood herein as the formation of somatic embryos, which can be grown to regenerate whole plants. Organogenesis is understood herein as the formation of new organs from (undifferentiated) cells. Preferably, the regeneration is at least one of ectopic apical meristem formation, shoot regeneration and root regeneration. The regeneration as defined herein can preferably concern at least *de novo* shoot formation. For example, regeneration can be the regeneration of a(n) (elongated) hypocotyl explant towards a(n) (inflorescence) shoot. Regeneration may further include the formation of a new plant from a single plant cell or from e.g. a callus, an explant, a tissue or an organ. The regeneration process can occur directly from parental tissues or indirectly, e.g. via the formation of a callus.

The term "wild type" as used in the context of the present invention in combination with a protein or nucleic acid means that said protein or nucleic acid consists of an amino acid or nucleotide sequence, respectively, that occurs as a whole in nature and can be isolated from organisms in nature as such, e.g. is not the result of modification techniques such as targeted or random mutagenesis or the like. A wild type protein is expressed in at least a particular developmental stage under particular environmental conditions, e.g. as it occurs in nature.

The term "endogenous" as used in the context of the present invention in combination with a protein or nucleic acid (e.g. gene) means that said protein or nucleic acid originates from the plant. Often an endogenous protein or nucleic acid will be present in its normal genetic context in the plant. In the present invention, an endogenous protein or nucleic acid may be modified in situ (in the plant or plant cell) using standard molecular biology methods, e.g. gene silencing, random mutagenesis or targeted mutagenesis.

The term "DnaA initiator-associating dia A" protein refers to a protein belonging to the family of DnaA initiator-associating dia A proteins, which preferably is, or is a homologue or orthologue of the protein comprising or consisting of the amino acid sequence of SEQ ID NO 1. An example of an orthologous DnaA initiator-associating dia A protein is a protein having the amino acid sequence of SEQ ID NO: 9, see also Table 1 herein. A DnaA initiator-associating dia A gene is to be understood herein as a gene comprising a sequence encoding a DnaA initiator-associating dia A protein.

The term "TAF-2" protein refers to a protein belonging family of TAF-2 proteins, which preferably is, or is a homologue or orthologue of the protein comprising or consisting of the amino acid sequence of SEQ ID NO 2. An example of an orthologous TAF-2 protein is a protein having the amino acid sequence of SEQ ID NO: 10. A TAF-2 gene is to be understood herein as a gene comprising a sequence encoding a TAF-2 protein.

The term "Adenylyl cyclase-associated" protein refers to a protein belonging family of Adenylyl cyclase-associated proteins, which preferably is, or is a homologue or orthologue of the protein comprising or consisting of the amino acid sequence of SEQ ID NO 3. An example of an orthologous Adenylyl cyclase-associated protein is a protein having the amino acid sequence of SEQ ID NO: 11 or SEQ ID NO: 12. An Adenylyl cyclase-associated gene is to be understood herein as a gene comprising a sequence encoding a Adenylyl cyclase-associated protein.

The term "Phosphoethanolamine N-methyltransferase" or "PMT" protein refers to a protein belonging family of PMT proteins, which preferably is, or is a homologue or orthologue of the protein comprising or consisting of the amino acid sequence of SEQ ID NO 4. An example of an orthologous PMT protein is a protein having the amino acid sequence of SEQ ID NO: 13, SEQ ID NO: 14 or SEQ ID NO: 15. A PMT gene is to be understood herein as a gene comprising a sequence encoding a PMT protein.

The term "DnaJ domain-containing" protein refers to a protein belonging family of DnaJ domain-containing proteins, which preferably is, or is a homologue or orthologue of the protein comprising or consisting of the amino acid sequence of SEQ ID NO 5. An example of an orthologous DnaJ domain-containing protein is a protein having the amino acid sequence of SEQ ID NO: 16. A DnaJ domain-containing gene is to be understood herein as a gene comprising a sequence encoding a DnaJ domain-containing protein.

The term "Elongation factor 1-gamma 1" protein or "eEF1B" protein refers to a protein belonging family of eEF1B proteins, which preferably is, or is a homologue or orthologue of the protein comprising or consisting of the amino acid sequence of SEQ ID NO 6. An example of an orthologous eEF1B protein is a protein having the amino acid sequence of SEQ ID NO: 17. An eEF1B gene is to be understood herein as a gene comprising a sequence encoding an eEF1B protein.

The term "Tetratricopeptide repeat (TPR)-like superfamily" protein refers to a protein belonging family of TPR-like superfamily proteins, which preferably is, or is a homologue or orthologue of the protein comprising or consisting of the amino acid sequence of SEQ ID NO 7. An example of an orthologous TPR-like superfamily protein is a protein having the amino acid sequence of SEQ ID NO: 18. A TPR-like superfamily gene is to be understood herein as a gene comprising a sequence encoding a TPR-like superfamily protein.

The term "XRI1-like" protein refers to a protein belonging family of XRl1-like proteins, which preferably is, or is a homologue or orthologue of the protein comprising or consisting of the amino acid sequence of SEQ ID NO 8. An example of an orthologous XRI1-like protein is a protein having the amino acid sequence of SEQ ID NO: 19. A XRI1-like gene is to be understood herein as a gene comprising a sequence encoding a XRI1-like protein.

"Mutagenesis" and/or "modification of a gene or nucleic acid" may be random mutagenesis or targeted mutagenesis resulting in an altered or mutated nucleic acid. Random mutagenesis may be, but is not limited to, chemical mutagenesis and gamma radiation. Non-limiting examples of chemical mutagenesis include, but are not limited to, EMS (ethyl methanesulfonate), MMS (methyl methanesulfonate), NaN3 (sodium azide) D), ENU (N-ethyl-N-nitrosourea), AzaC (azacytidine) and NQO (4-nitroquinoline 1-oxide). Optionally, mutagenesis systems such as TILLING (Targeting Induced Local Lesions IN Genomics; McCallum et al., 2000, Nat Biotech 18:455, and McCallum et al. 2000, Plant Physiol. 123, 439-442, both incorporated herein by reference) may be used to generate plant lines with a modified gene as defined herein. TILLING uses traditional chemical mutagenesis (e.g. EMS mutagenesis) followed by high-throughput screening for mutations. Thus, plants, seeds and tissues comprising a gene having one or more of the desired mutations may be obtained using TILLING. Targeted mutagenesis is mutagenesis that can be designed to alter a specific nucleotide or nucleic acid sequence, such as but not limited to, oligo-directed mutagenesis, RNA-guided endonucleases (e.g. CRISPR-technology), TALENs or Zinc finger technology.

A "control plant" as referred to herein is a plant of the same species and preferably same genetic background as the plant that is, or is a progeny of, a plant (or "putative test plant" or "test plant") that has been subjected to a method as taught herein, i.e. a method for improving tobamovirus resistance, preferably ToBRFV resistance. The control plant preferably comprises a wild type, preferably an endogenous, DnaA initiator-associating dia A gene, TAF-2 protein, Adenylyl cyclase-associated gene, PMTgene, DnaJ domain-containing gene, eEF1B gene, Tetratricopeptide repeat (TPR)-like superfamily gene, or XRl1-like gene, respectively. The control plant is susceptible to tobamovirus, infection, may develop symptoms of tobamovirus infection or has low resistance to tobamovirus infection. Preferably the tobamovirus is ToBRFV, e.g., ToBRFV can successfully invade, establish itself and/or spread in the control plant. Preferably, the control plant only differs from the putative test plant in the protein, nucleic acid and/or vector or construct of the invention. Preferably the control plant is grown under the same conditions as the test plant comprising the protein and/or nucleic acid of the invention.

The term "rootstock" as used herein refers to part of a plant, often an underground part, from which new above-ground growth can be produced. For instance, it may referto a rhizome or underground stem. In grafting, it refers to a plant, sometimes just a stump, which already has an established, healthy root system, onto which a cutting, a bud, or a scion from another plant is grafted.

The term "scion" as used herein refers to part of a plant, often an upper-ground part, that will produce the plant shoots. It will give rise to the plant's leaves, stems, flowers and/or fruits. The scion is typically the top part of a grafted plant.

"Tobamovirus resistance" refers herein to various levels of tobamovirus resistance or tolerance of a plant, including moderate resistance and high resistance or complete resistance to the tobamovirus, wherein said tobamovirus preferably is selected from the group consisting of: ToBRFV, ToMMV, ToMV, TMV and PMMoV, more preferably said virus is ToBRFV. It can be measured and optionally quantified by comparison of disease caused symptoms, preferably foliar and/or fruit symptoms relative to those seen in susceptible control plants when grown under similar conditions, including identical disease pressure. Such disease bioassays can be carried out using known methods such as described in Salem et al. Arch.Virol. 2016; 161 (2), 503-506 and/or as described in the "Protocol for Tests on Distinctness, Uniformity and Stability, Solanum lycopersicum, L.", adopted on April 14, 2021 and entered into force on June 1, 2021 by the Community Plant Variety Office (CPVO-TP/044/4-Rev.5 published on 14/04/2021; Ad.48.1 - 48.3: Resistance to Tomato mosaic virus (ToMV) - Strains 0, 1 and 2). Tobamovirus resistance can also be indirectly measured as higher yield of resistant plants compared to susceptible plants when grown under disease pressure, such as after inoculation with a tobamovirus, preferably with ToBRFV, preferably with a ToBRFV isolate having the sequence of GenBank accession KT383474 (Salem et al. Arch. Virol. (2016) 161:503-506). For instance, seedlings of test and control plants may be inoculated with ToBRFV at 4 weeks after sowing, and ToBRFV caused symptoms may be assessed visually at 2, 4, and 6 weeks after inoculation. Infection can be monitored by ELISA using antibodies detecting ToBRFV proteins and/or qPCR of ToBRFV nucleic acids.

"Tobamovirus caused symptoms" and "disease caused symptoms" can be used interchangeably herein and include any symptoms of disease, such as yellowing, mosaic pattern on leaves, leaf deformation (narrowing, mottling) that are caused by a tobamovirus infection, wherein said tobamovirus preferably is selected from the group consisting of: ToBRFV, ToMMV, ToMV, TMV and PMMoV, preferably said virus is ToBRFV.

"Improved tobamovirus resistance" refers to an increase in tobamovirus resistance of a plant or plant tissue compared to a suitable control plant, wherein said tobamovirus preferably is selected from the group consisting of: ToBRFV, ToMMV, ToMV, TMV and PMMoV, more preferably said virus is ToBRFV. Both a qualitative increase (e.g. from susceptible to resistant) and a quantitative increase are encompassed herein. Also encompassed is both a reduction of disease incidence (percentage of plants becoming infected), a delay in disease onset and/or reduction of disease severity. Preferably, a plant having improved tobamovirus resistance is a plant comprising at least 1%, 2%, 5%, 10%, 15%, 20%, 30%, 50%, 70%, 80%, 90%, or even 100% higher levels of resistance to said tobamovirus than the control plant, using suitable bioassays and/or field assays for assessing disease resistance under similar conditions. For example, a systemic response, preferably detectable by visual inspection of systemic leaves for mosaic, distortion, necrosis, vein banding, deformation and/or a combination thereof, developed in response to tobamovirus infection may be determined in both a plant as taught herein and a control plant, and may be compared, as shown in the Examples section. In addition or alternatively, systemic leaves or a plant as taught herein may be quantitatively analyzed, preferably by quantitative PCR, for the presence of tobamovirus nucleic acids in response to tobamovirus infection and preferably compared to such data of a control plant in response to the same infection.

The term "impairing the expression of a gene" as used herein, refers to a situation wherein the level of RNA and/or protein derived (wherein "derived" in this respect is to be understood as transcribed and/or translated, respectively) from said gene in a modified plant is reduced as compared to the level of said RNA and/or protein that is produced in a suitable control plant (e.g., a wild type plant) under similar conditions. Preferably, expression of a gene is impaired when the level of RNA and/or protein derived from said gene in a plant is at least 1%, 2%, 5%, 10%, 15%, 20%, 30%, 50%, 70%, 80%, 90%, or even 100% lower than the level of RNA and/or protein derived from said gene in the control plant. A decrease of 100% is understood herein that the RNA and/or protein is absent in the modified plant . Alternatively or in addition, expression of a gene is impaired when the level of RNA or protein encoded by said gene in a plant is statistically significantly lower than the level of RNA or protein that is produced in the control plant.

The term "impairing the expression of a protein" as used herein, refers to a situation wherein the level of said protein in a modified plant is reduced as compared to the level of said protein produced in a suitable control plant (e.g., a wild type plant) under similar conditions. Preferably, expression of a protein is impaired when the level of said protein produced in a plant is at least 1%, 2%, 5%, 10%, 15%, 20%, 30%, 50%, 70%, 80%, 90%, or even 100% lower than the level of said protein that is produced in the control plant. Alternatively or in addition, expression of a protein is impaired when the level of said protein produced in a plant is statistically significantly lower than the level of protein that is produced in the control plant. It is understood herein that "impaired expression" may include both e.g. a modification of one or more regulatory elements and/or a modification of the coding sequence. As a non-limiting example, modification of a regulatory element may result in lower transcript levels, while a modification of the coding sequence may result in the complete absence of any transcripts encoding the wild type protein.

The term "reduced activity of a protein" as used herein refers to a situation wherein activity of a protein, preferably the natural (or "wild type") activity, such as for example its ability to bind to a promoter element, to bind to a receptor, to catalyse an enzymatic reaction, to regulate gene expression, etc, is altered, reduced, blocked or inhibited, for instance due to a modification in structure, as compared to the activity of the same protein albeit without said modification, preferably in a plant maintained under similar conditions. Preferably, the activity of a modified protein may be considered to be impaired when the activity of said modified protein produced in a plant is at least 1%, 2%, 5%, 10%, 15%, 20%, 30%, 50%, 70%, 80%, 90%, or even 100% lower than the activity of the same protein without said modification as produced in a control plant. The skilled person will readily be capable of establishing whether or not activity of a protein is impaired.

### DETAILED DESCRIPTION OF THE INVENTION

In an aspect, the invention encompasses a nucleic acid comprising a gene or modified gene, wherein said gene comprises one or more modifications resulting in impaired expression and/or activity of an encoded protein selected from the group consisting of:
- DnaA initiator-associating dia A protein;
- TAF-2 protein;
- Adenylyl cyclase-associated protein;
- Phosphoethanolamine N-methyltransferase (PMT) protein;
- DnaJ domain-containing protein;
- Elongation factor 1-gamma 1 (eEF1B) protein;
- Tetratricopeptide repeat (TPR)-like superfamily protein; and
- XRl1-like protein.

In other words, the invention provides for a nucleic acid comprising a modified gene derived from a gene encoding a protein selected from the group consisting of:
- DnaA initiator-associating dia A protein;
- TAF-2 protein;
- Adenylyl cyclase-associated protein;
- Phosphoethanolamine N-methyltransferase (PMT) protein;
- DnaJ domain-containing protein;
- Elongation factor 1-gamma 1 (eEF1B) protein;
- Tetratricopeptide repeat (TPR)-like superfamily protein; and
- XRl1-like protein,
wherein said modified gene comprises one or more modifications resulting in impaired expression and/or activity of the encoded protein as compared to the originating gene. Preferably, the modified gene is derived from a wild type, preferably endogenous gene, by said one or more modifications.

Hence, the nucleic acid of the invention may also be considered as a nucleic acid comprising a modified wild type gene or a modified endogenous gene. Said modified gene may be a mutant gene. The mutant gene may be a naturally occurring mutant gene or a manmade mutant gene, e.g. obtainable by a technical process, such as, but not limited to, targeted and/or random mutagenesis. The nucleic acid of the invention comprising the modified gene, preferably in the absence of expression of the unmodified gene results in improved tobamovirus resistance of a plant when present in said plant as compared to a control plant comprising the unmodified gene, wherein the unmodified gene preferably is a wild type and/or endogenous gene.

Preferably, the modified gene of the nucleic acid of the invention is derived from a wild type and/or an endogenous gene by a step of genetic modification. Said wild type and/or endogenous gene is preferably a plant gene. The one or more modifications of the wild type and/or endogenous gene may result in impaired expression of the protein encoded by said wild type and/or endogenous gene. The modified gene of the nucleic acid of the invention preferably is a modified endogenous gene, wherein the modified gene shows at least one of a reduced expression and reduced activity of the encoded protein when present in a plant as compared to the endogenous gene in a control plant, preferably under similar conditions. Optionally, the modified gene is obtained from said wild type and/or endogenous gene by deletion, insertion and/or substitution of at least one nucleotide, wherein said deletion, insertion and/or substitution results in a gene with impaired expression and/or decreased activity of the encoded protein. Said modified gene may be obtained via random or targeted mutagenesis. Such modification may be within the coding sequence of said gene, resulting in a modified protein which may be less functional as compared to the protein encoded by the unmodified gene or which is a dysfunctional protein, wherein a dysfunctional protein is to be understood as a protein not being capable of fulfilling the function of the protein encoded by the unmodified gene. Optionally, the modification is an early stop which results in a truncated protein which has a reduced function or may be dysfunctional. Preferably, said stop being present in exon 1 of the sequence encoding the protein. Expression of said modified protein in a plant, preferably in the absence of expression of the unmodified protein encoded by the wild type, preferably endogenous, gene, results in improved tobamovirus resistance of said plant as compared to a control plant expressing the unmodified protein. Optionally, the modification of the coding sequence of the wild type and/ or endogenous gene is the deletion of all or most of the nucleotides of the sequence encoding the protein, resulting in an absence of said protein in the cell. Optionally, the modification of the coding sequence results in the expression of an aberrant mRNA molecule that e.g. is no longer recognized by the translational machinery and degraded prior to translation.

In addition or alternatively, such modification may be in a regulatory sequence, such as the promoter sequence, resulting in impaired or lost expression of the encoded protein.

In addition or alternatively, the modified gene may comprise one or more epigenetic modifications that silence or reduce gene expression.

In an embodiment, said mutant gene is not an *Arabidopsis thaliana* gene, i.e. is not a gene originating from *Arabidopsis thaliana,* preferably is not an *Arabidopsis thaliana* gene comprising a T-DNA insertion.

Preferably, the unmodified, wild type and/or endogenous gene encodes for a protein that is, or is a homologue or orthologue of, a protein having an amino acid sequence of any one of SEQ ID NO: 1, 2, 3, 4, 5, 6, 7 and 8.

Preferably, the modified gene shows an impaired expression and/or activity of the encoded protein as compared to the corresponding (unmodified) endogenous gene under similar and suitable conditions.

The modified gene of the nucleic acid of the invention may be derived by genetic modification from a DnaA initiator-associating dia A gene that comprises any one of:
(a) a sequence encoding a protein of SEQ ID NO: 1,
(b) a CDS of SEQ ID NO: 50; and
(c) a homologue or orthologue of any one of (a) and (b),
wherein preferably, said modified gene shows an impaired expression and/or activity of the encoded protein as compared to the (unmodified) endogenous gene encoding the protein of SEQ ID NO: 1 under similar and suitable conditions, preferably when present in a plant cell of *Solanum lycopersicum.*

The modified gene of the nucleic acid of the invention may be derived by genetic modification from a TAF-2 gene that comprises any one of:
(d) a sequence encoding a protein of SEQ ID NO: 2;
(e) a CDS of SEQ ID NO: 51; and
(f) a homologue or orthologue of any one of (d) and (e),
wherein preferably, said modified gene shows an impaired expression and/or activity of the encoded protein as compared to the (unmodified) endogenous gene encoding the protein of SEQ ID NO: 2 under similar and suitable conditions, preferably when present in a plant cell of *Solanum lycopersicum.*

The modified gene of the nucleic acid of the invention may derived by genetic modification from a Adenylyl cyclase-associated gene that comprises any one of:
(g) a sequence encoding a protein of SEQ ID NO: 3;
(h) a CDS of SEQ ID NO: 52; and
(i) a homologue or orthologue of any one of (g) and (h),
wherein preferably, said modified gene shows an impaired expression and/or activity of the encoded protein as compared to the (unmodified) endogenous gene encoding the protein of SEQ ID NO: 3 under similar and suitable conditions, preferably when present in a plant cell of *Solanum lycopersicum.*

The modified gene of the nucleic acid of the invention may be derived by genetic modification from a PMT gene that comprises any one of:
(j) a sequence encoding a protein of SEQ ID NO: 4;
(k) a CDS of SEQ ID NO: 53; and
(I) a homologue or orthologue of any one of (j) and (k),
wherein preferably, said modified gene shows an impaired expression and/or activity of the encoded protein as compared to the (unmodified) endogenous gene encoding the protein of SEQ ID NO: 4 under similar and suitable conditions, preferably when present in a plant cell of *Solanum lycopersicum.*

The modified gene of the nucleic acid of the invention may be derived by genetic modification from a DnaJ domain-containing gene that comprises any one of:
(m) a sequence encoding a protein of SEQ ID NO: 5;
(n) a CDS of SEQ ID NO: 54; and
(o) a homologue or orthologue of any one of (m) and (n),
wherein preferably, said modified gene shows an impaired expression and/or activity of the encoded protein as compared to the (unmodified) endogenous gene encoding the protein of SEQ ID NO: 5 under similar and suitable conditions, preferably when present in a plant cell of *Solanum lycopersicum.*

The modified gene of the nucleic acid of the invention may be derived by genetic modification from a eEF1B gene that comprises any one of:
(p) a sequence encoding a protein of SEQ ID NO: 6;
(q) a CDS of SEQ ID NO: 55;
(r) a homologue or orthologue of any one of (p) and (q),
wherein preferably, said modified gene shows an impaired expression and/or activity of the encoded protein as compared to the (unmodified) endogenous gene encoding the protein of SEQ ID NO: 6 under similar and suitable conditions, preferably when present in a plant cell of *Solanum lycopersicum.*

The modified gene of the nucleic acid of the invention may be derived by genetic modification from a TRP-like gene that comprises any one of:
(s) a sequence encoding a protein of SEQ ID NO: 7;
(t) a CDS of SEQ ID NO: 56;
(u) a homologue or orthologue of any one of (s) and (t),
wherein preferably, said modified gene shows an impaired expression and/or activity of the encoded protein as compared to the (unmodified) endogenous gene encoding the protein of SEQ ID NO: 7 under similar and suitable conditions, preferably when present in a cell of *Solanum lycopersicum.*

The modified gene of the nucleic acid of the invention may be derived by genetic modification from a XRI1-like gene that comprises any one of:
(v) a sequence encoding a protein of SEQ ID NO: 8;
(w) a CDS of SEQ ID NO: 57;
(x) a homologue or orthologue of any one of (v) and (w).
wherein preferably, said modified gene shows an impaired expression and/or activity of the encoded protein as compared to the (unmodified) endogenous gene encoding the protein of SEQ ID NO: 8 under similar and suitable conditions, preferably when present in a plant cell of *Solanum lycopersicum.*

A homologue or orthologue of a nucleotide sequence preferably has at least 50%, 60%, 70%, 80%, 90%, 95%, 96%, 97%, 98%, or 99% sequence identity to said nucleotide sequence over its whole length. A homologue or orthologue of an amino acid sequence preferably has at least 50%, 60%, 70%, 80%, 90%, 95%, 96%, 97%, 98%, or 99% sequence identity to said amino acid sequence over its whole length. The homologous or orthologous protein preferably has the same or a similar function. Preferably, the modified gene of the nucleic acid of the invention shows at least 50%, 60%, 70%, 80%, 90%, 95%, 96%, 97%, 98%, or 99% sequence identity to the unmodified, wild type and/or endogenous gene were it is derived from.

A modified protein derived from wild type, preferably an endogenous, protein as indicated herein preferably means that said modified protein differs from said wild type, preferably endogenous, protein in that it has one or more amino acid substitutions, insertions and/or deletions, optionally a truncation, resulting in a protein having a reduced function, a dysfunctional protein, and/or a truncated protein.

A modified gene derived from an endogenous gene as indicated herein preferably means that said gene differs from said endogenous gene in that it has one or more modifications, preferably one or more nucleotide substitutions, insertions and/or deletions. The modifications may result in a gene that encodes a protein having a reduced function, a dysfunctional protein and/or truncated protein and/or the modifications may result in a decrease in expression of the encoded gene, for instance by modification of a regulatory sequence such as the promoter sequence of said gene. The modification of the endogenous gene may thus be a modification in the coding region and/or in a non-coding region of the gene, e.g. by modifying the coding sequence, altering a regulatory sequence, and/or introducing or removing a splice (donor / acceptor) site. Optionally, the modification may be a synonymous or a nonsynonymous alteration of a codon.

Preferably, expression and/or activity of the protein of the modified gene is impaired at least in a fruit and/or a leaf of a plant and/or at least in a plant fruit cell and/or a leaf cell, which represents a main area typically infected by tobamovirus and where symptoms (e.g. mosaic, distortion, necrosis, vein banding, deformation and/or a combination thereof) of tobamovirus infection are typically visible or prominent. Optionally, expression and/or activity of the protein is impaired in a fruit and/or a leaf of said plant and/or in a plant fruit cell and/or a leaf cell. Preferably, expression and/or activity of the protein is impaired in all fruits and/or all leafs of said plant and/or in all plant fruit cells and/or all leaf cells. Optionally, expression and/or activity of the protein is not impaired in the root and/or not in the plant root cells. Optionally, expression and/or activity of the protein is impaired only in the fruits and/or leaves of said plant and/or at only in plant fruit cells and/or leaf cells.

In an embodiment, the phenotype of the plant as taught herein is not altered compared to a control plant, with the exception of said plant having an improved tobamovirus resistance compared to said control plant. For instance, yield, reproduction, flowering, growth, development, etc. is not affected in plants subjected to the methods according to the invention compared to a control plant or wild type plant, preferably of the same species.

The nucleic acid of the invention may be comprised within an expression construct or within the genome of a cell, preferably a plant cell. The invention therefore also provides for a construct or vector comprising the nucleic acid as defined herein and/or encoding the protein of the invention. The construct may be an expression construct for expressing the modified nucleic acid of the invention and/or expression of the modified protein of the invention. In addition or alternatively, the invention provides for a construct or vector for silencing the wild type, preferably endogenous, gene, which encodes an non-coding RNA designed to silence said endogenous gene (via RNAi). Expression of said silencing construct or gene silencing construct in a plant results in reduced expression of the endogenous protein in a plant cell, plant tissue and/or plant. The invention therefore also provides for a nucleic acid encoding for an RNAi designed to silence said endogenous gene.

In an embodiment, the silencing construct may express at least one of an miRNA or siRNA targeting the transcript of the endogenous gene. Said siRNA or miRNA may comprise at least 20, 21, 22, 23, 24 or at least 25 contiguous nucleotides. The mature siRNA or miRNA may comprise at least 20, 21, 22, 23, 24 or at least 25 contiguous nucleotides that have at least 95%, 96%, 97%, 98%, 99% or 100% sequence complementarity with a contiguous sequence in the transcript expressed from the endogenous gene. Expression of the miRNA or siRNA is preferably controlled by a suitable promoter for expression in a plant cell. Preferably, the promoter is suitable for expression at least in the fruits and/or leaf cells of a plant. Preferably, the promoter is only active in the fruits and/or leaf cells of a plant

The transcript is preferably the wild type and/or endogenous mRNA molecule, and preferably includes the 3' and 5' untranslated sequence. Hence, the sequence of the non-coding RNA, or non-coding small RNA, can be, partly or completely, complementary to a sequence comprised in the coding sequence or, partly or completely, complementary to a sequence comprised in the 3' or 5' untranslated region of the transcript. Preferably, the siRNA or miRNA can be partly or completely complementary to a sequence comprised in the coding sequence. For example, at least 20, 21, 22, 23, 24 or at least 25 contiguous nucleotides of the non-coding RNA, or non-coding small RNA, molecule has at least 80%, 85%, 90%, 95%, 96%, 97%, 98%, 99% or 100% sequence complementarity with a contiguous sequence of at least 20, 21, 22, 23, 24 or at least 25 contiguous nucleotides of the endogenous transcript, respectively.

The skilled person knows how to design a small non-coding RNA molecule that is capable of downregulating endogenous protein expression using conventional RNAi.

Preferably, within a construct or vector of the invention, the sequence encoding the modified protein of the invention is operably linked to one or more transcription regulatory elements for expression in a cell such as a 5' UTR and 3' UTR, preferably said sequence is operably linked at least to a promoter for expression in plant. Hence preferably, the construct comprises a sequence encoding a modified protein defined herein that is operably linked to a promoter for expression in a cell, such as a bacterial cell or a plant cell. Preferably, the sequence encoding a modified protein of the invention is operably linked to a promoter for expression in a plant cell. Optionally, the promoter is the native promoter of at least one of DnaA initiator-associating dia A protein, TAF-2, Adenylyl cyclase-associated protein, PMT, DnaJ domain-containing protein, eEF1B , TRP-like protein and a XRI1-protein.

The promoter for expression in plant cells can be a constitutive promoter, an inducible promoter or a tissue specific promoter. Preferably, the promoter is a constitutive promoter. The promoter for expression in plant cells is herein understood as a promoter that is active in plants or plant cells, i.e. the promoter has the general capability to drive transcription within a plant or plant cell. Preferably, the promoter is active in at least the fruits and/or leaf cells of a plant. Preferably, the promoter is only active in the fruits and/or leaf cells of a plant.

Preferably, the nucleic acid of the invention is capable of improving tobamovirus resistance of a plant when present in said plant in the absence of its unmodified endogenous counterpart, i.e. the wild type, preferably endogenous, gene wherefrom the nucleic acid of the invention is derived from by modification. Preferably, tobamovirus resistance of the plant comprising the nucleic acid of the invention is improved as compared to a control plant. Preferably, said (test) plant comprises said modified gene homozygously in its genome (i.e. both or all alleles of the endogenous gene have been modified to impair the expression and/or activity of the encoded protein as defined herein) and preferably said (test) plant has an improved tobamovirus resistance as compared to a control plant that only differs from the (test) plant in that said endogenous genes are unmodified, under similar conditions.

In addition or alternatively, gene expression may be silenced by introducing one or more non-coding RNAs (e.g. a miRNA or an siRNA) that are capable of RNA interference or RNAi, i.e. interfering with gene expression into a cell targeting and preferably degrading the transcript.

The nucleic acid of the invention may be DNA, CDS, cDNA or RNA. The nucleic acid can be transiently introduced into the plant cell, e.g. by transient transfection of a plasmid, optionally in combination with impairing or reducing expression, knocking out and/or silencing (e.g. by RNAi) the corresponding endogenous gene of said plant cell. Alternatively or in addition, the nucleic acid can be stably present in the genome of the plant cell. As a non-limiting example, the nucleic acid may be stably integrated into the genome of the plant cell. Alternatively or in addition, the nucleic acid can be a modified wild type nucleic acid, e.g. a wild type and/or endogenous nucleic acid that is modified to have reduced or absence of protein expression or is modified to encode the modified protein of the invention. The nucleic acid may be indicated as a mutant nucleic acid.

Preferably, said nucleic acid is capable of improving tobamovirus strain resistance to a plant.

In an aspect, the invention pertains to a nucleic acid encoding a modified protein as defined herein.

In a further aspect, the invention encompasses the protein or modified protein encoded by a nucleic acid of the first aspect having one or more modifications in the coding sequence of the gene encompassed in the nucleic acid. The modified protein may be a mutant protein. The mutant protein may be a naturally occurring mutant protein or a manmade mutant protein, e.g. obtainable by a technical process, such as, but not limited to, targeted and/or random mutagenesis of the sequence encoding the protein.

Said modified protein may be less functional or dysfunctional as compared to the protein encoded by an unmodified gene. The modified protein is capable of improving tobamovirus resistance when expressed in a plant in the absence of its unmodified counterpart, i.e. the protein encoded by the unmodified endogenous gene. In other words, the invention encompasses a modified protein having a modification that improves tobamovirus resistance when expressed in a plant. Preferably the modified protein is a modified endogenous protein of said plant, which is encoded by a modified endogenous gene. Preferably the modified protein is a modified endogenous protein of said plant. Preferably, tobamovirus resistance of a plant comprising the modified endogenous gene encoding the modified protein of the invention is improved as compared to a control plant not comprising said modified protein. The modification may be one or more amino acid insertions, deletions and/or substitutions. The modification may be a truncation of the protein for instance because of an early stop in the encoded gene.

The protein of the invention may be produced synthetically, or *in vivo* (in cell or in planta) for instance by transcription and translation of a gene or construct as defined herein, optionally comprising a transgene encoding such protein, e.g. a wild type gene modified to encode said protein, or by transcription and translation of an endogenous sequence modified to encode such protein. Preferably, the protein of the invention is derived from a wild type and/or endogenous protein. The expression of the protein of the invention may be controlled by an endogenous promoter, such as, but not limited to, the promoter naturally controlling the expression of the wild type or endogenous protein from which the protein of the invention is derived.

Preferably, the nucleic acid and/or protein of the invention is present in a plant as defined herein. Preferably, the nucleic acid and/or protein of the invention is derived from a wild type, preferably an endogenous, gene and/or protein of said plant.

The invention also relates to a nucleic acid encoding the modified protein of the invention as defined herein.

In a further aspect, the invention provides for a host cell comprising the nucleic acid and/or protein of the invention. Preferably the nucleic acid of the invention is comprised within the genome of said host cell.

Preferably, said host cell is a plant cell. Even more preferably, said host cell is a plant cell that is desired to have an improved tobamovirus resistance, preferably to be tobamovirus resistant. Preferably, all homologous genes within the genome of said plant cell are modified to result in a nucleic acid of the invention. Preferably, the modification results in an impaired expression and/or activity of a protein selected from the group consisting of:
- DnaA initiator-associating dia A protein;
- TAF-2 protein;
- Adenylyl cyclase-associated protein;
- Phosphoethanolamine N-methyltransferase (PMT) protein;
- DnaJ domain-containing protein;
- Elongation factor 1-gamma 1 (eEF1B) protein;
- Tetratricopeptide repeat (TPR)-like superfamily protein; and
- XRl1-like protein.

The modification of the homologous genes does not have to be the same or identical modifications. Preferably, said plant cell comprises the nucleic acid of the invention in its genome homozygously. Said plant cell may be from any plant species. The plant of the invention may be a monocot or dicot, and may be from a plant of any species belonging to the *Solanaceae* family (optionally of the genus *Solanum*)*, Brassicaceae* family, *Cucurbitaceae* family, *Fabaceae* family, *Rosaceae* family and *Poaceae* family. Preferred plant species belong to the *Solanaceae* family (optionally of the genus *Solanum* or *Capsicum*) and *Cucurbitaceae* (optionally of the genus *Cucumis*)*.* Preferably, the plant species is a tomato (*Solanum lycopersicum*)*,* pepper (*Capsicum annuum, Capsicum frutescens or Capsicum baccatum*)*,* aubergine or eggplant (*Solanum melongena*)*,* pepino (*Solanum muricatum*)*,* tabacum (*Nicotiana* species, e.g. *N*. *benthamiana, N*. *plumbaginifolia, N*. *tabacum,* etc.), or a cucumber *(Cucumis sativus).*

In an embodiment, the host cell of the invention is produced by at least one of mutagenesis of transformation of a nucleic acid as defined herein. In an embodiment, the host cell can be a mutagenized or transgenic host cell.

In an embodiment, the host cell of the invention is not, or is not exclusively, obtained by an essentially biological process. Preferably, the host cell of the invention is obtained by a method comprising a technical step. Preferably the host cell of the invention is manmade.

In a further aspect, the invention encompasses a method for producing a plant having improved tobamovirus resistance, wherein said method comprises the step of impairing expression and/or activity of a protein selected from the group consisting of:
- a DnaA initiator-associating dia A protein, preferably having an amino acid sequence of SEQ ID NO: 1 or a homologue or orthologue thereof;
- a TAF-2 protein, preferably having an amino acid sequence of SEQ ID NO: 2 or a homologue or orthologue thereof;
- an Adenylyl cyclase-associated protein, preferably having an amino acid sequence of SEQ ID NO: 3 or a homologue or orthologue thereof;
- a Phosphoethanolamine N-methyltransferase (PMT) protein, preferably having an amino acid sequence of SEQ ID NO: 4 or a homologue or orthologue thereof;
- a DnaJ domain-containing protein, preferably having an amino acid sequence of SEQ ID NO: 5 or homologue or orthologue thereof;
- an Elongation factor 1-gamma 1 (eEF1B) protein, preferably having an amino acid sequence of SEQ ID NO: 6 or a homologue or orthologue thereof;
- a Tetratricopeptide repeat (TPR)-like superfamily protein, preferably having an amino acid sequence of SEQ ID NO: 7 or a homologue or orthologue thereof; and
- an XRl1-like protein, preferably having an amino acid sequence of SEQ ID NO: 8 or a homologue or orthologue thereof.

Preferably said protein is a wild type and/or endogenous protein. As defined herein, said plant may be a plant cell or plant tissue. Impaired expression of the endogenous protein may involve a genetic modification of an endogenous gene as detailed herein. Preferably, all homologous genes within the genome have been modified to impair expression of the endogenous protein as defined herein. Preferably, the modifications results in an impaired expression and/or activity of the encoded protein. The modification of the homologous genes does not have to be the same or identical modifications. Preferably, said genome comprises the nucleic acid of the invention in its genome homozygously. Preferably, said genome is a plant genome.

Optionally expression of the endogenous protein is reduced by silencing or knocking out the endogenous gene. Knocking out the endogenous gene, e.g. by T-DNA insertion or introduction of an early stop in the coding sequence, may result in the expression a dys- or non-functional protein. Silencing an endogenous gene in a plant may be performed by transfecting and/or expressing a gene silencing construct as detailed herein in said plant.

In case the step of impairing expression of the endogenous protein is in a plant cell or plant tissue, the method may further comprise the step of regenerating the plant cell or plant tissue into a plant. The method for producing a plant having improved tobamovirus resistance can also be regarded as a method for improving or conferring tobamovirus resistance to said plant.

Optionally, the method of the invention comprises the step of introducing expression of the modified protein of the invention. Introducing expression of said protein may be achieved by transfection (transient or stable) of the plant by a nucleic acid and/or a construct encoding said protein. Such method may comprises an additional or simultaneous step of reducing or abolishing the expression of its endogenous counterpart. The step of introducing the expression of the protein of the invention may be performed using any conventional means known to the skilled person. Direct transformation of a nucleic acid encoding the protein of the invention into a plant can occur by one of many techniques known to one skilled in the art and the manner selected is not critical to the practice of the invention. Methods for introducing nucleic acids, constructs and expression vectors into plant tissue available to one skilled in the art are varied and will depend on the plant selected. Procedures for transforming a wide variety of plant species are well known and described throughout the literature. A nucleic acid encoding the protein of the invention may be introduced into a plant to introduce expression of the protein. The nucleic acid expressing the protein of the invention can be introduced into a plant using any conventional method known in the art. As a non-limiting example, this can occur by direct transformation methods, such as *Agrobacterium* transformation of plant tissue, microprojectile bombardment, electroporation, transfection or any one of many methods known to one skilled in the art.

Alternatively or in addition, introducing expression of the modified protein of the invention may be achieved by mutating an endogenous gene in a plant, resulting in decreased expression of the endogenously encoded protein. The endogenous coding sequence may be modified by mutagenesis to result in a sequence encoding the modified protein of the invention. Optionally, the modification results in a non-naturally occurring gene, i.e. a gene that does not occur in nature, and optionally the modification results in expression of a non-natural protein, i.e. a protein not occurring in nature.

The expression of the protein of the invention may be controlled by an endogenous promoter, such as, but not limited to the promoter controlling the expression of the endogenous, protein in a control plant. Alternatively or in addition, expression of the protein of the invention may be controlled by a promoter that is not a native promoter, i.e. the promoter sequence is introduced in the plant. Optionally, the method of the invention comprises a step of modifying a regulatory sequence of the gene, such as the promoter sequence resulting in reduced expression of the encoded protein. In such case, expression of the protein of the invention may be controlled by a modified promoter, preferably a modified endogenous promoter, wherein said modification results in reduced expression as compared to the expression level of said protein that is under the control of an unmodified (native) promoter, preferably an unmodified endogenous promoter.

The invention further pertains to a method for improving tobamovirus resistance in a plant as compared to a control plant, comprising treating the plant with one or more compounds that inhibit the activity of the unmodified endogenous protein as defined herein in said plant.

Optionally the plant is a scion. Optionally such scion is part of a chimera or grafted plant that further comprises a rootstock from another plant which may not have the modified gene and/or protein of the invention. Such rootstock may therefore comprise an endogenous, and/or unmodified gene and/or protein.

The plant or plant part (e.g. a scion) of the method of the invention may be a monocot or dicot, and may be from a plant of any species belonging to the *Solanaceae* family (optionaly of the genus *Solanum*)*, Brassicaceae* family, *Cucurbitaceae* family, *Fabaceae* family, *Rosaceae* family and *Poaceae* family. Preferred plant species belong to the *Solanaceae* family (optionally of the genus *Solanum* or *Capsicum*) and *Cucurbitaceae* (optionally of the genus *Cucumis*)*.* Preferably, the plant species is a tomato (*Solanum lycopersicum*)*,* pepper (*Capsicum annuum, Capsicum frutescens or Capsicum baccatum*)*,* aubergine or eggplant (*Solanum melongena*)*,* pepino (*Solanum muricatum*)*,* tabacum *(Nicotiana* species, e.g. *N*. *benthamiana, N*. *plumbaginifolia, N*. *tabacum,* etc.), or a cucumber (*Cucumis sativus*)*.*

The plant may be, or may be obtainable from, the family of *Solanaceae.* The plant may be of the genus *Solanum,* more preferably said plant is a *Solanum lycopersicum* plant. The plant produced by the method of the invention preferably has a modification in a gene selected from the group consisting of:
- DnaA initiator-associating dia A gene;
- TAF-2 gene;
- Adenylyl cyclase-associated gene;
- Phosphoethanolamine N-methyltransferase (PMT) gene;
- DnaJ domain-containing gene;
- Elongation factor 1-gamma 1 (eEF1B) gene;
- Tetratricopeptide repeat (TPR)-like superfamily gene; and
- XRl1-like gene,
   as defined herein, wherein preferably, said
- DnaA initiator-associating dia A gene is a gene that comprises a sequence encoding a protein of SEQ ID NO: 1, a CDS of SEQ ID NO: 50, and/or a homologue or orthologue thereof;
- TAF-2 gene is a gene that comprises a sequence encoding a protein of SEQ ID NO: 2, a CDS of SEQ ID NO: 51, and/or a homologue or orthologue thereof;
- Adenylyl cyclase-associated gene is a gene that comprises a sequence encoding a protein of SEQ ID NO: 3, a CDS of SEQ ID NO: 52, and/or a homologue or orthologue thereof;
- PMT gene is a gene that comprises a sequence encoding a protein of SEQ ID NO: 4, a CDS of SEQ ID NO: 53, and/or a homologue or orthologue thereof;
- DnaJ domain-containing gene is a gene that comprises a sequence encoding a protein of SEQ ID NO: 5, a CDS of SEQ ID NO: 54, and/or a homologue or orthologue thereof;
- eEF1B gene is a gene that comprises a sequence encoding a protein of SEQ ID NO: 6, a CDS of SEQ ID NO: 55, and/or a homologue or orthologue thereof;
- TRP-like gene that is a gene that comprises any one of a sequence encoding a protein of SEQ ID NO: 7; a CDS of SEQ ID NO: 56, and/or a homologue or orthologue thereof;
- XRl1-like gene that is a gene that comprises a sequence encoding a protein of SEQ ID NO: 8, a CDS of SEQ ID NO: 57, and/or a homologue or orthologue thereof;

In addition or alternatively, the plant may be of the genus *Solanum,* more preferably said plant is a *Solanum lycopersicum* plant, and preferably the modified protein of the method of the invention is derived from any one of:
- a DnaA initiator-associating dia A protein that preferably is, or is a homologue or orthologue of, the protein comprising an amino acid sequence of SEQ ID NO: 1;
- a TAF-2 protein that preferably is, or is a homologue or orthologue of, the protein comprising an amino acid sequence of SEQ ID NO: 2;
- an Adenylyl cyclase-associated protein that preferably is, or is a homologue or orthologue of, the protein comprising an amino acid sequence of SEQ ID NO: 3;
- a PMT protein that preferably is, or is a homologue or orthologue of, the protein comprising an amino acid sequence of SEQ ID NO: 4;
- a DnaJ domain-containing protein that preferably is, or is a homologue or orthologue of, the protein comprising an amino acid sequence of SEQ ID NO: 5;
- an eEF1B protein that preferably is, or is a homologue or orthologue of, the protein comprising an amino acid sequence of SEQ ID NO: 6;
- a TRP-like protein that preferably is, or is a homologue or orthologue of, the protein comprising an amino acid sequence of SEQ ID NO: 7;
- a XRI1-like protein that preferably is, or is a homologue or orthologue of, the protein comprising an amino acid sequence of SEQ ID NO: 8.

The plant produced by the method of the invention may be, or may be obtainable from, the family of *Solanaceae* and of the genus *Capsicum,* more preferably said plant is a *Capsicum annuum* plant, and preferably the modified gene of the method of the invention is derived from any one of:
- a DnaA initiator-associating dia A gene that comprises a sequence encoding a protein of SEQ ID NO: 9, a CDS of SEQ ID NO: 58, and/or a homologue or orthologue thereof;
- a TAF-2 gene that comprises a sequence encoding a protein of SEQ ID NO: 10, a CDS of SEQ ID NO: 59, and/or a homologue or orthologue thereof;
- an Adenylyl cyclase-associated gene that comprises a sequence encoding a protein of SEQ ID NO: 11 or SEQ ID NO: 12, a CDS of SEQ ID NO: 60 or SEQ ID NO: 61, and/or a homologue or orthologue thereof;
- a PMT gene that comprises a sequence encoding a protein of SEQ ID NO: 13, SEQ ID NO: 14, or SEQ ID NO: 15, a CDS of SEQ ID NO: 62, SEQ ID NO: 63 or SEQ ID NO: 64, and/or a homologue or orthologue thereof;
- a DnaJ domain-containing gene that comprises a sequence encoding a protein of SEQ ID NO: 16, a CDS of SEQ ID NO: 65, and/or a homologue or orthologue thereof;
- an eEF1B gene that comprises a sequence encoding a protein of SEQ ID NO: 17, a CDS of SEQ ID NO: 66, and/or a homologue or orthologue thereof;
- a TRP-like gene that comprises a sequence encoding a protein of SEQ ID NO: 18, a CDS of SEQ ID NO: 67, and/or a homologue or orthologue thereof;
- a XRl1-like gene that comprises a sequence encoding a protein of SEQ ID NO: 19, a CDS of SEQ ID NO: 68, and/or a homologue or orthologue thereof.

In addition or alternatively, the plant produced by the method of the invention may be, or may be obtainable from, the family of *Solanaceae* and of the genus *Capsicum,* more preferably said plant is a *Capsicum annuum* plant, and preferably the modified protein of the method of the invention is derived from any one of:
- a DnaA initiator-associating dia A protein that preferably is, or is a homologue or orthologue of, the protein comprising an amino acid sequence of SEQ ID NO: 9;
- a TAF-2 protein that preferably is, or is a homologue or orthologue of, the protein comprising an amino acid sequence of SEQ ID NO: 10;
- an Adenylyl cyclase-associated protein that preferably is, or is a homologue or orthologue of, the protein comprising an amino acid sequence of SEQ ID NO: 11 or 12;
- a PMT protein that preferably is, or is a homologue or orthologue of, the protein comprising an amino acid sequence of SEQ ID NO: 13, 14 or 15;
- a DnaJ domain-containing protein that preferably is, or is a homologue or orthologue of, the protein comprising an amino acid sequence of SEQ ID NO: 16;
- an eEF1B protein that preferably is, or is a homologue or orthologue of, the protein comprising an amino acid sequence of SEQ ID NO: 17;
- a TRP-like protein that preferably is, or is a homologue or orthologue of, the protein comprising an amino acid sequence of SEQ ID NO: 18;
- a XRI1-like protein that preferably is, or is a homologue or orthologue of, the protein comprising an amino acid sequence of SEQ ID NO: 19.

Optionally the plant is a scion, preferably a *Solanum lycopersicum* scion or *Capsicum annuum* scion.

A homologue or orthologue of a gene as defined herein preferably has at least 50%, 60%, 70%, 80%, 90%, 95%, 96%, 97%, 98%, or 99% sequence identity to the nucleotide sequence of said gene, over its full length. A homologue or orthologue of a protein as defined herein preferably has the same or similar functionality and at least 50%, 60%, 70%, 80%, 90%, 95%, 96%, 97%, 98%, or 99% sequence identity to said protein, over its full length. The homologous or orthologous protein preferably has the same or a similar function.

Optionally, the method of the invention further comprises a step for transferring the nucleic acid of the invention to offspring of the plant produced by the method of the invention, which may be performed by introgression. Breeding techniques for introgression are well known to one skilled in the art.

Preferably, the method of the invention results in a plant having improved tobamovirus resistance compared to a control plant as defined herein.

The method of the invention may further comprise a step of screening or testing the plant for improved tobamovirus resistance. Any screening or testing method known in the art can be used for screening the plant, such as, but not limited to, the methods described herein. Said screening or testing can be a biological assay by assessing symptoms of tobamovirus infection after inoculation by the tobamovirus. Alternatively or in addition, expression levels of modified and/or unmodified protein at a molecular level (protein or mRNA) may be determined. In addition or alternatively, the presence of a nucleic acid or construct comprising the modified gene of the nucleic acid of the invention and/or encoding the modified protein of the invention may be determined. The person skilled in the art is aware of techniques to determine protein expression levels and/or the presence or absence of a nucleic acid sequence within a plant.

The method for producing a plant of the invention having improved tobamovirus resistance as defined herein may further comprise a step of assessing expression of the protein of the invention and/or detecting the presence of the nucleic acid of the invention in said plant and optionally subsequently selecting said plant.

Expression levels of the protein of the invention can be determined using any conventional method known to the skilled person. Such methods include detecting the transcript (e.g. mRNA) or detecting the protein of the invention. Non-limiting examples for detecting the transcript include e.g. PCR, q-PCR and northern blotting. Non-limiting examples for detecting the presence of the protein of the invention includes e.g. western blotting and mass spectrometry on full polypeptides and peptide digests. The person skilled in the art is also aware of using methods for screening for the presence of the nucleic acid of the invention. The person in the art is well aware of molecular techniques to identify such sequences, e.g. using Sequence Based Genotyping (Hoa T. Truong, A. Marcos Ramos, Feyruz Yalcin, Marjo de Ruiter, Hein J. A. van der Poel, Koen H. J. Huvenaars, René C. J. Hogers, Leonora. J. G. van Enckevort, Antoine Janssen, Nathalie J. van Orsouw, and Michiel J. T. van Eijk. Sequence-Based Genotyping for Marker Discovery and Co-Dominant Scoring in Germplasm and Populations. PLoS One. 2012; 7(5): e37565), oligo-ligation (SNPSelect; René C. J. Hogers, Marjo de Ruiter, Koen H. J. Huvenaars, Hein van der Poel, Antoine Janssen, Michiel J. T. van Eijk, Nathalie J. van Orsouw. SNPSelect: A scalable and flexible targeted sequence-based genotyping solution; PLoS One. 2018; 13(10): e0205577), AFLP (Zabeau,M. and Vos,P. (1993) Selective restriction fragment amplification; a general method for DNA fingerprinting; Vos,P., Hogers,R., Bleeker,M., Reijans,M., van de Lee,T., Hornes,M., Frijters,A., Pot,J., Peleman,J., Kuiper,M. et al. (1995) AFLP: a new technique for DNA fingerprinting. Nucl. Acids Res., 21, 4407-4414), and the like.

As also indicated herein above, the method may further comprise a step of producing progeny of the plant comprising the nucleic acid of the invention and/or expressing the protein of the invention. The method can comprise a further step of producing seeds from the plant expressing the protein of the invention. The method may further comprise growing the seeds into plants that have improved tobamovirus resistance.

In a further aspect, the invention also provides for a method of screening plant for the presence of the nucleic acid of the invention and/or for expression of the protein of the invention. Said method comprises a step of assessing the presence of the nucleic acid of the invention in said plant and/or assessing expression of the protein of the invention in said plant. Optionally the method further comprises a step of selecting said plant, essentially as described herein above. As defined herein, said plant may be a plant cell or plant tissue. In case multiple plants are screened, said method may further comprise a stop identifying, detecting and/or selecting a plant comprising the nucleic acid of the invention and/or expressing the protein of the invention. Therefore, the invention also provides for a method of identifying, detecting and/or selecting a plant comprising the nucleic acid of the invention and/or expressing the protein of the invention. Optionally, said method further comprises a step of phenotyping, i.e. assessing tobamovirus resistance.

In a further aspect, the invention provides for a plant comprising the protein, nucleic acid and/or construct of the invention, and/or a plant obtainable from a method as defined herein. The plant may comprise a mutation in an endogenous genomic gene as defined herein. The plant may comprise a mutation in an endogenous genomic coding sequence of the gene, wherein the mutation results in the impaired expression and/or activity of an unmodified endogenous protein. Preferably, all homologous genes within the genome of said plant are modified to result in a nucleic acid of the invention. Preferably, the modification results in an impaired expression and/or activity of a protein selected from the group consisting of:
- a DnaA initiator-associating dia A protein, preferably having an amino acid sequence of SEQ ID NO: 1 or a homologue or orthologue thereof;
- a TAF-2 protein, preferably having an amino acid sequence of SEQ ID NO: 2 or a homologue or orthologue thereof;
- an Adenylyl cyclase-associated protein, preferably having an amino acid sequence of SEQ ID NO: 3 or a homologue or orthologue thereof;
- a Phosphoethanolamine N-methyltransferase (PMT) protein, preferably having an amino acid sequence of SEQ ID NO: 4 or a homologue or orthologue thereof;
- a DnaJ domain-containing protein, preferably having an amino acid sequence of SEQ ID NO: 5 or homologue or orthologue thereof;
- an Elongation factor 1-gamma 1 (eEF1B) protein, preferably having an amino acid sequence of SEQ ID NO: 6 or a homologue or orthologue thereof;
- a Tetratricopeptide repeat (TPR)-like superfamily protein, preferably having an amino acid sequence of SEQ ID NO: 7 or a homologue or orthologue thereof; and
- an XRI1-like protein, preferably having an amino acid sequence of SEQ ID NO: 8 or a homologue or orthologue thereof.

The modification of the homologous genes does not have to be the same or identical modification. Preferably, said plant comprises the nucleic acid of the invention in its genome homozygously. The plant of the invention may be characterized by a modified protein, optionally a disrupted protein, which shows a decreased or lost function and/or activity. Further, the plant of the invention may be characterized by a reduced or abolished expression of the endogenous protein as defined herein. The plant comprising the modified gene of the nucleic acid of the invention and/or the modified protein of the invention has, or has improved, tobamovirus resistance as compared to a control plant, which can be tested for and/or screened for as indicated herein. As defined herein, the plant may be a plant cell or plant tissue. Optionally the plant cell, tissue or plant of the invention is a leaf, fruit and/or scion.

As a non-limiting example, the conferred or improved tobamovirus resistance can be determined by comparing a control plant with a plant of the invention, under controlled conditions chosen such that in the control plant at least one sign of infection can be observed after a certain period. Such controlled conditions include e.g. infection of infestation of plants with the tobamovirus.

Under the controlled conditions chosen, the control plant shows preferably at least one disease sign (i.e. one symptom) upon tobamovirus infection as known in the art and as exemplified herein. The control plant can show one or more of the symptoms indicated herein, preferably at least two, three, four or five the signs of disease after a certain period of time.

A certain period as used herein is preferably any period prior to the observing at least one, two or three signs of disease in the control plant after inoculation and this period may be dependent on the experimental set up. Such period can be easily determined by the person skilled in the art. Preferably, such period before observing at least one, two or three signs of disease is at least about 2, 3, 4, 5, 10, 20, 30, 35, 40, 45, 50, 60 or about 70 days, preferably at least about 1, 2, 3, 4, 5, 6 or 7 weeks.

Preferably, the plant of the invention will show less, reduced and/or no signs of disease as compared to the control plant after the certain period as defined herein.

Alternatively or in addition, the plant of the invention may show one or more signs of disease as defined herein at a similar or same severity as the control plant, however the one or more signs of disease will be at a later time period as compared to the control plant, e.g. there will be a delay in onset of one or more symptoms of the disease. As a non-limiting example, there can be a delay of at least 1, 2, 3, 4, 5, 6, 7 or 8 weeks as compared to the control plants. The skilled person knows how to select suitable conditions.

When a plant has an improved tobamovirus resistance, it is preferably capable of sustaining a normal growth and/or a normal development when the plant is subjected to tobamovirus infection, which infection would otherwise have resulted in reduced growth and/or reduced development of the plant. Hence, an improved tobamovirus resistance can be determined by comparing plants. As a non-limiting example, one plant of the invention may be compared with one control plant. Alternatively or in addition, a group of plants of the invention may be compared with a group of control plants. Each group can comprise e.g. at least about 2 ,3, 4, 5, 10, 15, 20, 25, 50 or 100 individual plants.

The skilled person is well aware how to select appropriate conditions to determine improved tobamovirus resistance and how to measure signs of infection.

As a non-limiting example, tobamovirus resistance can be measured by visual inspection, as exemplified herein. For instance the number of root-knots (e.g.) can be counted in case of infection with tobamovirus preferably after about 1, 2, 3, 4, 5 or 6 weeks of tobamovirus infection.

The plant may be a transformant, transgenic and/or manmade (non-natural) mutant plant cell, tissue or plant, i.e. not being a wild type or naturally occurring plant cell, tissue or plant as it comprises the modified gene of the invention and/or expresses the modified protein of the invention.

In an embodiment, the plant of the invention is not, or is not exclusively, obtained by an essentially biological process. Preferably, the plant of the invention is obtained by a method comprising a technical step. Preferably, the plant of the invention is manmade.

The plant may be, but is not limited to, any plant from a species belonging to the *Solanaceae* family (optionaly of the genus *Solanum* or *Capsicum*) and *Cucurbitaceae* (optionally of the genus Cucumis). Preferably, the plant is a tomato (*Solanum lycopersicum*)*,* pepper (*Capsicum annuum, Capsicum frutescens or Capsicum baccatum*)*,* aubergine or eggplant (*Solanum melongena*)*,* pepino (*Solanum muricatum*)*,* tabacum (*Nicotiana* species, e.g. *N*. *benthamiana, N*. *plumbaginifolia, N*. *tabacum,* etc.), or a cucumber (*Cucumis sativus*)*.*

Preferably, the plant of the invention and/or of the method of the invention may be a crop plant or a cultivated plant, i.e. plant species which is cultivated and bred by humans. A crop plant may be cultivated for food or feed purposes (e.g. field crops), or for ornamental purposes (e.g. production of flowers for cutting, grasses for lawns, etc.). A crop plant as defined herein also includes plants from which non-food products are harvested, such as oil for fuel, plastic polymers, pharmaceutical products, cork, fibres (such as cotton) and the like. Preferably, the plant part, leaf, fruit, plant cell, seed, and/or scion as taught herein are from a crop plant.

The plant may be, or may be obtainable from, the family of *Solanaceae.* The plant may be of the genus *Solanum,* more preferably said plant is a *Solanum lycopersicum* plant, comprising a modified gene of the invention that is derived from any one of:
- a DnaA initiator-associating dia A gene that preferably comprises a sequence encoding a protein of SEQ ID NO: 1, a CDS of SEQ ID NO: 50, and/or a homologue or orthologue thereof;
- a TAF-2 gene that preferably comprises a sequence encoding a protein of SEQ ID NO: 2, a CDS of SEQ ID NO: 51, and/or a homologue or orthologue thereof;
- an Adenylyl cyclase-associated gene that preferably comprises a sequence encoding a protein of SEQ ID NO: 3, a CDS of SEQ ID NO: 52, and/or a homologue or orthologue thereof;
- a PMT gene that preferably a sequence encoding a protein of SEQ ID NO: 4, a CDS of SEQ ID NO: 53, and/or a homologue or orthologue thereof;
- a DnaJ domain-containing gene that preferably comprises a sequence encoding a protein of SEQ ID NO: 5, a CDS of SEQ ID NO: 54, and/or a homologue or orthologue thereof;
- an eEF1B gene that preferably comprises a sequence encoding a protein of SEQ ID NO: 6, a CDS of SEQ ID NO: 55, and/or a homologue or orthologue thereof;
- a TRP-like gene that preferably comprises any one of a sequence encoding a protein of SEQ ID NO: 7; a CDS of SEQ ID NO: 56, and/or a homologue or orthologue thereof;
- a XRl1-like gene that preferably comprises a sequence encoding a protein of SEQ ID NO: 8, a CDS of SEQ ID NO: 57, and/or a homologue or orthologue thereof;

In addition or alternatively, the plant may be, or may be obtainable from the family of *Solanaceae,* preferably of the genus *Solanum,* more preferably said plant is a Solanum lycopersicum plant, comprising a modified protein of the invention that is derived from any one of:
- a DnaA initiator-associating dia A protein that preferably is, or is a homologue or orthologue of, the protein comprising an amino acid sequence of SEQ ID NO: 1;
- a TAF-2 protein that preferably is, or is a homologue or orthologue of, the protein comprising an amino acid sequence of SEQ ID NO: 2;
- an Adenylyl cyclase-associated protein that preferably is, or is a homologue or orthologue of, the protein comprising an amino acid sequence of SEQ ID NO: 3;
- a PMT protein that preferably is, or is a homologue or orthologue of, the protein comprising an amino acid sequence of SEQ ID NO: 4;
- a DnaJ domain-containing protein that preferably is, or is a homologue or orthologue of, the protein comprising an amino acid sequence of SEQ ID NO: 5;
- an eEF1B protein that preferably is, or is a homologue or orthologue of, the protein comprising an amino acid sequence of SEQ ID NO: 6;
- a TRP-like protein that preferably is, or is a homologue or orthologue of, the protein comprising an amino acid sequence of SEQ ID NO: 7;
- a XRI1-like protein that preferably is, or is a homologue or orthologue of, the protein comprising an amino acid sequence of SEQ ID NO: 8.

The plant may be, or may be obtainable from, the family of *Solanaceae.* The plant may be of the genus *Capsicum,* more preferably said plant is a *Capsicum annuum* plant, comprising a modified gene of the invention that is derived from any one of:
- a DnaA initiator-associating dia A gene that preferably comprises a coding sequence that is, or is a homologue or orthologue of, the coding sequence of SEQ ID NO: 58;
- a TAF-2 gene that preferably comprises a coding sequence that is, or is a homologue or orthologue of, the coding sequence of SEQ ID NO: 59;
- an Adenylyl cyclase-associated gene that preferably comprises a coding sequence that is, or is a homologue or orthologue of, the coding sequence of SEQ ID NO: 60 or 61;
- a PMT gene that preferably comprises a coding sequence that is, or is a homologue or orthologue of, the coding sequence of SEQ ID NO: 62, 63 or 64;
- a DnaJ domain-containing gene that preferably comprises a coding sequence that is, or is a homologue or orthologue of, the coding sequence of SEQ ID NO: 65;
- an eEF1B gene that preferably comprises a coding sequence that is, or is a homologue or orthologue of, the coding sequence of SEQ ID NO: 66;
- a TRP-like gene that preferably comprises a coding sequence that is, or is a homologue or orthologue of, the coding sequence of SEQ ID NO: 67;
- a XRl1-like gene that preferably comprises a coding sequence that is, or is a homologue or orthologue of, the coding sequence of SEQ ID NO: 68;

In addition or alternatively, the plant may be, or may be obtainable from, the family of *Solanaceae.* The plant may be of the genus *Capsicum,* more preferably said plant is a *Capsicum annuum* plant, comprising a modified protein of the invention that is derived from any one of:
- a DnaA initiator-associating dia A protein that preferably is, or is a homologue or orthologue of, the protein comprising an amino acid sequence of SEQ ID NO: 9;
- a TAF-2 protein that preferably is, or is a homologue or orthologue of, the protein comprising an amino acid sequence of SEQ ID NO: 10;
- an Adenylyl cyclase-associated protein that preferably is, or is a homologue or orthologue of, the protein comprising an amino acid sequence of SEQ ID NO: 11 or 12;
- a PMT protein that preferably is, or is a homologue or orthologue of, the protein comprising an amino acid sequence of SEQ ID NO: 13, 14 or 15;
- a DnaJ domain-containing protein that preferably is, or is a homologue or orthologue of, the protein comprising an amino acid sequence of SEQ ID NO: 16;
- an eEF1B protein that preferably is, or is a homologue or orthologue of, the protein comprising an amino acid sequence of SEQ ID NO: 17;
- a TRP-like protein that preferably is, or is a homologue or orthologue of, the protein comprising an amino acid sequence of SEQ ID NO: 18;
- a XRI1-like protein that preferably is, or is a homologue or orthologue of, the protein comprising an amino acid sequence of SEQ ID NO: 19.

A further aspect of the invention pertains to a seed produced by the plant having improved tobamovirus resistance as defined herein and comprising the modified gene and/or modified protein of the invention.

An additional aspect of the invention pertains to plants grown from the seeds or regenerated from the plant cell, comprising the nucleic acid and/or protein of the invention as defined herein.

An additional aspect of the invention described herein pertains to progeny of the plant of the invention, wherein the progeny has improved tobamovirus resistance as specified herein and wherein the progeny comprises the nucleic acid and/or protein of the invention. The progeny may be obtained by selfing or breeding and selection, wherein the selected progeny retains the increased tobamovirus resistance of the parent plant and/or retain the expression of the protein of the invention.

In an aspect, the invention further concerns the use of a nucleic acid, protein, construct, or host cell of the invention for increasing tobamovirus resistance in a plant.

In an aspect, the invention pertains to plant parts and plant products derived from the plant of the invention and/or plant obtained or obtainable by the method of the invention, wherein the plant part and/or plant product comprise the modified gene, modified protein and/or silencing constructs of the invention, or parts thereof. Such plant parts and/or plant products may be seed or fruit and/or products derived therefrom. Such plant parts, plant products may also be non-propagating material.

The present invention has been described above with reference to a number of exemplary embodiments as shown in the drawings. Modifications and alternative implementations of some parts or elements are possible, and are included in the scope of protection as defined in the appended claims.

**Table 1: Description sequences**

| | |
|---|---|
| SEQ ID NO: 1 | DnaA initiator-associating dia A protein (tomato) |
| SEQ ID NO: 2 | TAF-2 protein (tomato) |
| SEQ ID NO: 3 | Adenylyl cyclase-associated protein (tomato) |
| SEQ ID NO: 4 | PMT protein (tomato) |
| SEQ ID NO: 5 | DnaJ domain-containing protein (tomato) |
| SEQ ID NO: 6 | Elongation factor 1-gamma 1 (eEF1B) protein (tomato) |
| SEQ ID NO: 7 | Tetratricopeptide repeat (TPR)-like superfamily protein (tomato) |
| SEQ ID NO: 8 | XRl1-like protein (tomato) |
| SEQ ID NO: 9 | DnaA initiator-associating dia A protein (pepper) |
| SEQ ID NO: 10 | TAF-2 protein (pepper) |
| SEQ ID NO: 11 | Adenylyl cyclase-associated protein (pepper) |
| SEQ ID NO: 12 | Adenylyl cyclase-associated protein (pepper) |
| SEQ ID NO: 13 | PMT protein (pepper) |
| SEQ ID NO: 14 | PMT protein (pepper) |
| SEQ ID NO: 15 | PMT protein (pepper) |
| SEQ ID NO: 16 | DnaJ domain-containing protein (pepper) |
| SEQ ID NO: 17 | Elongation factor 1-gamma 1 (eEF1B) protein (pepper) |
| SEQ ID NO: 18 | TPR-like superfamily protein (pepper) |
| SEQ ID NO: 19 | XRl1-like protein (pepper) |
| SEQ ID NO: 20 | pJL462 plasmid |
| SEQ ID NO: 21 | DnaJ domain-containing protein Oligo for guide Forward |
| SEQ ID NO: 22 | DnaJ domain-containing protein Oligo for guide Reversed |
| SEQ ID NO: 23 | XRI1-like protein Oligo for guide Forward |
| SEQ ID NO: 24 | XRI1-like protein Oligo for guide Reversed |
| SEQ ID NO: 25 | XRl1-like protein Oligo for guide Forward |
| SEQ ID NO: 26 | XRI1-like protein Oligo for guide Reversed |
| SEQ ID NO: 27 | DnaA initiator-associating diaA protein Oligo for guide Forward |
| SEQ ID NO: 28 | DnaA initiator-associating diaA protein Oligo for guide Reversed |
| SEQ ID NO: 29 | DnaA initiator-associating diaA protein Oligo for guide Forward |
| SEQ ID NO: 30 | DnaA initiator-associating diaA protein Oligo for guide Reversed |
| SEQ ID NO: 31 | TAF-2 protein Oligo for guide Forward |
| SEQ ID NO: 32 | TAF-2 protein Oligo for guide Reversed |
| SEQ ID NO: 33 | TAF-2 protein Oligo for guide Forward |
| SEQ ID NO: 34 | TAF-2 protein Oligo for guide Reversed |
| SEQ ID NO: 35 | PMT protein Oligo for guide Forward |
| SEQ ID NO: 36 | PMT protein Oligo for guide Reversed |
| SEQ ID NO: 37 | PMT protein Oligo for guide Forward |
| SEQ ID NO: 38 | PMT protein Oligo for guide Reversed |
| SEQ ID NO: 39 | eEF1B protein Oligo for guide Forward |
| SEQ ID NO: 40 | eEF1B protein Oligo for guide Reversed |
| SEQ ID NO: 41 | eEF1B protein Oligo for guide Forward |
| SEQ ID NO: 42 | eEF1B protein Oligo for guide Reversed |
| SEQ ID NO: 43 | TPR-like superfamily protein Oligo for guide Forward |
| SEQ ID NO: 44 | TPR-like superfamily protein Oligo for guide Reversed |
| SEQ ID NO: 45 | Adenylyl cyclase-associated protein Oligo for guide Forward |
| SEQ ID NO: 46 | Adenylyl cyclase-associated protein Oligo for guide Reversed |
| SEQ ID NO: 47 | Adenylyl cyclase-associated protein Oligo for guide Forward |
| SEQ ID NO: 48 | Adenylyl cyclase-associated protein Oligo for guide Reversed |
| SEQ ID NO: 49 | U6pro-F Oligo Forward |
| SEQ ID NO: 50 | DnaA initiator-associating dia A CDS (tomato) |
| SEQ ID NO: 51 | TAF-2 cDNA (tomato) |
| SEQ ID NO: 52 | Adenylyl cyclase-associated CDS (tomato) |
| SEQ ID NO: 53 | PMT cDNA (tomato) |
| SEQ ID NO: 54 | DnaJ domain-containing CDS (tomato) |
| SEQ ID NO: 55 | Elongation factor 1-gamma 1 (eEF1B) CDS (tomato) |
| SEQ ID NO: 56 | Tetratricopeptide repeat (TPR)-like superfamily CDS (tomato) |
| SEQ ID NO: 57 | XRl1-like CDS (tomato) |
| SEQ ID NO: 58 | DnaA initiator-associating dia A CDS (pepper) |
| SEQ ID NO: 59 | TAF-2 CDS (pepper) |
| SEQ ID NO: 60 | Adenylyl cyclase-associated CDS (pepper) |
| SEQ ID NO: 61 | Adenylyl cyclase-associated CDS (pepper) |
| SEQ ID NO: 62 | PMT CDS (pepper) |
| SEQ ID NO: 63 | PMT CDS (pepper) |
| SEQ ID NO: 64 | PMT CDS (pepper) |
| SEQ ID NO: 65 | DnaJ domain-containing CDS (pepper) |
| SEQ ID NO: 66 | Elongation factor 1-gamma 1 (eEF1B) CDS (pepper) |
| SEQ ID NO: 67 | TPR-like superfamily CDS (pepper) |
| SEQ ID NO: 68 | XRl1-like CDS (pepper) |
| SEQ ID NO: 69 | ToBRFV viral movement protein CDS |

### EXAMPLES

### Example 1

### Binding partners of ToBRFV viral movement protein

In order to investigate interaction with the proteins of the invention with the ToBRFV viral movement protein, a yeast two hybrid screening was performed. The yeast two-hybrid has been performed mainly as described e.g. in Naba et al. EMBO J. (2008) 27: 38-50. As bait, the ToBRFV - movement protein (aa 1-266) of SEQ ID NO: 69 was used. A cDNA prey library generated from tomato leave, stem and petiole was used from Hybrigenics Services SAS (Evry, France). The following proteins were identified in this yeast two-hybrid screening as binding partners of the ToBRFV movement protein:
- DnaA initiator-associating dia A protein (Solyc02g086130.3);
- TAF-2 protein (Solyc01g103540.3.1);
- Adenylyl cyclase-associated protein (Solyc10g051340.2.1);
- Phosphoethanolamine N-methyltransferase (PMT) protein (Solyc06g068950.3.1);
- DnaJ domain-containing protein (Solyc03g007280.3.1);
- Elongation factor 1-gamma 1 (eEF1B) protein (Solyc06g011280.3.1);
- Tetratricopeptide repeat (TPR)-like superfamily protein (Solyc01g095900.3.1); and
- XRI1-like protein (Solyc01g005010.3.1).

### Example 2

### Tobamovirus resistant mutants

### Design of guide RNA and constructs

For each target gene, i.e. gene encoding DnaJ domain-containing protein (SEQ ID NO: 5), gene encoding XRI1-like protein (SEQ ID NO: 8), gene encoding DnaA initiator-associating diaA protein (SEQ ID NO: 1), gene encoding TAF-2 protein (SEQ ID NO: 2); gene encoding PMT protein (SEQ ID NO: 4); gene encoding eEF1B protein (SEQ ID NO: 6), gene encoding Tetratricopeptide repeat (TPR)-like superfamily protein (SEQ ID NO: 7) and gene encoding Adenylyl cyclase-associated protein (SEQ ID NO: 3), one or two different guide RNAs were selected from the genomic sequence, preferably in the first exon. All guides were assembled by annealing complementary oligonucleotides of 21 target bases and a Bsal flanking site. For each guide, complementary forward and reverse oligonucleotides (see Table 2) were annealed and oligoduplexes were fused to the scaffold by cloning into Esp3I site of pJL462 (SEQ ID NO: 20); a plasmid containing Lbcas12a under control of 35S promoter and nos terminator, a guide scaffold under control of U6 promoter and nptll selection. Plasmids for multiple gene knockouts contain supplementary guide RNA expression cassette cloned into the Bsal site.

Transformed into competent E. coli 10-beta (NEB C3019H; New England Biolabs) according to provider instructions. Kanamycin resistant colonies were analyzed by colony PCR using the U6proF primer and the corresponding reverse primer.

**Table 2: Sequence of oligo used for guides**

| **Protein** | **Oligo for guide Forward** | **Oligo for guide Reverse** |
|---|---|---|
| DnaJ domain-containing protein | SEQ ID NO: 21 | SEQ ID NO: 22 |
| XRl1-like protein | SEQ ID NO: 23 | SEQ ID NO: 24 |
| XRl1-like protein | SEQ ID NO: 25 | SEQ ID NO: 26 |
| DnaA initiator-associating diaA protein | SEQ ID NO: 27 | SEQ ID NO: 28 |
| DnaA initiator-associating diaA protein | SEQ ID NO: 29 | SEQ ID NO: 30 |
| TAF-2 protein | SEQ ID NO: 31 | SEQ ID NO: 32 |
| TAF-2 protein | SEQ ID NO: 33 | SEQ ID NO: 34 |
| PMT protein | SEQ ID NO: 35 | SEQ ID NO: 36 |
| PMT protein | SEQ ID NO: 37 | SEQ ID NO: 38 |
| eEF1B protein | SEQ ID NO: 39 | SEQ ID NO: 40 |
| eEF1B protein | SEQ ID NO: 41 | SEQ ID NO: 42 |
| TPR-like superfamily protein | SEQ ID NO: 43 | SEQ ID NO: 44 |
| Adenylyl cyclase-associated protein | SEQ ID NO: 45 | SEQ ID NO: 46 |
| Adenylyl cyclase-associated protein | SEQ ID NO: 47 | SEQ ID NO: 48 |
| U6pro-F | SEQ ID NO: 49 | |

### Stable transformation

Moneyberg Plus tomato line is used for *Agrobacterium* transformation with GE constructs (Lbcas12a cassette, guideRNA cassette and nptll as selectable marker). Seeds are surface-sterilized for 20 min in 2% sodium hypochlorite containing 2 drops of Tween20 under agitation then washed 3 times with sterile distilled water. Seeds are then cultivated in plastic jars with MS medium (M0222, Duchefa) pH 5.9 containing 20 g/l sucrose and 0.8% microagar and placed at 25°C under light (3000 - 4000 lux, 16h photoperiod).

Explants are excised from cotyledons of 10 days seedlings. Using a sterile forceps and razorblade, both ends of each cotyledon are removed and cotyledons are then cut into 2 pieces. Explants are placed onto 10 cm diameter Petri dishes containing CC medium (MS medium pH 5.9 containing 20g/l sucrose and 0.8% microagar supplemented with 2 mg/l NAA, 1 mg/l BAP, 160 mg/l glucuronic acid and 40 mg/l acetosyringone) and placed at 25°C under light (3000 - 4000 lux, 16h photoperiod) for one day.

*Agrobacterium tumefaciens* strains are obtained by electroporation of the binary plasmids (guide RNA cloned into the pJL462 plasmid containing Lbcas12a, guide scaffold and nptll selectable marker gene) into *Agrobacterium tumefaciens* strain GV3101.
A single colony of *Agrobacterium tumefaciens* containing T-DNA plasmid is cultivated in 15 ml LB broth containing rifampicin 10 µg/ml and kanamycin 50 µg/ml in a shaker (200 rpm) at 28°C for 20 h.

Bacteria are pelleted by centrifugation of the overnight suspension for 20 min at 1000g and resuspended in sterile CC liquid medium (MS medium pH5.9 containing 20g/l sucrose supplemented with 2 mg/l NAA, 1 mg/l BAP, 160 mg/l glucuronic acid and 40 mg/l acetosyringone) to an OD600nm of 0.1.

In a sterile beaker, cotyledonary explants are soaked in the *Agrobacterium* suspension for 15 min under slow agitation (100 rpm). With sterile forceps, explants are blotted on a sterile filter paper then transferred to solid CC medium (MS medium pH5.9 containing 20g/l sucrose and 0.8% microagar supplemented with 2 mg/l NAA, 1 mg/l BAP, 160 mg/l glucuronic acid and 40 mg/l acetosyringone). Plates are placed at 25°C under light (3000 - 4000 lux, 16h photoperiod) for 48 hr.

Explants are rinsed two times with 100ml of liquid MS medium pH 5.9 containing 20 g/l sucrose and supplemented of 100 mg/l amoxicillin 20 mg/l clavulanic acid then blotted onto a sterile paper then transferred to solid selection medium (MS medium containing 20g/l sucrose and 0.8% microagar supplemented with 1 mg/l zeatin, 100 mg/l amoxicillin 20 mg/l clavulanic acid and 100 mg/l kanamycin) (10 explants/Petri dish). Petri dishes are placed at 25°C under light (3000 - 4000 lux, 16h photoperiod) and medium is refreshed every 2 weeks until shoot regeneration.

Shoots and plantlets are isolated and placed in plastic jars containing rooting medium (MS medium pH5.9 containing 20 g/l sucrose and 0.8% microagar supplemented with 0.5 mg/l IAA, 100 mg/l amoxicillin 20 mg/l clavulanic acid and 100 mg/l kanamycin). Jars are placed at 25°C under light (3000 - 4000 lux, 16h photoperiod).

Well rooted plantlets are transferred to soil. Agar is carefully removed by rinsing roots with water and plants are put in trays with soil and transferred to greenhouse for seed production.

Two weeks after acclimatization, plants are sampled and a piece of young leaf is analyzed by flow cytometry to select diploid plants.

Genomic DNA is extracted from a young leaf disk. Target gene region are amplified by PCR using forward and reverse primers designed upstream and downstream the guide RNA (see primer table). PCR products from transformed plant and non-transformed plant are mixed together then denatured at 95°C for 5 minutes and cool down slowly to room temperature for annealing. Duplexes are incubated with T7 endonuclease I for 20 minutes at 37°C. Enzymatic reactions are analyzed by electrophoresis on 2% agarose gel. Duplexes with a mismatch are digested by T7 endonuclease I.

PCR fragments are Sanger sequenced to confirm presence of indels in the guide region and to characterize position and length of the deleted sequence.

T0 plants displaying mutations are transplanted in a greenhouse for seed production. In the case of homozygous or bi-allelic mutation, seeds are used directly for plant phenotyping. Alternatively, seeds from heterozygous T0 mutant plants are sown and genotyping of T1 plants allows to identify homozygous mutants. Genomic DNA of T1 plants is extracted from a leaf disk and target sequence is PCR amplified and Sanger sequenced. Homozygous T1 plants are planted in the greenhouse for seed production.

### Protocol for evaluation of tobamovirus resistance:

All homozygous seed obtained are tested for resistance to Tobamoviruses. Several Tobamovirus isolates are used to perform a bioassay: ToBRFV isolates (internal ToBRFV strain *inter alia* Jordan_15 which shares more than 99% of nucleotide similarity with the ToBRFV isolate of GenBank accession KT383474; Salem et al. Arch. Virol. (2016) 161:503-506) and/or ToMV strain 0 as described in CPVO-TP/044/4-Rev.5 published on 14/04/2021 (Ad 48.1 - 48.3) and/or TMV and/or other tobamovirus. Virus isolates are maintained by frozen storage of infectious juice coming from 14 days old, infected tomato leaves grinded in water (inoculum proportion: 1g leaves for 4 ml of water). Bioassays are carried out by sap-inoculation of tomato plantlets at two-leaf stage (i.e. 14-16 days after seeding) by rubbing the cotyledons with the index finger. At least 18 plantlets (separated in 2 or 3 repetitions) per tomato genotypes (GE variant) are tested for tobamovirus resistance. Phenotypic evaluation of plant is carried out by plant by plant scoring, without contacting the plants. Systemic symptoms evaluation is carried out at 14, 21 and 28 DPI, the last evaluation being optional. Symptoms are visually assessed according the following scale: 9: No visible symptoms / 7: weak symptoms (weak mosaic) / 5: mild symptoms (mosaic and/or light vein banding) / 3: strong symptoms (strong mosaic and/or pronounced vein banding and/or small leaves deformation) / 1: very strong symptoms (leaves deformation and/or mosaic and/or highly pronounced vein banding).

After 21 and/or 28 days of test, the plants without symptoms are isolated (to avoid any contact with contaminated plants) for at least 7 days and all new systemic leaves are tested by quantitative PCR to evaluate the presence of tobamovirus in plant.

## Claims

1. Method for producing a plant having improved tobamovirus resistance as compared to a control plant, comprising the step of impairing expression and/or activity of at least one of:
- a DnaA initiator-associating dia A protein, preferably having an amino acid sequence of SEQ ID NO: 1 or a homologue or orthologue thereof;
- a TAF-2 protein, preferably having an amino acid sequence of SEQ ID NO: 2 or a homologue or orthologue thereof;
- an Adenylyl cyclase-associated protein, preferably having an amino acid sequence of SEQ ID NO: 3 or a homologue or orthologue thereof;
- a Phosphoethanolamine N-methyltransferase (PMT) protein, preferably having an amino acid sequence of SEQ ID NO: 4 or a homologue or orthologue thereof;
- a DnaJ domain-containing protein, preferably having an amino acid sequence of SEQ ID NO: 5 or a homologue or orthologue thereof;
- an Elongation factor 1-gamma 1 (eEF1B) protein, preferably having an amino acid sequence of SEQ ID NO: 6 or a homologue or orthologue thereof;
- a Tetratricopeptide repeat (TPR)-like superfamily protein, preferably having an amino acid sequence of SEQ ID NO: 7 or a homologue or orthologue thereof; and
- an XRl1-like protein, preferably having an amino acid sequence of SEQ ID NO: 8 or a homologue or orthologue thereof.

2. Method according to claim 1, wherein expression and/or activity of said at least one protein is impaired by modifying an endogenous gene encoding said protein.

3. Method according to claim 1 or 2, wherein the step of impairing expression and/or activity comprises the insertion, deletion and/or substitution of at least one nucleotide in the sequence encoding the endogenous protein, resulting in a sequence encoding a modified protein with reduced function.

4. Method according to claim 1 or 2, wherein the step of impairing the expression comprises the insertion, deletion and/or substitution of at least one nucleotide in a transcription regulatory sequence of the gene encoding the endogenous protein resulting in decreased protein expression.

5. Method according to any one of the preceding claims, wherein said tobamovirus is at least one of tomato brown rugose fruit virus (ToBRFV) and tomato mosaic virus (ToMV).

6. Method according to any one of the preceding claims, wherein the method further comprises a step of regenerating said plant.

7. Method according to any one of the preceding claims, wherein expression and/or activity of the protein is impaired at least in the leaves and/or fruits of said plant.

8. A nucleic acid comprising a gene encoding a protein, wherein said gene comprises one or more modifications resulting in impaired expression and/or activity of the encoded protein selected from the group consisting of:
- a DnaA initiator-associating dia A protein, preferably having an amino acid sequence of SEQ ID NO: 1 or a homologue or orthologue thereof;
- a TAF-2 protein, preferably having an amino acid sequence of SEQ ID NO: 2 or a homologue or orthologue thereof;
- an Adenylyl cyclase-associated protein, preferably having an amino acid sequence of SEQ ID NO: 3 or a homologue or orthologue thereof;
- a Phosphoethanolamine N-methyltransferase (PMT) protein, preferably having an amino acid sequence of SEQ ID NO: 4 or a homologue or orthologue thereof;
- a DnaJ domain-containing protein, preferably having an amino acid sequence of SEQ ID NO: 5 or a homologue or orthologue thereof;
- an Elongation factor 1-gamma 1 (eEF1B) protein, preferably having an amino acid sequence of SEQ ID NO: 6 or a homologue or orthologue thereof;
- a Tetratricopeptide repeat (TPR)-like superfamily protein, preferably having an amino acid sequence of SEQ ID NO: 7 or a homologue or orthologue thereof; and
- an XRl1-like protein, preferably having an amino acid sequence of SEQ ID NO: 8 or a homologue or orthologue thereof.

9. A construct, vector or host cell comprising the nucleic acid of claim 8.

10. A protein capable of improving tobamovirus resistance when expressed in a plant, wherein the protein has one or more modifications as compared to the protein as defined in claim 1.

11. A plant obtainable by a method according to any one of claims 1-7, or progeny thereof.

12. A plant having improved tobamovirus resistance as compared to a control plant, wherein said plant shows impaired expression and/or activity of at least one protein selected from the group consisting of:
- a DnaA initiator-associating dia A protein, preferably having an amino acid sequence of SEQ ID NO: 1 or a homologue or orthologue thereof;
- a TAF-2 protein, preferably having an amino acid sequence of SEQ ID NO: 2 or a homologue or orthologue thereof;
- an Adenylyl cyclase-associated protein, preferably having an amino acid sequence of SEQ ID NO: 3 or a homologue or orthologue thereof;
- a Phosphoethanolamine N-methyltransferase (PMT) protein, preferably having an amino acid sequence of SEQ ID NO: 4 or a homologue or orthologue thereof;
- a DnaJ domain-containing protein, preferably having an amino acid sequence of SEQ ID NO: 5 or homologue or orthologue thereof;
- an Elongation factor 1-gamma 1 (eEF1B) protein, preferably having an amino acid sequence of SEQ ID NO: 6 or a homologue or orthologue thereof;
- a Tetratricopeptide repeat (TPR)-like superfamily protein, preferably having an amino acid sequence of SEQ ID NO: 7 or a homologue or orthologue thereof; and
- an XRl1-like protein, preferably having an amino acid sequence of SEQ ID NO: 8 or a homologue or orthologue thereof.

13. A plant according to claim 11 or 12, wherein said plant comprises a nucleic acid of claim 8 or construct, vector or host cell according to claim 9, or wherein said plant expresses a protein of claim 10, or progeny thereof.

14. Use of a nucleic acid of claim 8, construct, vector or host cell of claim 9 or protein of claim 10 for improving tobamovirus resistance in a plant.

15. Method of screening a plant for tobamovirus resistance, wherein said method comprises the steps of:
- assessing the presence of the nucleic acid of claim 8, a construct, vector or host cell of claim 9 or a protein of claim 10 in said plant; and
- optionally selecting said plant.


SEQUENCE LISTING

<110>  Keygene N.V.

<120>  Tobamovirus resistant plants

<130>  P6107004EP

<160>  69

<170>  PatentIn version 3.5

<210>  1
<211>  962
<212>  PRT
<213>  Lycopersicon esculentum

<400>  1

Met Asn Asp Ser Leu Ala Ile Thr Ala Ser Ser Leu Ala Ile Thr Glu
1               5                   10                  15

Lys Lys Pro Gln Arg Pro Gly Gly Cys Val Gly Ile Phe Phe Gln Leu
            20                  25                  30

Phe Asp Trp Asn Arg Arg Phe Ala Lys Lys Lys Leu Phe Pro Lys Lys
        35                  40                  45

Leu Leu Ser Pro Ala Arg Leu Lys Gln Ala Ser Lys Lys Phe Gly Gly
    50                  55                  60

Asp Glu Lys Gln Pro Lys His Arg Leu Ile Ala Asn Glu Asn Ser Gly
65                  70                  75                  80

Gly Phe Pro Ile Ala Lys Ser Asn Gly Met Ser Asn Thr Arg Cys Glu
                85                  90                  95

Ser Lys Arg Glu Met Lys Ala Pro Ser Leu Val Ala Arg Leu Met Gly
            100                 105                 110

Leu Glu Ser Met Pro Ala Gly Pro Gly Ser Lys Ala Lys Lys Ala Ser
        115                 120                 125

Ala Ser Glu Thr Gly Ser Tyr Val Ala Glu Lys Leu Asp Ala Arg Pro
    130                 135                 140

Gly Gly Ser Asp Lys Glu Asp Met Asp Cys Glu Lys Ala Glu Ile Lys
145                 150                 155                 160

Arg Glu Leu Arg Pro Gln Lys Leu Gln Lys Ile Gly Val Ser Glu Arg
                165                 170                 175

Arg Pro Val Ser Arg Phe Ser Ala Glu Ala Leu Gln Leu Arg Thr Val
        180                 185                 190

Leu Ser Arg Pro Arg Lys His Gln Pro Lys Leu Thr Ser Pro Val Lys
    195                 200                 205

Ser Pro Arg Asn Val Ser Gly Arg Asn Ala Ser Arg Leu Ile Gly Ala
    210                 215                 220

Ala Thr Arg Ile Leu Glu Pro Gly Leu Gln Lys Ser Arg Ala Lys Cys
225                 230                 235                 240

Ala Leu Thr Tyr Pro Lys Tyr Phe Ser Pro Leu Glu Asp Lys Ala Asp
                245                 250                 255

Leu Ala Leu His His Leu Glu Val Pro Asn Pro Cys Val Asp Ser Lys
            260                 265                 270

Thr Ser Glu Val Arg Ala Ser Val Pro Ser Cys Lys Asn Cys Gly Tyr
        275                 280                 285

Met Leu His Ser Lys Asn Gly Thr Pro Asn Gly Glu Glu His Pro Ser
    290                 295                 300

Ser Val Ser Ser Pro Val Ser Ser Tyr Ser Gln Pro Ser Cys Gln Gly
305                 310                 315                 320

Pro Gly Arg Asn Met Leu Arg Leu Pro Ile Ile Asn Ser Arg Asp Gln
                325                 330                 335

Leu Glu Arg Val Phe Glu Gly Ser Ser Ser Asp Ala Asn Ala Glu Ile
            340                 345                 350

Asp Asp Val Ser Tyr Cys Ala Glu Leu Ile Leu Gly Lys Arg Pro Ile
        355                 360                 365

Ser Arg Ser Arg Ile Ala Met His Gly Ala Cys Gln Gly Ser Asn Val
    370                 375                 380

Lys Lys Asp Ala Ser Ser Val Thr His Val Leu Asn Gln Lys Gln Asn
385                 390                 395                 400

Gln Thr Ser Gln Asn Arg Glu Arg Gly Phe Met Lys Ser Lys Gln Ser
                405                 410                 415

Ser Leu Gln Ser Asn Arg Val Leu Ala Ala Ala Glu Ser Thr Ile Asn
            420                 425                 430

Thr Lys Ser Phe Val Ala Gln Asn Arg Arg Leu Gly Ala Ser Thr Arg
        435                 440                 445

Leu Arg Met Pro Ala Thr Ala Asp Gly Cys Lys Phe Glu Thr Glu Arg
    450                 455                 460

Lys Pro Tyr Ser Arg Arg Ser Asp Ser Leu Ser Pro Val Arg Lys Lys
465                 470                 475                 480

Arg Leu Met Asn Val Ser Arg Gln Gly Glu Ser Ser Ser Phe Val Asn
                485                 490                 495

Ala Asn Leu Gly Arg Glu Ser Ser Pro Tyr Ser Asp Lys Thr Ser Arg
            500                 505                 510

Lys Asp Val Phe Pro Ile Ser Ser Val Asn Ser His Ser Thr Lys Pro
        515                 520                 525

Lys Leu Pro Cys Leu Arg Glu Ser Gly Ala Thr Asn Asn Ser Ser Glu
    530                 535                 540

Gly Ser Asn Val Val Ser Phe Thr Phe Arg Ser Ala Met Lys Gln Lys
545                 550                 555                 560

Ala Gly Ile His Ala Glu Val Thr Lys Arg Lys Ser Gln Asn Ser Ser
                565                 570                 575

Ser Phe Asp Ala Thr Pro Gly Arg Ser Phe Phe Thr Gly Asn Asp Glu
            580                 585                 590

Thr Ala Cys Leu Gln Lys Ser Phe Pro Leu Lys Gly Asp Ile Leu Gly
        595                 600                 605

Ala Leu Leu Glu Gln Lys Leu Lys Glu Leu Thr Ser Glu Glu Glu Phe
    610                 615                 620

Ala Glu Gly Asp Ala Ala Pro Arg Lys Ser Thr Ala Thr Ile Leu Gln
625                 630                 635                 640

Glu Leu Ile Thr Ala Leu Asn Asp Glu Thr Gln Phe His Leu Asp Ser
                645                 650                 655

Leu Pro Ser Lys Pro Asn Arg Lys Glu Asp Leu Tyr Asp Asp Arg Glu
            660                 665                 670

Val Ser Ser Arg Asn Thr Ser Met Asn Phe Gln Ala Ile Pro Asp Ser
        675                 680                 685

Ala Thr Asp Leu Val Gly Asn Ser Leu Asp Asn Asp His Leu Ser Pro
    690                 695                 700

Gly Cys Val Leu Glu Ala Thr Phe Ser Thr Asp Ser Tyr Leu Ser Ser
705                 710                 715                 720

Ser Pro Asn Ser Ser Ser Lys Asp Lys Val Leu Ala Glu Ser Val Asp
                725                 730                 735

Ser Ile Tyr Asp Glu Pro Leu Phe Pro Glu Pro Asp Arg Asp Leu Ser
            740                 745                 750

Asp Cys Ala Thr Ser Leu Phe Thr Arg Arg Ser Cys Arg Ala Leu Ile
        755                 760                 765

Thr Asp His Val Asn Asn Ile Ser Gly Val Leu Ser Lys Ile Asn Gln
    770                 775                 780

Leu Lys Gly Ser Lys Leu Gly Tyr Ala Asn Glu Val Ile Leu Asn Thr
785                 790                 795                 800

Glu Leu Ile Leu Gly Thr Ser Pro Glu Gln Gln Ala Leu Pro Val Asp
                805                 810                 815

Asp Gly Leu Ser Val Ser His Phe Leu Leu Asn Glu Leu Glu Met Leu
            820                 825                 830

Ser Ser Leu Leu Trp Met Thr Phe Gly Gln Leu Leu Gly Cys Asn Asp
        835                 840                 845

Pro Lys Gln Met Asn Gln Leu Lys Gly Phe Ala Phe Asp Cys Leu Leu
    850                 855                 860

Glu Tyr Leu Asp Ser Lys Phe Gly Arg Tyr Ser Asp Ser Gly Phe Arg
865                 870                 875                 880

Ile Trp Ser Lys Leu Pro Ser Ser Met Thr Lys Glu Ile Leu Ile Ala
                885                 890                 895

Asp Ile Ile Glu Glu Val Lys Glu Trp Thr Glu Phe Val Gly Leu Ile
            900                 905                 910

Pro Asp Glu Leu Ile Glu Trp Asp Met Ser His Ser Leu Gly Lys Trp
        915                 920                 925

Thr Asp Phe Glu Ile Glu Glu Phe Glu Cys Gly Thr Glu Val Asp Arg

930                 935                 940

His Ile Leu Gln Val Leu Val Asp Glu Val Val Leu Asp Leu Tyr Ser
945                 950                 955                 960

Ser Ser

<210>  2
<211>  556
<212>  PRT
<213>  Lycopersicon esculentum

<400>  2

Met Met Ala Gly Glu Lys Ile Ile Glu Lys Pro Glu Ala Val Ala Pro
1               5                   10                  15

Gly Leu Lys Ser Asp Pro Ser Asn Lys Leu Ile Glu Lys Asp Leu Val
            20                  25                  30

Ser Lys Lys Asp Gly Lys Ala Ala Asp Ser Val Ala Ser Leu Gly Ala
        35                  40                  45

Val Thr Ser Ile Lys Cys Glu Asn Asp Gln Pro Pro Ala Ala Glu Gln
    50                  55                  60

Gly Tyr Asn Gly Thr Tyr Asn Gln Leu Asp Glu Gln Ala Tyr Phe Asn
65                  70                  75                  80

Ala Gly Val Gln Ser Asp Asn Gly Ser Leu Leu Tyr Tyr Met Pro Gly
                85                  90                  95

Tyr Asn Pro Tyr Ser Ala Gly Phe Val Gly Gly Asp Gly Lys Gln Pro
            100                 105                 110

Tyr Pro Ser Ser Gly Tyr Leu Gln Gln Pro Val Ser Tyr Gly Ser Asp
        115                 120                 125

Ser Met Pro Cys Tyr Thr Trp Gly Ser Pro Tyr Cys Ala Asp Ile Thr
    130                 135                 140

Asn Ser Ala Ala Pro Lys Ser Gly Asn Val Lys Ser Thr Phe Gly Arg
145                 150                 155                 160

Asn Gly Ser Val Lys Ser Asn Gly Phe Asn Ser Thr Lys Thr Asn Ser
                165                 170                 175

Ser Phe Ser Ser Lys Asn Ser Thr Val Leu Phe Asn Pro Lys Ser Arg

180                 185                 190

Pro Ala Thr Ala Met Ser Asn Pro Pro Lys Ser Phe His Gln Ala Gln
        195                 200                 205

Pro Phe Asn Pro Val Asn Lys Phe Gln Ser Asp Val Gln Ser Gly Gly
    210                 215                 220

Leu Met Lys Gly Phe His Leu Val Gly Asp Tyr Pro Ser Tyr Thr Ser
225                 230                 235                 240

Gln Asn Gln Gly Phe Phe Met Pro Tyr Asp Pro Ile Asn Cys Gln Thr
                245                 250                 255

Asn Ser Arg Met Trp Asn Gly Asn Tyr Arg Ala Lys Pro Arg Gly Asn
            260                 265                 270

Phe Thr Arg Asn Gly Val Phe Glu Ala Thr Asn Glu Leu Pro Arg Gly
        275                 280                 285

Pro Arg Ala Asn Gly Arg Ser Val Pro Ser Lys Pro Ser Ala Glu Glu
    290                 295                 300

Asp Gln Leu Val Pro Ala Val Gln Arg Glu Lys Tyr Asn Lys Glu Asp
305                 310                 315                 320

Phe Lys Thr Gln Tyr Asp Asn Ala Lys Phe Tyr Ile Ile Lys Ser Tyr
                325                 330                 335

Ser Glu Asp Asp Ile His Lys Cys Val Lys Tyr Asp Val Trp Ser Ser
            340                 345                 350

Thr Pro Asn Gly Asn Lys Lys Leu Asp Thr Ala Phe Val Glu Ser Glu
        355                 360                 365

Ala Lys Ala Ser Gly Thr Gly Ser Ser Cys Pro Val Phe Leu Phe Phe
    370                 375                 380

Ser Val Asn Gly Ser Gly Gln Phe Leu Gly Val Ala Glu Met Val Gly
385                 390                 395                 400

Gln Val Asp Phe Asn Arg Asn Met Asp Phe Trp Gln Leu Asp Lys Trp
                405                 410                 415

Ser Gly Phe Phe Pro Leu Lys Trp His Ile Val Lys Asp Val Pro Asn
            420                 425                 430

Thr Gln Phe Arg His Ile Ile Leu Glu Asn Asn Asp Asn Arg Pro Val
        435                 440                 445

Thr Tyr Ser Arg Asp Thr Gln Glu Ile Gly Leu Lys Glu Gly Leu Glu
    450                 455                 460

Met Leu Asn Ile Leu Lys Asn Tyr Ser Glu Lys Thr Ser Ile Leu Asp
465                 470                 475                 480

Asp Phe Asn Phe Tyr Glu Lys Arg Glu Lys Val Leu Lys Ala Lys Arg
                485                 490                 495

Ser Ser Lys Pro Val Ile Gln Ala Asp Ala Tyr Glu Lys Ala Asp Ser
            500                 505                 510

Leu Lys Gln Phe Lys Gly Gly Asp Lys Val Leu Glu Glu Glu Leu Lys
        515                 520                 525

Thr Asn Ser Thr Asp Pro Thr Ala Pro Leu Val Ser Leu Thr Lys Asn
    530                 535                 540

Leu Ser Ile Asn Ser Arg Pro Phe Lys Ser Ser Val
545                 550                 555

<210>  3
<211>  471
<212>  PRT
<213>  Lycopersicon esculentum

<400>  3

Met Asp Glu Lys Leu Ile Gln Arg Leu Glu Ser Ala Val Ser Arg Leu
1               5                   10                  15

Glu Ala Leu Ser Val Gly Gly Gly Val Ser Leu Gly Asp Gly Glu Ala
            20                  25                  30

Ala Ala Ala Leu Asp Pro Ser Ile Ile Ala Phe Asp Asp Leu Arg Ser
        35                  40                  45

Arg Phe Val Gly Arg Val Cys Thr Ala Ala Glu Lys Ile Gly Gly Gln
    50                  55                  60

Val Leu Asp Val Thr Lys Ile Val Glu Glu Ala Phe Glu Ala Gln Arg
65                  70                  75                  80

Glu Leu Leu Ile Lys Ile Lys Glu Thr Gln Lys Pro Ser Met Ser Gly
                85                  90                  95

Leu Ala Glu Phe Leu Lys Pro Leu Asn Asp Val Ile Val Lys Gly Thr
            100                 105                 110

Lys Met Thr Glu Gly Arg Arg Ser Asp Leu Phe Asn His Leu Lys Ala
        115                 120                 125

Ala Val Asp Ser Leu Thr Ala Leu Ala Trp Ile Ala Tyr Thr Gly Lys
    130                 135                 140

Asp Cys Gly Met Ser Met Pro Ile Ala His Val Glu Glu Ser Trp Gln
145                 150                 155                 160

Met Ala Glu Phe Tyr Asn Asn Lys Val Leu Val Glu Phe Arg Asn Lys
                165                 170                 175

Asp Pro Asp His Val Glu Trp Ala Lys Ala Leu Lys Glu Ile Tyr Leu
            180                 185                 190

Pro Gly Leu Arg Asp Tyr Val Lys Ser His Tyr Pro Leu Gly Pro Ala
        195                 200                 205

Trp Ser Ala Thr Gly Lys Val Ala Val Ser Ala Pro Pro Lys Ala Pro
    210                 215                 220

Ala Pro Ser Arg Pro Met Pro Pro Pro Leu Pro Pro Ala Ser Leu Phe
225                 230                 235                 240

Ser Ser Glu Ser Pro Gln Ala Ser Ser Ser Arg Pro Lys Lys Gly Met
                245                 250                 255

Ser Ala Val Phe Asp Glu Ile Asn Ser Gly Lys Pro Val Thr Gly Gly
            260                 265                 270

Leu Arg Lys Val Thr Asp Asp Met Lys Thr Lys Asn Arg Ala Asp Arg
        275                 280                 285

Thr Gly Val Ile Ser Ala Gly Glu Lys Glu Arg Arg Val Ser Ser Pro
    290                 295                 300

Ser Phe Ser Lys Ala Gly Pro Pro Lys Leu Glu Leu Gln Met Gly Arg
305                 310                 315                 320

Lys Trp Val Val Glu Asn Gln Ile Gly Arg Lys Asn Leu Val Ile Asp
                325                 330                 335

Asp Cys Asp Ala Lys Gln Ser Val Tyr Ile Phe Gly Cys Lys Asp Ser
            340                 345                 350

Val Leu Gln Ile Gln Gly Lys Val Asn Asn Ile Thr Ile Asp Lys Cys
        355                 360                 365

Thr Lys Met Gly Val Val Phe Ser Asp Val Val Ala Ala Cys Glu Val
    370                 375                 380

Val Asn Cys Asn Gly Val Glu Val Gln Cys Gln Gly Ser Ala Pro Thr
385                 390                 395                 400

Ile Ser Val Asp Asn Thr Thr Gly Cys Gln Leu Tyr Leu Ser Lys Asp
                405                 410                 415

Ser Leu Gly Thr Ser Ile Thr Thr Ala Lys Ser Ser Glu Ile Asn Val
            420                 425                 430

Leu Val Pro Gly Ala Gly Ala Asp Ser Asp Trp Gly Glu His Ala Leu
        435                 440                 445

Pro Gln Gln Phe Ser His Val Tyr Lys Ser Gly Gln Phe Val Thr Thr
    450                 455                 460

Pro Val Ser His Ser Gly Gly
465                 470

<210>  4
<211>  494
<212>  PRT
<213>  Lycopersicon esculentum

<400>  4

Met Ala Ala Ile Ala Pro Glu Val Gly Glu Glu Arg Glu Ile Gln Lys
1               5                   10                  15

Ser Tyr Trp Met Glu Gln Thr Ser Glu Leu Asn Leu Glu Ala Met Met
            20                  25                  30

Arg Asp Ser Ala Ala Ser Asp Leu Asp Lys Glu Asp Arg Pro Glu Val
        35                  40                  45

Leu Ser Leu Leu Pro Ser Tyr Glu Gly Lys Ser Val Leu Glu Leu Gly
    50                  55                  60

Ala Gly Ile Gly Arg Phe Thr Gly Asp Leu Ala Glu Lys Ala Gly Glu
65                  70                  75                  80

Leu Val Ala Val Asp Phe Ile Glu Glu Val Thr Lys Lys Asn Glu Ser
                85                  90                  95

Ile Asn Gly His His Lys Asn Val Lys Phe Val Cys Ala Asp Val Thr
            100                 105                 110

Ser Pro Asp Leu Thr Val Leu Pro Glu Ser Val Asp Leu Ile Phe Ser
        115                 120                 125

Asn Trp Leu Leu Met Tyr Leu Ser Asn Glu Glu Val Gln Asp Leu Ala
    130                 135                 140

Glu Arg Met Ile Lys Trp Leu Lys Val Gly Gly Tyr Ile Phe Phe Arg
145                 150                 155                 160

Glu Thr Cys Phe His Gln Ser Gly Asp His Lys Gly Lys Asn Asn Pro
                165                 170                 175

Thr His Tyr Arg Glu Pro Arg Phe Tyr Thr Lys Val Phe Gln Glu Cys
            180                 185                 190

Thr Leu Lys Asp Asp Ala Gly Lys Ser Tyr Glu Leu Ser Leu Ile Gly
        195                 200                 205

Tyr Lys Cys Ile Glu Thr Tyr Val Lys Asn Lys Lys Asn Gln Asn Glu
    210                 215                 220

Ile Cys Trp Ile Trp Gln Lys Val His Ser Glu Asp Asp Arg Gly Phe
225                 230                 235                 240

Gln His Phe Leu Asp Thr Val Gln Tyr Lys Ser Asn Gly Ile Leu Arg
                245                 250                 255

Tyr Glu Arg Val Phe Gly Ala Gly Phe Val Ser Thr Gly Gly Ile Asp
            260                 265                 270

Thr Thr Lys Glu Phe Val Ala Met Met Asp Leu Gln Pro Gly Glu Lys
        275                 280                 285

Val Leu Asp Val Gly Cys Gly Ile Gly Gly Gly Asp Phe Tyr Met Ala
    290                 295                 300

Glu Lys Tyr Asp Val His Val Val Gly Ile Asp Leu Ser Val Asn Met
305                 310                 315                 320

Ile Thr Leu Ala Phe Glu Arg Ala Ile Gly Arg Lys Cys Ala Val Glu
                325                 330                 335

Phe Glu Val Ala Asp Cys Thr Val Lys Thr Tyr Pro Glu Glu Ser Phe
            340                 345                 350

Asp Val Ile Tyr Ser Arg Asp Thr Ile Leu His Ile Gln Asp Lys Pro
        355                 360                 365

Ala Leu Phe Arg Ser Phe Tyr Lys Trp Leu Lys Pro Gly Gly Arg Val
    370                 375                 380

Leu Ile Ser Asp Tyr Cys Lys Lys Ala Gly Pro Ala Ser Glu Asp Phe
385                 390                 395                 400

Ala Ala Tyr Ile Lys Gln Arg Gly Tyr Asp Leu His Asp Val Glu Ala
                405                 410                 415

Tyr Gly Gln Met Leu Arg Asp Ala Gly Phe Asn Glu Val Ile Ala Asp
            420                 425                 430

Asp Arg Thr Glu Gln Phe Met Gln Val Leu Gln Lys Glu Leu Asp Thr
        435                 440                 445

Val Glu Asn Glu Arg Glu Leu Phe Ile Gln Glu Phe Ser Glu Gln Asp
    450                 455                 460

Tyr Asn Asp Ile Val Gly Gly Trp Lys Ala Lys Leu Val Arg Ser Ser
465                 470                 475                 480

Ser Gly Glu Gln Arg Trp Gly Leu Phe Phe Ala Lys Lys Asn
                485                 490

<210>  5
<211>  508
<212>  PRT
<213>  Lycopersicon esculentum

<400>  5

Met Glu Cys Asn Lys Asp Glu Ala Phe Arg Ala Lys Lys Ile Ala Glu
1               5                   10                  15

Arg Lys Phe Glu Gln Lys Asp Tyr Ala Gly Ala Lys Lys Phe Ala Leu
            20                  25                  30

Lys Ala Gln Val Leu Tyr Pro Gly Leu Asp Asp Leu Thr Gln Met Leu
        35                  40                  45

Thr Thr Phe Asp Val Tyr Ile Ser Ala Glu Asn Lys Val Ser Gly Glu
    50                  55                  60

Val Asp Trp Tyr Gly Val Leu Gly Val Ser Pro Ser Ser Asp Asp Glu
65                  70                  75                  80

Thr Val Lys Lys Gln Tyr Arg Lys Leu Ala Leu Val Leu His Pro Asp
                85                  90                  95

Lys Asn Lys Ser Ile Gly Ala Glu Gly Ala Phe Gln Leu Leu Ser Glu
            100                 105                 110

Ala Trp Ser Leu Leu Ser Asp Lys Ser Lys Arg Leu Ala Tyr Asn Gln
        115                 120                 125

Arg Arg Ser Ser Lys Gly Pro Gln Gln Lys Gln Gln Lys Val Pro Val
    130                 135                 140

Pro Ser Gly Gly Pro Ser Thr Phe Trp Thr Ile Cys His Arg Cys Lys
145                 150                 155                 160

Met His Tyr Glu Tyr Leu Lys Ile Tyr Leu Asn His Thr Leu Leu Cys
                165                 170                 175

Pro Asn Cys His Glu Ser Thr Ser Gln Trp Lys Arg Glu Arg Arg Leu
            180                 185                 190

Lys Gly Val Lys Lys Val Pro Asp Pro Asp Phe His Asn Phe Asp Gln
        195                 200                 205

Asp Arg Ser Glu Ser Cys Phe Glu Asp Asn Glu Val Trp Ala Ser Tyr
    210                 215                 220

Asp Ala Asp Asp Gly Met Pro Arg Phe Tyr Ala Leu Ile Asn Lys Val
225                 230                 235                 240

Ile Ser Arg Glu Pro Phe Lys Val Arg Leu Ser Trp Leu Asn Ser Lys
                245                 250                 255

Thr Asn Thr Glu Phe Gly Pro Met Glu Trp Val Ala Ser Gly Phe Tyr
            260                 265                 270

Lys Thr Ser Gly Glu Phe Arg Ile Gly Arg Tyr Glu Thr Gly Lys Ser
        275                 280                 285

Val Asn Ser Phe Ser His Lys Val Arg Trp Ser Lys Gly Pro Arg Gly
    290                 295                 300

Thr Val Leu Ile Tyr Pro Gln Lys Gly Asp Val Trp Ala Leu Phe Arg
305                 310                 315                 320

Asn Trp Ser Ala Asp Trp Asn Gln Asn Thr Pro Asp Asp Val Ile His

                325                 330                 335

Lys Tyr Asp Met Val Leu Val Leu Asp Asp Tyr Asn Glu Glu Gln Gly
            340                 345                 350

Ile Ser Val Ala Pro Leu Ile Lys Val Ala Gly Phe Lys Thr Val Phe
        355                 360                 365

Arg Pro Asp Leu Asn Pro Glu Lys Met Ile Arg Ile Thr Arg Glu Glu
    370                 375                 380

Met Phe Arg Phe Ser His Gln Val Pro Ser His Leu Leu Thr Gly Glu
385                 390                 395                 400

Glu Gly Gln Asn Ala Pro Lys Gly Cys Gln Glu Leu Asp Pro Ala Ala
                405                 410                 415

Thr Pro Leu Glu Leu Leu Gln Thr Val Thr Glu Thr Asn Glu Val Pro
            420                 425                 430

Ala Met Gln Asn Asp Lys Glu Ala Asn Gly Gly Ser Ser Gln Lys Val
        435                 440                 445

Gln Glu Thr Lys Thr Ser Glu Ala Ala Asp His Thr Leu Lys Ser Arg
    450                 455                 460

Glu Gly Gly Met Val Glu Ser Glu Gly Glu Ile Ser Leu Gly Ser Glu
465                 470                 475                 480

Gln Met Val Glu Lys Val Thr Met Glu Gln Thr Gln Val Asn Pro Val
                485                 490                 495

Lys Thr Asp Lys Ala Thr Lys Phe Gly Thr Glu Thr
            500                 505

<210> 6
<211> 413
<212> PRT
<213> Lycopersicon esculentum

<400> 6

Met Ala Leu Ile Leu His Ser Thr Asp Asn Asn Lys Asn Ala Ser Lys
1               5                   10                  15

Ala Leu Ile Ala Ala Glu Tyr Thr Gly Val Lys Val Asp Leu Ala Lys
            20                  25                  30

Asp Phe Gln Met Gly Val Ser Asn Lys Thr Pro Glu Phe Leu Glu Met

35                  40                  45

Asn Pro Ile Gly Lys Val Pro Val Leu Gln Thr Pro Asp Gly Pro Val
    50                  55                  60

Phe Glu Ser Asn Ala Ile Ala Arg Tyr Val Thr Lys Thr Lys Pro Asn
65                  70                  75                  80

Asn Pro Leu Phe Gly Ser Ser Leu Ile Glu Tyr Ala Gln Ile Glu Gln
                85                  90                  95

Trp Asn Asp Phe Ser Ala Thr Glu Ile Asp Ala Asn Ile Ala Arg Trp
            100                 105                 110

Leu Tyr Pro Arg Leu Gly Tyr Ala Val Tyr Ile Ser Gln Ala Glu Glu
        115                 120                 125

Gly Ala Val Ala Ala Leu Lys Arg Ala Leu Gly Ala Leu Asn Thr His
    130                 135                 140

Leu Ala Ser Asn Thr Tyr Leu Val Gly His Phe Ile Thr Leu Ala Asp
145                 150                 155                 160

Ile Ile Met Val Cys Asn Leu Ser Ile Gly Phe Arg Met Ile Leu Thr
                165                 170                 175

Lys Ser Phe Thr Lys Glu Phe Pro His Val Glu Arg Tyr Phe Trp Thr
            180                 185                 190

Val Val Asn Gln Pro Asn Phe Val Lys Ile Leu Gly Glu Val Lys Gln
        195                 200                 205

Ala Glu Ser Ile Pro Ala Val Gln Ser Lys Pro Ala Gln Pro Glu Lys
    210                 215                 220

Leu Lys Ala Lys Glu Glu Pro Lys Lys Glu Val Lys Lys Glu Glu Pro
225                 230                 235                 240

Ser Pro Val Glu Glu Glu Ala Ala Pro Lys Pro Lys Ala Lys Asn Pro
                245                 250                 255

Leu Asp Leu Leu Pro Pro Ser Lys Met Ile Leu Asp Asp Trp Lys Arg
            260                 265                 270

Leu Tyr Ser Asn Thr Lys Thr Asn Phe Arg Glu Val Ala Val Lys Gly
        275                 280                 285

Phe Trp Asp Met Tyr Asp Pro Glu Gly Tyr Ser Leu Trp Phe Cys Asp
    290                 295                 300

Tyr Lys Tyr Asn Asp Glu Asn Thr Val Ser Phe Val Thr Leu Asn Lys
305                 310                 315                 320

Val Gly Gly Phe Leu Gln Arg Met Glu Leu Val Arg Lys Tyr Ala Phe
                325                 330                 335

Gly Lys Met Leu Ile Val Gly Ser Glu Ala Pro Phe Lys Val Gln Gly
            340                 345                 350

Leu Trp Leu Phe Arg Gly Lys Glu Ile Pro Lys Phe Val Met Glu Glu
        355                 360                 365

Val Tyr Asp Met Glu Leu Tyr Glu Trp Lys Glu Val Asp Ile Asn Asp
    370                 375                 380

Glu Ala Gln Lys Glu Arg Val Ser Gln Met Ile Glu Asp His Glu Pro
385                 390                 395                 400

Phe Glu Gly Gln Ala Leu Leu Asp Ala Lys Cys Phe Lys
                405                 410

<210>  7
<211>  1924
<212>  PRT
<213>  Lycopersicon esculentum

<400>  7

Met Ala Pro Asn Lys Asn Gly Arg Gly Lys Thr Lys Gly Asp Lys Lys
1               5                   10                  15

Lys Lys Glu Glu Lys Val Leu Pro Val Val Met Asp Ile Thr Ile Asn
            20                  25                  30

Leu Pro Glu Glu Thr Gln Val Ile Leu Lys Gly Ile Ser Thr Asp Arg
        35                  40                  45

Ile Ile Asp Val Arg Arg Leu Leu Ser Val Asn Thr Thr Thr Cys Asn
    50                  55                  60

Val Thr Asn Phe Ser Leu Ser His Glu Leu Arg Gly Pro Arg Leu Lys
65                  70                  75                  80

Glu Thr Val Asp Val Ser Ala Leu Lys Pro Cys Ile Leu Thr Leu Ile
                85                  90                  95

Glu Glu Glu Tyr Asp Glu Glu Ser Ala Thr Ala His Val Arg Arg Leu
        100                 105                 110

Leu Asp Ile Val Ala Cys Thr Thr Ser Phe Gly Pro Ser Gly Thr Ser
    115                 120                 125

Gly Lys Glu Leu Lys Thr Asp Ser Cys Lys Asn Ala Arg Gly Val Gln
    130                 135                 140

Asp Asn Lys Asn Ala Lys Lys Ser Asn Lys Val Arg Gly Asn Asp Lys
145                 150                 155                 160

Ser Ser Ser Pro Pro Gln Thr Pro Thr Pro Val Ala Gln Gln Leu Gly
                165                 170                 175

Lys Asp Ala Gly Ser Glu Glu Val Asp Gly Glu Met Ser Asn Thr Cys
            180                 185                 190

Pro Lys Ile Gly Ser Phe Tyr Glu Phe Phe Ser Leu Ser His Leu Thr
        195                 200                 205

Pro Pro Leu Gln Cys Asn Ala Pro Ser Leu Ser His Ile Val Ser Leu
    210                 215                 220

Phe Ser Cys Phe Leu Leu Lys Leu Arg Leu Ile Leu Ile Leu Ala Val
225                 230                 235                 240

Ile Arg Arg Ala Thr Arg Gln Gln Asp Asp Glu Val Leu Pro Asp Asp
                245                 250                 255

His Leu Phe Ser Leu Glu Val Lys Leu Cys Asn Gly Lys Leu Val Ile
            260                 265                 270

Val Glu Ala Cys Lys Lys Gly Phe Tyr Asn Phe Gly Lys Gln Gly Ile
        275                 280                 285

Leu Cys His Asn Leu Val Asp Leu Leu Arg Gln Leu Ser Arg Ala Phe
    290                 295                 300

Asp Asn Ala Tyr Asp Asp Leu Met Lys Ala Phe Leu Glu Arg Asn Lys
305                 310                 315                 320

Phe Gly Asn Leu Pro Tyr Gly Phe Arg Ala Asn Thr Trp Leu Ile Pro
                325                 330                 335

Pro Val Ala Ala Gln Leu Pro Ala Ile Phe Pro Pro Leu Pro Val Glu
            340                 345                 350

Asp Asp Thr Trp Gly Ala Asn Gly Gly Gly Leu Gly Arg Asp Gly Lys
        355                 360                 365

Phe Asp Ser Leu Pro Phe Ala Asn Glu Phe Leu Asn Val Ala Ser Met
    370                 375                 380

Ala Cys Lys Thr Thr Glu Glu Arg Gln Ile Arg Asp Arg Lys Ala Phe
385                 390                 395                 400

Ile Leu His Ser Leu Phe Val Asp Val Ala Ile Leu Arg Ala Ile Ser
                405                 410                 415

Ala Val Lys His Val Met Glu Lys Val Lys Pro Ala His Cys Asp Leu
            420                 425                 430

Asn Gly Glu Ile Ile Tyr Asn Glu Thr Val Gly Asp Leu Ser Ile Phe
        435                 440                 445

Val Thr Lys Asp Ser Ser Asn Ala Ser Cys Lys Val Asp Thr Lys Ile
    450                 455                 460

Asp Gly Phe Gln Ala Thr Gly Ile Ala Met Lys Asn Leu Met Glu Arg
465                 470                 475                 480

Asn Leu Leu Lys Gly Ile Thr Ala Asp Glu Asn Thr Ala Ala His Asp
                485                 490                 495

Ile Ala Thr Leu Gly Val Leu Asn Val Arg His Cys Gly Tyr Ile Ala
            500                 505                 510

Thr Val Lys Val Gln Gly Lys Glu Asn Asp Lys Val Gly Ser Pro Leu
        515                 520                 525

Gln Ser Met Glu Leu Ala Asp Gln Pro Asp Gly Gly Ala Asn Ala Leu
    530                 535                 540

Asn Ile Asn Ser Leu Arg Leu Leu Leu His Lys Lys Val Asp Asn Lys
545                 550                 555                 560

Val Met His Ser Lys Pro Ser Glu Thr Glu Glu Pro Asn Cys Ser Gln
                565                 570                 575

Ala Phe Val Arg Arg Ile Leu Glu Glu Ser Leu Thr Lys Leu Glu Glu
            580                 585                 590

Glu Lys Ile Glu Gly Asp Ser Phe Ile Arg Trp Glu Leu Gly Ala Cys
        595                 600                 605

Trp Ile Gln His Leu Gln Asp Gln Lys Lys Ser Glu Lys Asp Lys Lys
    610                 615                 620

Pro Ser Ala Glu Lys Lys Lys Asn Glu Met Lys Val Glu Gly Leu Gly
625                 630                 635                 640

Ile Pro Leu Lys Ser Leu Lys Asn Arg Lys Lys Ser Thr Asp Gly Thr
                645                 650                 655

Asn Met Glu Ser Gln Ser Glu Ser Phe Lys Ser Ala Ala Asp Gly Val
            660                 665                 670

Gly Gly Gly Ser Glu Lys Pro Val Leu Gln Ser Gly Glu Ser Gln Phe
        675                 680                 685

Glu Thr Asp Thr Asp Gln Asn Gln Val Val Leu Lys Ala Leu Leu Ser
    690                 695                 700

Asp Ala Gly Phe Thr Arg Leu Lys Glu Ser Glu Thr Gly Leu His Leu
705                 710                 715                 720

Lys Ser Leu Glu Glu Leu Ile Asp Leu Ser Gln Lys Tyr Tyr Asn Glu
                725                 730                 735

Val Ala Leu Pro Lys Leu Val Ala Asp Phe Gly Ser Leu Glu Leu Ser
            740                 745                 750

Pro Val Asp Gly Arg Thr Leu Thr Asp Phe Met His Thr Arg Gly Leu
        755                 760                 765

Arg Met Arg Ser Leu Gly Gln Val Val Lys Leu Ser Glu Lys Leu Ser
    770                 775                 780

His Val Gln Ser Leu Cys Ile His Glu Met Ile Val Arg Ala Phe Lys
785                 790                 795                 800

His Ile Leu Gln Ala Ala Ile Ala Ser Val Val Asp Ile Glu Asp Ile
                805                 810                 815

Ala Ala Ile Ile Ala Ala Ala Leu Asn Met Met Leu Gly Val Pro Glu
            820                 825                 830

Asn Asp Asp Ser Asn Glu Tyr Gly Val Asp Ser Leu Ile Trp Arg Trp
        835                 840                 845

Leu Lys Leu Phe Leu Lys Lys Arg Tyr Glu Trp Asp Val Gly Ser Leu

    850                 855                 860

Asn Tyr Lys Asp Met Arg Lys Phe Ala Ile Leu Arg Gly Leu Cys His
865                 870                 875                 880

Lys Val Gly Ile Glu Leu Val Pro Arg Asp Tyr Asp Met Ser Ser Ala
                885                 890                 895

Ser Pro Phe Gln Lys Val Asp Ile Val Ser Leu Val Pro Val His Lys
            900                 905                 910

Gln Ala Ala Cys Ser Ser Ala Asp Gly Arg Gln Leu Leu Glu Ser Ser
        915                 920                 925

Lys Thr Ala Leu Asp Lys Gly Lys Leu Glu Asp Ala Val Ser Tyr Gly
    930                 935                 940

Thr Lys Ala Leu Ala Lys Leu Val Ala Val Cys Gly Pro Tyr His Arg
945                 950                 955                 960

Met Thr Ala Gly Ala Tyr Ser Leu Leu Ala Val Val Leu Tyr His Thr
                965                 970                 975

Gly Asp Phe Asn Gln Ala Thr Ile Tyr Gln Gln Lys Ala Leu Asp Ile
            980                 985                 990

Asn Glu Arg Glu Leu Gly Leu Asp His Pro Asp Thr Met Lys Ser Tyr
        995                 1000                1005

Gly Asp Leu Ala Val Phe Tyr Tyr Arg Leu Gln His Thr Glu Leu
    1010                1015                1020

Ala Leu Lys Tyr Val Lys Arg Ala Leu Tyr Leu Leu His Leu Thr
    1025                1030                1035

Cys Gly Pro Ser His Pro Asn Thr Ala Ala Thr Tyr Ile Asn Val
    1040                1045                1050

Ala Met Met Glu Glu Gly Leu Gly Asn Val His Val Ala Leu Arg
    1055                1060                1065

Tyr Leu His Lys Ala Leu Lys Cys Asn Gln Lys Leu Leu Gly Pro
    1070                1075                1080

Asp His Ile Gln Thr Ala Ala Ser Tyr His Ala Ile Ala Ile Ala
    1085                1090                1095

Leu Ser Leu Met Glu Ala Tyr Pro Leu Ser Val Gln His Glu Gln
    1100                1105                1110

Thr Thr Leu Gln Ile Leu Arg Ala Lys Leu Gly Pro Asp Asp Leu
    1115                1120                1125

Arg Thr Gln Asp Ala Ala Ala Trp Leu Glu Tyr Phe Glu Ser Lys
    1130                1135                1140

Ala Phe Glu Gln Gln Glu Ala Ala Arg Asn Gly Thr Lys Lys Pro
    1145                1150                1155

Asp Ala Ser Ile Ala Ser Lys Gly His Leu Ser Val Ser Asp Leu
    1160                1165                1170

Leu Asp Tyr Ile Asn Pro Ser Pro Asp Ala Lys Gly Arg Asp Val
    1175                1180                1185

Gly Ser Lys Arg Arg Gly Phe Val Ser Lys Ala Leu Ile Ser Gln
    1190                1195                1200

Val Lys Gly Lys Ser Asp Gln Asn Asn Val Ala Ile Pro Asn Ser
    1205                1210                1215

Asp Thr Phe Lys Asp Val Pro Lys Glu Glu Thr Asp Glu Lys Lys
    1220                1225                1230

Gln Ile Val Glu Asp His Thr Asp Pro Lys Met Asn Met Glu Pro
    1235                1240                1245

Val Asp Thr Val Ile Glu Ser His His Asn Gly Asp Gly Gly Ile
    1250                1255                1260

Thr Glu Asn Lys Pro Ile Gln Ser Gly Pro Leu Leu Lys Glu Thr
    1265                1270                1275

Ser Ile Glu Lys Ser Met Val Arg Glu Val Leu Ser Glu Pro Ser
    1280                1285                1290

Ala Glu Ala Glu Asp Gly Trp Gln Pro Val Gln Arg Pro Arg Ser
    1295                1300                1305

Gly Gly Phe Tyr Gly Arg Arg Arg Arg Gln Arg Arg Gln Thr Ile
    1310                1315                1320

Ser Lys Val Ile Gly Tyr Gln Lys Lys Asp Pro Ile Ser Asp Val
    1325                1330                1335

Asp His Ala Lys Leu Lys Asn Asn Tyr Gln Ala Ser Lys Tyr Tyr
    1340                1345                1350

Val Leu Lys Lys Arg Thr Ser Pro Gly Ser Tyr Ala Asp Tyr Tyr
    1355                1360                1365

Leu Ala Lys Ser Gln Ala Ser Gly Thr Lys Leu Gly Arg Arg Val
    1370                1375                1380

Ile Lys Ala Val Ala Tyr Arg Val Lys Ser Val Ser Ser Ser Val
    1385                1390                1395

Arg Asp Ala Val Pro Glu Ile Ser Thr Thr Gly Gly Asp Leu Leu
    1400                1405                1410

Asn Thr Ser Ser Glu Gln Val Gln Val Ser Ala Thr Lys Glu Val
    1415                1420                1425

Gly Ser Leu Ser Lys Arg Ser Ser Ile Val Asn Leu Gly Lys Ser
    1430                1435                1440

Pro Ser Tyr Lys Glu Val Ala Leu Ala Pro Pro Gly Thr Ile Ser
    1445                1450                1455

Met Leu Gln Glu Arg Val Ser Glu Asp Glu Ile Pro Asp Asn Pro
    1460                1465                1470

Asp Val Met Lys Leu Glu Lys Glu Ser Asn Gly Ala Glu Glu Asn
    1475                1480                1485

Ser Lys Ile Met Gly Arg Asp Ala Glu Ser Met Glu Lys Glu Asn
    1490                1495                1500

Ile Gln Asp Leu Val Ala Asn Ser Ser Asp His Val Lys Ser Glu
    1505                1510                1515

Thr Val Asp Thr Asp Ser Lys Glu Glu Ile Gln Met Ser Asp Leu
    1520                1525                1530

Lys Gly Gly Glu Ile Ser Asp Leu Ile Ser Ala Asn Ala Ser Ile
    1535                1540                1545

Gln Pro Gly His Val Asp Val Ser Pro Met Glu Gln Gly Ser Val
    1550                1555                1560

Lys Thr His Asn Val Pro Thr Ser Asp Asn Ser Pro Lys Ala Asp
    1565                1570                1575

Pro Cys Glu Lys Asp Ser Ser Ser Asn Leu Asn Pro Gly Val Ile
    1580                1585                1590

Ser Asn Met Thr Leu Gln Asp Met Asp His Leu Lys Val Lys Ser
    1595                1600                1605

Ala Ser Ser His Ala Ser Asp Ala Ser Arg Glu Leu Ser Arg Lys
    1610                1615                1620

Leu Ser Ala Ser Ala Ala Pro Phe Ser Pro Ser Pro Ala Val Pro
    1625                1630                1635

Arg Gly Thr Pro Leu Pro Met Asn Ile Asn Leu Pro Ser Pro Pro
    1640                1645                1650

Gly Thr Arg Pro Pro Ile Gly Pro Trp Ser Val Thr Met Ser Leu
    1655                1660                1665

His Gln Gly Pro Pro Thr Ile Leu Pro Ser Pro Met Cys Ser Ser
    1670                1675                1680

Pro His His Leu Tyr Pro Ser Pro Pro His Thr Pro Asn Met Met
    1685                1690                1695

His Pro Leu Arg Phe Ile Tyr Pro Pro Tyr Ser Gln Pro Gln Thr
    1700                1705                1710

Leu Pro Pro Asn Thr Phe Pro Met Ser Ser Ser Thr Phe His Pro
    1715                1720                1725

Asn His Tyr Ala Trp Gln Cys Asn Ile Ala Pro Asn Ala Ser Glu
    1730                1735                1740

Tyr Val Pro Ala Thr Val Trp Pro Gly Cys His Pro Val Glu Phe
    1745                1750                1755

Ser Ile Ser Pro Pro Val Ile Glu Pro Ile Thr Asp Ser Ile Ser
    1760                1765                1770

Ser Ala Lys Glu Ile Ser Asp Asn Pro Glu Asn Ile Thr Leu Thr
    1775                1780                1785

Thr Ser Leu Leu Val Asp Leu Asn Thr Gly Asp Glu Val Lys Glu
    1790                1795                1800

Asp Val Asn Leu Pro Ala Ser Glu Thr Val Glu Asn Ile Ala Ala

1805                    1810                    1815

Val Val  Pro Glu Lys Glu Arg  Ala Ser Asn Thr Pro  Asp Ser His
    1820                    1825                    1830

Phe Val  Thr Ser Ser Ser Asp  Gln Ser Lys Glu Gly  Ser Gly Ser
    1835                    1840                    1845

Asn His  Val Gln Arg Asn Leu  Thr Glu Thr Asp Asn  Glu Lys Thr
    1850                    1855                    1860

Phe Asn  Ile Leu Val Arg Gly  Arg Arg Asn Arg Lys  Gln Thr Leu
    1865                    1870                    1875

Arg Met  Pro Ile Ser Leu Leu  Lys Arg Pro Tyr Ser  Ser Gln Pro
    1880                    1885                    1890

Phe Lys  Ala Val Tyr Ser Arg  Val Ile Arg Glu Thr  Glu Val Pro
    1895                    1900                    1905

Ser Ser  Thr Ser Phe Asp Pro  His Glu His Gly Ile  Thr Thr Ala
    1910                    1915                    1920

Thr

<210>  8
<211>  250
<212>  PRT
<213>  Lycopersicon esculentum

<400>  8

Met Gly Asp Leu His Ser Ile Ala Tyr Asn Ser Phe Ser Ser Leu Gly
1               5                   10                  15

Cys Asp Phe His Gly Leu Glu Val Leu Asn Asn Asp Met Ser Pro Phe
            20                  25                  30

Met Glu Ser Thr Pro Pro Phe Leu Ser Asn Phe Asp Cys Glu Leu Ser
        35                  40                  45

Ser Gly Tyr Leu Gln Asp Ala Leu Phe Lys Phe Asn Ser Lys Arg Arg
    50                  55                  60

Arg Leu Phe Leu Phe Asn Asp Asp Asp Asp Lys Glu Asn Tyr Gln Asn
65                  70                  75                  80

Lys Asp Ser Ile Lys Asn Leu Trp Ser Ser Thr Ile Asp Gln Gln Phe

85                  90                  95

Ser Glu Asp Tyr Asp Ser Phe Ser Gln Ile Thr Lys Cys Asp Ser Phe
            100                 105                 110

Ser Gly Asp Pro Met Ser Lys Met Ser Glu Glu Tyr Ser Lys Arg Thr
        115                 120                 125

Glu Glu Glu Ala Ile Ser Glu Asn Tyr Tyr Tyr Ser Ser Asn Ser Ser
    130                 135                 140

Pro Thr Ser Ser Ser His His Asn Lys Gln Pro Leu Gln Tyr Gly Gly
145                 150                 155                 160

Gly Asp Lys Lys Arg Arg Met Cys Gly Lys Ile Val Tyr Pro Phe Gly
                165                 170                 175

Leu Val Lys Pro Gly Gly Gln Glu Gly Asp Val Thr Leu Asn Asp Ile
            180                 185                 190

Asn Glu Arg Ile Leu Met Arg Pro Thr Arg Pro Val Arg His Pro Val
        195                 200                 205

Gly Asp Phe Ala Cys Arg Ser Thr Val Cys Pro Ala Gly Pro Gly Leu
    210                 215                 220

Ser Gly Lys Thr Val Val Ala Leu Thr Lys Ile His Thr Gln Gly Arg
225                 230                 235                 240

Gly Thr Ile Thr Ile Ile Arg Thr Arg Gly
                245                 250

<210>  9
<211>  969
<212>  PRT
<213>  Capsicum annuum

<400>  9

Met Asn Val Ser Ser Ala Arg Glu Met Asn Asp Ser Leu Ala Ile Thr
1               5                   10                  15

Ala Ser Ser Leu Ala Ile Thr Glu Lys Lys Pro Gln Arg Pro Gly Gly
            20                  25                  30

Cys Val Gly Ile Phe Phe Gln Leu Phe Asp Trp Asn Arg Arg Phe Ala
        35                  40                  45

Lys Lys Lys Leu Phe Pro Lys Lys Leu Leu Ser Pro Asp Arg Leu Lys

50                  55                  60

Gln Ala Ser Lys Lys Phe Gly Gly Asp Glu Lys Gln Pro Lys Ile Arg
65                  70                  75                  80

Leu Ile Ala Asn Glu Asn Ser Gly Gly Phe Pro Asn Val Lys Asn Asn
                85                  90                  95

Gly Met Thr Asn Thr Cys Cys Glu Ser Lys Arg Lys Met Lys Ala Pro
            100                 105                 110

Ser Leu Val Ala Arg Leu Met Gly Leu Glu Ser Met Pro Ala Ala Ser
        115                 120                 125

Gly Ser Lys Pro Lys Lys Ala Ser Gly Ser Glu Ile Gly Ser Asn Val
    130                 135                 140

Ala Glu Lys Leu Gly Gly Gln Pro Gly Gly Ser Asp Lys Glu Asp Met
145                 150                 155                 160

Asp Phe Glu Lys Ala Glu Ile Lys Arg Glu Leu Arg Pro Gln Lys Leu
                165                 170                 175

Gln Lys Ile Gly Leu Ser Glu Arg Arg Ser Val Cys Arg Phe Ser Ala
            180                 185                 190

Glu Ala Leu Gln Leu Arg Thr Val Leu Ser Arg Pro Arg Lys His Gln
        195                 200                 205

Pro Lys Leu Val Ser Pro Val Lys Ser Pro Arg Asn Val Ser Gly Arg
    210                 215                 220

Asn Ala Ser Arg Leu Ile Gly Ala Ala Thr Arg Ile Leu Glu Pro Gly
225                 230                 235                 240

Leu Gln Lys Ser Arg Val Lys Cys Ala Leu Thr Tyr Pro Thr Arg Tyr
                245                 250                 255

Phe Ser Pro Leu Glu Asp Lys Ala Asp Leu Ala Pro His His Leu Glu
            260                 265                 270

Gly Pro Asn Pro Cys Val Asp Ser Lys Thr Leu Lys Gly Thr Ser Val
        275                 280                 285

Pro Ser Cys Lys Asn Cys Gly Tyr Phe Leu His Ser Lys Asn Gly Ile
    290                 295                 300

Pro Asn Val Glu Glu His Ser Ser Ser Val Ser Ser Pro Ile Ser Ser
305                 310                 315                 320

Tyr Ser Gly Pro Pro Cys His Gly Pro Gly Arg Asn Ile Pro Lys Leu
                325                 330                 335

Pro Ile Phe Ser Ser Arg Asp Gln Leu Glu Arg Val Ser Glu Gly Ser
            340                 345                 350

Ser Ser Asp Ala Thr Ala Glu Ile Asp Asp Val Ser Tyr Cys Ala Glu
        355                 360                 365

Leu Ile Leu Gly Lys Arg Ser Ile Ser Arg Ser Arg Met Glu Met His
    370                 375                 380

Gly Thr Arg Gln Gly Asn Asn Val Lys Lys Asp Ala Ser Pro Val Thr
385                 390                 395                 400

His Val Leu Asn Gln Lys Gln Asn Gln Thr Ser Gln Asn Arg Glu Arg
                405                 410                 415

Gly Leu Met Lys Ser Lys Pro Ser Ser Leu Gln Ser Asn Arg Ile Leu
            420                 425                 430

Ala Ala Ala Glu Ser Met Asn Asn Thr Lys Asn Phe Val Ala Gln Asn
        435                 440                 445

Arg Arg Leu Gly Ala Ser Thr Arg Leu Arg Met Pro Ala Thr Ala Asp
    450                 455                 460

Gly Cys Lys Phe Glu Thr Glu Arg Lys Pro Tyr Ser Ser Arg Ser Asp
465                 470                 475                 480

Ser Phe Ser Pro Val Arg Lys Lys Arg Leu Asn Asn Val Ser Arg Gln
                485                 490                 495

Gly Asp Ser Ser Ser Phe Val Asn Ala Asn Met Gly Gly Glu Ser Ser
            500                 505                 510

Pro Ser Ser Asp Lys Thr Ser Arg Lys Asp Val Ala Phe Pro Ile Cys
        515                 520                 525

Ser Val Asp Ser His Ser Thr Lys Pro Lys Leu Pro Cys Leu Arg Glu
    530                 535                 540

Gly Val Thr Thr Asn Asn Ser Ser Glu Gly Cys Asn Val Val Ser Phe
545                 550                 555                 560

Thr Phe Lys Ser Ala Met Lys Gln Lys Ala Gly Thr His Ala Glu Val
                565                 570                 575

Thr Lys Arg Lys Ser Gln Asn Tyr Ser Ser Val Asp Ala Thr Arg Gly
            580                 585                 590

Arg Ser Phe Phe Lys Gly Asn Asp Glu Ala Pro Cys Leu His Lys Ser
        595                 600                 605

Phe Pro Leu Lys Gly Asp Ile Leu Gly Ala Leu Leu Glu Gln Lys Leu
    610                 615                 620

Lys Glu Leu Thr Ser Glu Glu Glu Phe Gly Glu Gly Gly Ala Ala Pro
625                 630                 635                 640

Arg Lys Ser Thr Ala Thr Ile Leu Gln Glu Leu Ile Thr Ala Leu Asn
                645                 650                 655

Glu Glu Lys Gln Phe His Leu Asp Ser Ser Pro Val Arg Pro Ser Arg
            660                 665                 670

Lys Glu Asp Leu Cys Asp Asp Gly Asp Val Ser Ser Arg Asn Thr Cys
        675                 680                 685

Met Asn Phe Gln Ala Ile Pro Asp Ser Ala Ala Thr Asp Leu Val Gly
    690                 695                 700

Asn Ser Leu Asp His His Ser Pro Gly Cys Val Leu Glu Ala Ala Phe
705                 710                 715                 720

Ser Thr Asp Ser Tyr Leu Ser Ser Ser Pro Asn Ser Ser Ser Lys Asp
                725                 730                 735

Lys Val Leu Ala Gly Ser Val Asp Ser Ile Asp Asp Glu Pro Leu Phe
            740                 745                 750

Pro Glu Pro Asp Arg Asp Leu Ser Asp Cys Glu Thr Ser Leu Ser Thr
        755                 760                 765

Arg Ser Cys Arg Ala Leu Ile Thr Asp His Phe Asn Asn Val Ser Gly
    770                 775                 780

Leu Leu Ser Lys Ile Asp Gln Leu Lys Gly Ser Lys Leu Ser Tyr Ala
785                 790                 795                 800

Lys Glu Val Ile Leu Asn Thr Glu Leu Ile Phe Arg Thr Thr Pro Gln
                805                 810                 815

Gln Gln Ala Leu Pro Val Glu Asp Gly Phe Ser Val Ser His Phe Leu
            820                 825                 830

Leu Asn Glu Leu Glu Met Leu Ser Ser Leu Leu Trp Met Thr Phe Ser
        835                 840                 845

Gln Leu Leu Gly Cys Asn Asp Pro Lys Gln Met Asn Gln Leu Lys Gly
    850                 855                 860

Phe Ala Phe Asp Cys Leu Leu Glu Tyr Leu Asp Ser Lys Phe Ala Arg
865                 870                 875                 880

Tyr Ser Asp Ser Gly Phe Arg Thr Trp Thr Lys Leu Pro Ser Ser Met
                885                 890                 895

Thr Lys Glu Ile Leu Ile Ala Asp Ile Ile Glu Glu Val Lys Glu Trp
            900                 905                 910

Thr Glu Phe Val Gly Leu Ile Pro Asp Glu Leu Ile Glu Trp Asp Met
        915                 920                 925

Ser His Ser Leu Gly Lys Trp Thr Asp Phe Glu Ile Glu Glu Phe Glu
    930                 935                 940

Cys Gly Thr Glu Val Ala Arg His Ile Leu Gln Glu Leu Val Asp Glu
945                 950                 955                 960

Val Val Leu Asp Leu Tyr Ser Phe Ser
                965

<210>  10
<211>  569
<212>  PRT
<213>  Capsicum annuum

<400>  10

Met Ala Gly Glu Lys Ile Ile Glu Lys Ser Glu Ala Val Ala Pro Gly
1               5                   10                  15

Leu Lys Ser Asp Ser Ser Thr Lys Leu Thr Glu Lys Asp Leu Val Ser
            20                  25                  30

Lys Asn Asp Gly Lys Val Ser Asp Ser Val Ser Ser Leu Gly Ala Val
        35                  40                  45

Thr Gly Ile Lys Gly Glu Ile Asp Gln Pro Pro Val Ala Glu Gln Gly
    50                  55                  60

Ala Tyr Tyr Pro Pro Thr Ser Tyr Cys Asp Tyr Tyr Tyr Pro Gly Tyr
65                  70                  75                  80

Asn Gly Thr Tyr Asn Gln Leu Asp Asp Gln Ala Tyr Phe Asn Ser Gly
                85                  90                  95

Val Gln Ser Glu Asn Gly Ser Leu Leu Tyr Tyr Met Pro Gly Tyr Asn
            100                 105                 110

Pro Tyr Gly Ala Gly Tyr Ile Gly Gly Asp Gly Lys Gln Pro Tyr Leu
        115                 120                 125

Ser Ser Gly Tyr Leu Gln Gln Pro Val Ser Tyr Gly Ser Asp Ser Met
    130                 135                 140

Pro Cys Tyr Ser Trp Gly Gly Ser Thr Tyr Cys Ala Asp Ile Thr Asn
145                 150                 155                 160

Ser Ala Ala Pro Lys Ser Gly Asn Val Lys Ser Thr Phe Gly Arg Asn
                165                 170                 175

Gly Ser Val Arg Ser Asn Gly Phe Asn Pro Thr Lys Thr Asn Gly Ser
            180                 185                 190

Phe Ser Ser Lys Asn Ser Thr Met Leu Phe Asn Pro Lys Asn Arg Pro
        195                 200                 205

Ser Thr Ala Ile Ser Asn Pro Pro Lys Ser Ile His Gln Ala Gln Pro
    210                 215                 220

Phe Asn Pro Ala Asn Lys Ser Asp Val Gln Ser Gly Gly Leu Thr Lys
225                 230                 235                 240

Gly Tyr His Met Val Gly Asp Phe Pro Ser Tyr Ser Ser Gln Asn Gln
                245                 250                 255

Gly Phe Phe Met Pro Tyr Asp Pro Ile His Tyr Gln Thr Asn Ser Arg
            260                 265                 270

Leu Trp Asn Gly Asn Tyr Arg Gly Lys Pro Arg Gly Asn Phe Thr Arg
        275                 280                 285

Asn Gly Val Phe Glu Ala Ser Asn Glu Leu Pro Arg Gly Pro Arg Ala
    290                 295                 300

Asn Ser Arg Ser Ala Pro Ser Lys Pro Ser Ala Glu Glu Asp Gln Leu

305                 310                 315                 320

Glu Pro Thr Ile Gln Arg Glu Lys Tyr Asn Lys Glu Asp Phe Lys Thr
                325                 330                 335

Gln Tyr Asp Asn Ala Lys Phe Tyr Ile Ile Lys Ser Tyr Ser Glu Asp
            340                 345                 350

Asp Ile His Lys Cys Val Lys Tyr Ala Val Trp Ser Ser Thr Pro Asn
        355                 360                 365

Gly Asn Lys Lys Leu Asp Thr Ala Phe Leu Asp Ala Glu Gly Lys Ala
    370                 375                 380

Ser Gly Thr Asp Ser Ser Cys Pro Val Phe Leu Phe Phe Ser Val Asn
385                 390                 395                 400

Gly Ser Gly Gln Phe Leu Gly Val Ala Glu Met Val Gly Gln Val Asp
                405                 410                 415

Phe Asn Arg Asn Met Asp Phe Trp Gln Leu Asp Lys Trp Ser Gly Phe
            420                 425                 430

Phe Pro Val Lys Trp His Ile Val Lys Asp Val Pro Asn Thr Gln Phe
        435                 440                 445

Arg His Ile Ile Leu Lys Asn Asn Asp Asn Arg Pro Val Thr Tyr Ser
    450                 455                 460

Arg Asp Thr Gln Glu Ile Gly Leu Lys Glu Gly Leu Glu Met Leu Asn
465                 470                 475                 480

Ile Leu Lys Asn Tyr Ser Glu Lys Thr Ser Ile Leu Asp Asp Phe Asn
                485                 490                 495

Phe Tyr Glu Lys Arg Glu Lys Val Leu Lys Ala Lys Arg Ser Ser Lys
            500                 505                 510

Pro Ala Ile Gln Ala Asp Val Phe Glu Lys Ala Asp Ser Val Arg Gln
        515                 520                 525

Phe Lys Gly Gly Asp Lys Val Arg Glu Glu Glu Leu Lys Ser Asn Ser
    530                 535                 540

Thr Asp Pro Thr Ala Ser Leu Val Ser Leu Ala Lys Asn Leu Ser Ile
545                 550                 555                 560

Asn Ser Arg Pro Phe Lys Ser Ser Val
                565

<210>  11
<211>  475
<212>  PRT
<213>  Capsicum annuum

<400>  11

Met Glu Glu Lys Leu Val Gln Arg Leu Glu Ser Ala Val Ala Arg Leu
1               5                   10                  15

Glu Ala Leu Ser Ala Thr Gly Phe Arg Ser Gly Gly Ser Val Val Ala
            20                  25                  30

Gly Gly Asp Gly Thr Ala Val Leu Asp Pro Ser Ile Ile Ala Phe Asp
        35                  40                  45

Asp Leu Arg Ser Gln Phe Leu Ser Lys Val Leu Ser Ala Ala Glu Lys
    50                  55                  60

Ile Gly Gly Gln Val Leu Asp Val Ser Lys Ile Val Glu Glu Ala Phe
65                  70                  75                  80

Glu Ala Gln Arg Glu Leu Leu Ile Lys Ile Lys Glu Thr Lys Lys Pro
                85                  90                  95

Asp Asn Ser Gly Leu Val Glu Phe Leu Lys Pro Leu Asn Asp Val Ile
            100                 105                 110

Val Lys Ala Thr Lys Met Thr Glu Gly Arg Arg Ser Asp Phe Phe Asn
        115                 120                 125

His Leu Lys Ser Ala Val Asp Ser Leu Ser Ala Leu Ala Trp Ile Ala
    130                 135                 140

Tyr Ile Gly Lys Asp Cys Gly Met Ser Met Pro Ile Ala Phe Val Glu
145                 150                 155                 160

Glu Ser Trp Gln Met Ala Glu Phe Tyr Asn Asn Lys Ile Leu Val Glu
                165                 170                 175

Phe Lys Asn Lys Asp Pro Asn His Val Glu Trp Ala Lys Ala Leu Lys
            180                 185                 190

Glu Leu Tyr Leu Pro Gly Leu Arg Asp Tyr Val Lys Ser His Tyr Pro
        195                 200                 205

Leu Gly Pro Val Trp Ser Ala Thr Gly Lys Pro Ala Val Ser Ala Pro
    210                 215                 220

Ser Lys Ala Pro Ser Pro Ser Ala Pro Ala Pro Pro Val Pro Pro Pro
225                 230                 235                 240

Ala Ser Leu Phe Ser Ser Glu Ser Pro Gln Ala Ser Ser Ser Arg Pro
                245                 250                 255

Lys Gln Gly Met Ser Ala Val Phe Asp Glu Ile Asn Ser Gly Lys Pro
            260                 265                 270

Val Thr Ser Gly Leu Arg Lys Val Thr Asp Asp Met Lys Thr Lys Asn
        275                 280                 285

Arg Phe Asp Arg Thr Gly Val Val Asn Ala Gly Asp Lys Glu Ala Arg
    290                 295                 300

Met Ser Ser Pro Pro Val Ser Lys Thr Gly Pro Pro Lys Leu Glu Leu
305                 310                 315                 320

Gln Met Gly Arg Lys Trp Val Val Glu Asn Gln Val Gly Gln Lys Asn
                325                 330                 335

Leu Val Ile Asp Asp Cys Asp Thr Lys Gln Ser Val Tyr Val Tyr Gly
            340                 345                 350

Cys Lys Asp Ser Val Leu Gln Ile Lys Gly Lys Val Asn Asn Leu Thr
        355                 360                 365

Ile Asp Lys Cys Thr Lys Met Gly Ile Val Phe Ala Gly Val Val Ala
    370                 375                 380

Ala Cys Glu Val Val Asn Cys Asn Ser Val Glu Val Gln Cys Gln Gly
385                 390                 395                 400

Ser Ala Pro Thr Ile Ser Ile Asp Asn Thr Asn Gly Cys Gln Leu Tyr
                405                 410                 415

Leu Asn Lys Asp Ser Leu Glu Gly Ser Ile Thr Thr Ala Lys Ser Ser
            420                 425                 430

Glu Ile Asn Val Leu Val Pro Gly Ala Gly Pro Asp Asp Asp Trp Gly
        435                 440                 445

Glu His Ala Leu Pro Gln Gln Tyr Ala His Glu Tyr Lys Asp Gly His
    450                 455                 460

Phe Val Thr Thr Pro Val Ser His Ser Gly Ala
465                 470                 475

<210>  12
<211>  472
<212>  PRT
<213>  Capsicum annuum

<400>  12

Met Asp Glu Lys Leu Ile Gln Arg Leu Glu Ser Ala Val Ile Arg Leu
1               5                   10                  15

Glu Ala Leu Ser Val Gly Gly Gly Ala Ser Val Gly Asp Gly Val Ala
            20                  25                  30

Ala Ala Ala Leu Asp Pro Ser Ile Ile Ala Phe Asp Asp Leu Arg Ser
        35                  40                  45

Gln Phe Val Gly Arg Val Leu Gly Ala Ala Glu Lys Ile Gly Gly Gln
    50                  55                  60

Val Leu Asp Val Thr Lys Ile Val Glu Glu Ala Phe Glu Ala Gln Arg
65                  70                  75                  80

Glu Leu Leu Ile Lys Ile Lys Glu Thr Gln Lys Pro Ser Met Ser Gly
                85                  90                  95

Leu Pro Glu Phe Leu Lys Pro Leu Asn Asp Val Ile Leu Lys Ala Thr
            100                 105                 110

Lys Met Thr Glu Gly Trp Arg Leu Asp Phe Phe Asn His Leu Lys Ser
        115                 120                 125

Ala Val Asp Ser Leu Ile Ala Leu Ala Trp Ile Ala Tyr Thr Gly Lys
    130                 135                 140

Asp Cys Gly Met Ser Met Pro Ile Ala His Val Glu Glu Ser Trp Gln
145                 150                 155                 160

Met Ala Glu Phe Tyr Asn Asn Lys Val Leu Val Glu Phe Arg Asn Lys
                165                 170                 175

Asp Pro Asn His Ile Glu Trp Ala Lys Ala Leu Lys Glu Leu Tyr Leu
            180                 185                 190

Pro Gly Leu Trp Asp Tyr Val Lys Ser His Tyr Pro Leu Gly Pro Val
        195                 200                 205

Trp Ser Ala Thr Gly Lys Val Ala Val Ser Ala Pro Ser Lys Ala Pro
    210                 215                 220

Leu Pro Thr Ser Arg Pro Met Pro Pro Pro Leu Pro Pro Ala Ser Leu
225                 230                 235                 240

Phe Ser Ser Gly Ser Phe Gln Ala Ser Ser Ser Arg Pro Lys Lys Gly
                245                 250                 255

Met Ala Ala Val Phe Asp Glu Ile Asn Ser Gly Lys Pro Val Thr Thr
            260                 265                 270

Gly Leu Arg Lys Val Thr Asp Asp Met Lys Thr Lys Asn Arg Ala Asp
        275                 280                 285

Arg Thr Gly Val Ile Ser Ala Gly Glu Lys Glu Gly Arg Leu Ser Ser
    290                 295                 300

Pro Ser Phe Ser Lys Ala Gly Pro Pro Lys Leu Glu Leu Gln Met Gly
305                 310                 315                 320

Arg Lys Trp Met Val Glu Asn Gln Ile Gly Arg Lys Asn Leu Val Ile
                325                 330                 335

Asp Asp Cys Asp Ala Lys Gln Ser Val Tyr Ile Phe Gly Cys Lys Asp
            340                 345                 350

Ser Val Leu Gln Ile Gln Gly Lys Val Asn Asn Ile Thr Ile Asp Lys
        355                 360                 365

Cys Thr Lys Met Gly Val Val Phe Thr Asp Val Val Ala Ala Cys Glu
    370                 375                 380

Val Val Asn Cys Asn Gly Val Glu Met Gln Cys Gln Gly Ser Ala Pro
385                 390                 395                 400

Thr Ile Ser Val Asp Asn Thr Thr Gly Cys Gln Leu Tyr Leu Gly Lys
                405                 410                 415

Asp Ser Ser Glu Thr Ser Ile Thr Thr Ala Lys Ser Ser Glu Ile Asn
            420                 425                 430

Val Leu Val Pro Gly Ala Gly Pro Asp Ser Asp Trp Gly Glu His Ala
        435                 440                 445

Leu Pro Gln Gln Phe Ser His Val Tyr Lys Asn Gly Gln Phe Val Thr
    450                 455                 460

Thr Ser Val Ser His Ser Gly Gly
465                 470

<210>  13
<211>  489
<212>  PRT
<213>  Capsicum annuum

<400>  13

Met Ala Pro Gly Ile Gly Gln Glu Arg Glu Ile Gln Lys Ser Tyr Trp
1               5                   10                  15

Ile Glu Lys Thr Ala Gly Leu Lys Leu Glu Ala Met Met His Val His
            20                  25                  30

Asp Leu Glu Ser Ser Asp Leu Asp Lys Glu Asp Arg His Glu Val Leu
        35                  40                  45

Ser Leu Leu Pro Ser Tyr Glu Gly Lys Ser Val Leu Glu Leu Gly Ala
    50                  55                  60

Gly Ile Gly Arg Phe Thr Gly Asp Leu Ala Lys Lys Ala Gly Glu Leu
65                  70                  75                  80

Val Ala Val Asp Phe Ile Glu Gly Val Ile Lys Glu Asn Glu Ser Ile
                85                  90                  95

Ser Gly His His Lys Asn Val Lys Phe Lys Cys Ala Asp Val Thr Ser
            100                 105                 110

Pro Asp Leu Thr Phe Ser Pro Glu Ser Val Asp Leu Ile Phe Ser Asn
        115                 120                 125

Trp Leu Leu Met Tyr Leu Ser Asp Glu Glu Val Gln Asp Leu Ala Glu
    130                 135                 140

Arg Met Met Thr Trp Leu Lys Val Gly Gly Tyr Ile Phe Phe Arg Glu
145                 150                 155                 160

Thr Cys Phe His Gln Ser Gly Asp Asn Lys Gly Lys Asn Asn Pro Thr
                165                 170                 175

His Tyr Arg Glu Pro Arg Phe Tyr Thr Lys Val Phe Gln Glu Cys Asn
            180                 185                 190

Ala Gly Lys Ser Tyr Glu Leu Ser Leu Val Gly Tyr Lys Cys Met Glu
        195                 200                 205

Thr Tyr Val Arg Asn Lys Arg Asn Gln Asn Glu Ile Cys Trp Ile Trp
    210                 215                 220

Gln Lys Val Arg Ser Asp Asp Asp Arg Gly Phe Gln His Phe Leu Asp
225                 230                 235                 240

Thr Val Gln Tyr Lys Ser Asn Gly Ile Leu Arg Tyr Glu Arg Val Phe
                245                 250                 255

Gly Lys Asp Phe Val Ser Thr Gly Gly Ile Asp Thr Thr Lys Glu Phe
            260                 265                 270

Val Ala Met Leu Asp Leu Gln Pro Gly Glu Lys Val Leu Asp Val Gly
        275                 280                 285

Cys Gly Ile Gly Gly Gly Asp Phe Tyr Met Ala Glu Lys Tyr Asp Val
    290                 295                 300

His Val Leu Gly Ile Asp Leu Ser Val Asn Met Ile Ser Leu Ser Phe
305                 310                 315                 320

Glu Arg Ala Ile Gly Arg Lys Cys Ala Val Glu Phe Glu Val Ala Asp
                325                 330                 335

Cys Thr Val Lys Thr Tyr Pro Glu Gly Ser Phe Asp Val Ile Tyr Ser
            340                 345                 350

Arg Asp Thr Ile Leu His Ile Gln Asp Lys Pro Ala Leu Phe Gln Ser
        355                 360                 365

Phe Tyr Lys Trp Leu Lys Pro Gly Gly Arg Val Leu Ile Ser Asp Tyr
    370                 375                 380

Cys Lys Lys Ala Gly Pro Ala Ser Glu Glu Phe Ala Ala Tyr Ile Lys
385                 390                 395                 400

Gln Arg Gly Tyr Asp Leu His Asp Val Glu Ala Tyr Gly Gln Met Leu
                405                 410                 415

Arg Asp Ala Gly Phe Asn Glu Val Ile Ala Glu Asp Arg Thr Glu Leu
            420                 425                 430

Phe Met Lys Val Leu Gln Lys Glu Leu Asp Ile Val Glu Lys Glu Arg
        435                 440                 445

Glu Ser Phe Ile Gln Glu Phe Ser Glu Gln Asp Tyr Asn Asp Ile Val

450                 455                 460

Gly Gly Trp Lys Ala Lys Leu Val Arg Ser Ser Ser Gly Glu Gln Arg
465                 470                 475                 480

Trp Gly Leu Phe Ile Ala Lys Lys Asn
                485

<210>  14
<211>  491
<212>  PRT
<213>  Capsicum annuum

<400>  14

Met Asp Val Ala Ser Gly Gln Glu Arg Glu Val Gln Lys Ser Tyr Trp
1               5                   10                  15

Ile Glu His Thr Ala Glu Leu Thr Val Glu Ala Met Met Leu Asp Ser
            20                  25                  30

Lys Ala Ala Asp Leu Asp Lys Glu Glu Arg Pro Glu Val Leu Ser Leu
        35                  40                  45

Leu Pro Ala Tyr Glu Gly Lys Thr Val Leu Glu Leu Gly Ala Gly Ile
    50                  55                  60

Gly Arg Phe Thr Gly Glu Leu Ala Lys Lys Ala Gly Gln Leu Ile Ala
65                  70                  75                  80

Leu Asp Phe Ile Glu Gly Ala Ile Lys Lys Asn Glu Ser Ile Asn Gly
                85                  90                  95

Cys His Lys Asn Val Lys Phe Met Cys Ala Asp Val Thr Ser Pro Asp
            100                 105                 110

Leu Asn Phe Ser Pro Glu Ser Val Asp Leu Ile Phe Ser Asn Trp Leu
        115                 120                 125

Leu Met Tyr Leu Ser Asp Glu Glu Val Gln Asp Leu Ala Lys Arg Met
    130                 135                 140

Val Lys Trp Leu Lys Val Gly Gly Tyr Ile Phe Phe Arg Glu Ser Cys
145                 150                 155                 160

Phe His Gln Ser Gly Asp His Lys Arg Lys Asn Asn Pro Thr His Tyr
                165                 170                 175

Arg Asp Pro Ser Phe Tyr Thr Lys Val Phe Arg Glu Cys His Val Asn

180                 185                 190

Ala Gly Asp Gly Lys Ser Phe Glu Leu Ser Leu Gly Gly Cys Lys Cys
        195                 200                 205

Ile Gly Ala Tyr Val Arg Asn Lys Lys Asn Gln Asn Gln Ile Cys Trp
    210                 215                 220

Ser Trp Gln Lys Val Gly Ser Glu Asn Asp Met Glu Phe Gln Arg Phe
225                 230                 235                 240

Leu Asp Thr Val Gln Tyr Lys Cys Ser Gly Ile Leu Arg Tyr Glu Arg
                245                 250                 255

Val Phe Gly Gln Gly Phe Val Ser Thr Gly Gly Leu Glu Thr Thr Lys
            260                 265                 270

Glu Phe Val Ala Met Leu Asp Leu Gln Pro Gly Gln Lys Val Leu Asp
        275                 280                 285

Val Gly Cys Gly Ile Gly Gly Gly Asp Phe Tyr Met Ala Glu Lys Tyr
    290                 295                 300

Asp Val His Val Val Gly Ile Asp Leu Ser Ile Asn Met Ile Ser Phe
305                 310                 315                 320

Ala Leu Glu Arg Ala Ile Gly Leu Lys Cys Ala Val Glu Phe Glu Val
                325                 330                 335

Ala Asp Cys Thr Lys Lys Thr Tyr Pro Val Gly Thr Phe Asp Val Ile
            340                 345                 350

Tyr Ser Arg Asp Thr Ile Leu His Ile Gln Asp Lys Pro Ala Leu Phe
        355                 360                 365

Arg Ser Phe Tyr Lys Trp Leu Lys Pro Gly Gly Lys Val Leu Ile Ser
    370                 375                 380

Asp Tyr Cys Lys Arg Ala Gly Pro Ala Ser Glu Glu Phe Ala Gly Tyr
385                 390                 395                 400

Ile Lys Gln Arg Gly Tyr Asp Leu His Asp Val Glu Ala Tyr Gly Gln
                405                 410                 415

Met Leu Arg Asp Ala Gly Phe His Asp Val Val Ala Glu Asp Arg Thr
            420                 425                 430

Glu Gln Phe Ile Gly Val Leu Gln Lys Glu Leu Asp Thr Val Glu Lys
        435                 440                 445

Gly Arg Glu Ser Phe Ile His Glu Phe Ser Glu Gln Asp Tyr Asn Glu
    450                 455                 460

Ile Val Gly Gly Trp Lys Ala Lys Leu Ile Arg Ser Ser Ser Gly Glu
465                 470                 475                 480

Gln Arg Trp Gly Leu Phe Val Ala Lys Lys Asn
                485                 490

<210>  15
<211>  271
<212>  PRT
<213>  Capsicum annuum

<400>  15

Met Leu Cys Trp Leu Trp Gln Lys Val Asn Ser Glu Asp Asp Arg Gly
1               5                   10                  15

Phe Gln Arg Phe Leu Asp Asn Ala Gln Tyr Lys Cys Ser Gly Ile Leu
            20                  25                  30

Arg Tyr Glu Arg Val Phe Gly Glu Gly Tyr Val Ser Thr Gly Gly Ile
        35                  40                  45

Asp Thr Thr Lys Glu Phe Val Ser Met Leu Asp Leu Gln Pro Gly Gln
    50                  55                  60

Lys Val Leu Asp Val Gly Cys Gly Ile Gly Gly Gly Asp Phe Tyr Met
65                  70                  75                  80

Ala Glu Asn Tyr Asp Val His Val Val Pro Ile Asp Leu Ser Ile Asn
                85                  90                  95

Met Ile Ser Phe Ala Leu Glu Arg Ala Ile Gly Leu Lys Cys Ala Val
            100                 105                 110

Lys Phe Glu Val Ala Asp Cys Thr Lys Lys Thr Tyr Pro Asp Gly Thr
        115                 120                 125

Phe Asp Val Ile Tyr Ser Arg Asp Thr Ile Leu His Ile Gln Asp Lys
    130                 135                 140

Leu Ala Leu Phe Arg Ser Phe Tyr Arg Trp Leu Lys Pro Gly Gly Lys
145                 150                 155                 160

Val Leu Ile Ser Asp Tyr Cys Lys Arg Pro Gly Pro Ala Ser Glu Asp
                165                 170                 175

Phe Ser Gly Tyr Ile Lys Arg Arg Gly Tyr Asp Leu His Tyr Val Glu
            180                 185                 190

Ala Tyr Gly Leu Met Leu Arg Glu Ala Gly Phe Asp Glu Val Ile Ala
        195                 200                 205

Lys Asp Arg Thr Glu Gln Phe Ile Asn Val Leu Arg Lys Glu Leu Tyr
    210                 215                 220

Thr Val Glu Lys Glu Arg Asp Ser Phe Phe Gln Glu Phe Ser Glu Gln
225                 230                 235                 240

Gly Tyr Asn Glu Ile Val Gly Gly Trp Asn Ala Lys Leu Leu Arg Ser
                245                 250                 255

Ser Ser Gly Glu Gln Arg Trp Gly Leu Phe Ile Ala Lys Lys Lys
            260                 265                 270

<210>  16
<211>  730
<212>  PRT
<213>  Capsicum annuum

<400>  16

Met Glu Cys Asn Lys Asp Glu Ala Leu Arg Ala Lys Ile Ile Ala Glu
1               5                   10                  15

Arg Lys Phe Glu Gln Lys Asp Tyr Ala Gly Ala Lys Lys Phe Ala Leu
            20                  25                  30

Lys Ala Gln Ala Leu Tyr Pro Gly Leu Asp Ala Leu Ala Gln Met Leu
        35                  40                  45

Thr Thr Leu Asp Ile Tyr Ile Ala Ala Glu Asn Lys Ile Ser Gly Glu
    50                  55                  60

Val Asp Trp Tyr Gly Val Leu Gly Val Ser Pro Ser Thr Asp Asp Glu
65                  70                  75                  80

Thr Val Arg Lys Gln Tyr Arg Lys Leu Ala Leu Ile Leu His Pro Asp
                85                  90                  95

Lys Asn Lys Ser Val Gly Ala Glu Gly Ala Phe Lys Leu Leu Ser Glu
            100                 105                 110

Ala Trp Ser Leu Leu Ser Asp Lys Ser Lys Arg Leu Ala Tyr Asn Gln
        115                 120                 125

Arg Arg Ser Ser Lys Gly Phe Gln Gln Lys Ala Pro Val His Ser Gly
    130                 135                 140

Gly Pro Ser Ala Pro Pro Arg Asn Gly Phe His Asn Phe Thr Ser Lys
145                 150                 155                 160

Thr Ser Lys Thr Gln Lys Asn Ala Ser Arg Val Pro Ser Ser Ser Val
                165                 170                 175

Asn Ser Ser Ala Asn Gln Arg Ser Asp Thr Phe Trp Thr Ile Cys His
            180                 185                 190

Arg Cys Lys Met His Tyr Glu Tyr Leu Lys Ile Tyr Leu Asn His Thr
        195                 200                 205

Leu Leu Cys Pro Asn Cys His Glu Ala Phe Met Ala Thr Glu Thr Ala
    210                 215                 220

Pro Pro Phe Asn His Ala Lys Ser Ser Asn Ser Thr Arg Gln Trp His
225                 230                 235                 240

Gln Asn Ala Gly Asn His Ala Pro Asn Gly Asn Gln Phe Pro Ile Gly
                245                 250                 255

Arg Asn Ala Ser Ala Gly Lys Thr Ser Gly Ser Ala Pro Ala Ser Ser
            260                 265                 270

Asn Thr Ala Asn Tyr Ser Asn Phe Gln Gln Asp Ser Phe Ser Arg Met
        275                 280                 285

Ala Gly Val Gly Ser Ser Asp Pro Ser Ile Ala Ala Lys Ala Ala Asn
    290                 295                 300

Val Val Gln Gln Ala His Glu Lys Met Lys Arg Glu Arg Asp Asp Ser
305                 310                 315                 320

Gln Thr Arg Gln Pro Pro Leu Lys Gln Arg Lys Leu Asp Asp Asn Gly
                325                 330                 335

Ile Arg Tyr Gly Ser Asn Ile Pro His Tyr Ala Glu Arg Ala Tyr Gly
            340                 345                 350

Phe Ser Ser Ser Lys Phe Asn Ser Thr Arg Glu Leu Thr Pro Leu Glu
        355                 360                 365

Asn Arg Asn Met Leu Met Gly Lys Ala Arg Lys Glu Ile Leu Ser Lys
    370                 375                 380

Leu Asn Glu Trp Arg Ser Gln Pro Gln Asp Ala Gln Lys Asn Lys Val
385                 390                 395                 400

Lys Glu Gly Lys Lys Glu Lys Gln Arg Ala Ala Asn Val Leu Gly Gln
                405                 410                 415

Asn Val Asn Gly Asn Val Glu Ser Ser Ala Met Lys Gly Val Lys Lys
            420                 425                 430

Asp Val Asn Ala Ser Ala Asn Asp Ala Lys Gln Glu Asp Leu Leu Pro
        435                 440                 445

Gln Ser Met Asn Val Pro Asp Pro Asp Phe His Asn Phe Asp Gln Asp
    450                 455                 460

Arg Ser Glu Ser Cys Phe Gly Asp Asn Glu Val Trp Ala Ala Tyr Asp
465                 470                 475                 480

Ala Asp Asp Gly Met Pro Arg Phe Tyr Ala Leu Ile Ser Lys Val Val
                485                 490                 495

Ser Arg Glu Pro Phe Lys Val Arg Leu Ser Trp Leu Asn Ser Lys Thr
            500                 505                 510

Asn Ser Glu Phe Gly Pro Met Glu Trp Val Ala Ser Gly Phe Tyr Lys
        515                 520                 525

Thr Ser Gly Glu Phe Arg Ile Gly Arg Tyr Glu Thr Ser Lys Ser Val
    530                 535                 540

Asn Ser Phe Ser His Lys Val Arg Trp Ser Lys Gly Pro Arg Gly Ser
545                 550                 555                 560

Val Leu Ile Phe Pro Met Lys Gly Glu Val Trp Ala Leu Tyr Arg His
                565                 570                 575

Trp Ser Pro Asp Trp Asn Gln Asn Thr Pro Asp Asp Val Ile His Lys
            580                 585                 590

Tyr Asp Met Val Leu Val Leu Asp Asp Tyr Asn Glu Glu Gln Gly Ile
        595                 600                 605

Ser Val Ala Pro Leu Val Lys Val Ala Gly Phe Lys Thr Val Phe Arg
    610                 615                 620

Pro Asp Leu Asn Ser Glu Lys Val Met Arg Ile Thr Arg Glu Glu Met
625                 630                 635                 640

Phe Arg Phe Ser His Arg Val Pro Ser His Leu Leu Thr Gly Glu Glu
                645                 650                 655

Ala Gln Asn Ala Pro Lys Gly Cys Gln Glu Leu Asp Pro Ala Ala Thr
            660                 665                 670

Pro Leu Glu Leu Leu Gln Thr Leu Pro Glu Thr Asn Gly Val Ser Ala
        675                 680                 685

Met Gln Asn Asp Lys Asp Phe Asn Gly Leu Ser Pro Gln Asn Val Gln
    690                 695                 700

Glu Thr Lys Thr Arg Glu Thr Ala Asp His Thr Leu Asn Ser Ser Glu
705                 710                 715                 720

Asp Gly Met Val Asn Gly Glu Glu Ala Pro
                725                 730

<210>  17
<211>  414
<212>  PRT
<213>  Capsicum annuum

<400>  17

Met Ala Leu Ile Leu His Ser Thr Ser Asn Asn Lys Asn Ala Ala Lys
1               5                   10                  15

Gly Leu Ile Ala Ala Glu Tyr Thr Gly Val Lys Val Glu Leu Ala Lys
            20                  25                  30

Asp Phe Gln Met Gly Val Ser Asn Lys Thr Pro Glu Phe Leu Glu Met
        35                  40                  45

Asn Pro Ile Gly Lys Val Pro Val Leu Gln Thr Pro Asp Gly Pro Val
    50                  55                  60

Phe Glu Ser Asn Ala Ile Ala Arg Tyr Val Thr Lys Thr Lys Pro Asp
65                  70                  75                  80

Asn Leu Leu Phe Gly Ser Ser Leu Ile Glu Tyr Ala Gln Ile Glu Gln
                85                  90                  95

Trp Asn Asp Phe Ser Ala Thr Glu Val Asp Ala Asn Ile Ala Arg Trp
            100                 105                 110

Leu Tyr Pro Arg Leu Gly Tyr Ala Val Tyr Ile Pro Pro Ala Glu Glu
        115                 120                 125

Ala Ala Val Ala Ala Leu Lys Arg Ala Leu Gly Ala Leu Asn Thr His
    130                 135                 140

Leu Ala Ser Asn Thr Tyr Leu Val Gly His Ser Ile Thr Leu Ala Asp
145                 150                 155                 160

Ile Ile Met Val Cys Asn Leu Ser Ile Gly Phe Gly Val Ile Met Thr
                165                 170                 175

Lys Ser Phe Thr Lys Glu Phe Pro His Val Glu Arg Tyr Phe Trp Thr
            180                 185                 190

Val Val Asn Gln Pro Asn Phe Cys Lys Ile Leu Gly Glu Val Lys Gln
        195                 200                 205

Ala Glu Ser Ile Pro Ala Pro Pro Ser Lys Lys Pro Ala Pro Ala Thr
    210                 215                 220

Glu Lys Pro Lys Ala Lys Glu Glu Pro Lys Lys Glu Val Lys Glu Glu
225                 230                 235                 240

Pro Lys Pro Ala Glu Glu Glu Ala Ala Pro Lys Pro Lys Pro Lys Asn
                245                 250                 255

Pro Leu Asp Leu Leu Pro Pro Ser Pro Met Ile Leu Asp Asp Trp Lys
            260                 265                 270

Arg Leu Tyr Ser Asn Thr Lys Thr Asn Phe Arg Glu Val Ala Val Lys
        275                 280                 285

Gly Phe Trp Asp Met Tyr Asp Pro Glu Gly Tyr Ser Leu Trp Phe Cys
    290                 295                 300

Asp Tyr Lys Tyr Asn Asp Glu Asn Thr Val Ser Phe Val Thr Leu Asn
305                 310                 315                 320

Lys Val Gly Gly Phe Leu Gln Arg Met Asp Leu Val Arg Lys Tyr Ala
                325                 330                 335

Phe Gly Lys Met Leu Ile Val Gly Ser Glu Pro Pro Phe Lys Val Gln
            340                 345                 350

Gly Leu Trp Leu Phe Arg Gly Lys Glu Ile Pro Lys Phe Val Met Asp

355                 360                 365

Glu Val Tyr Asp Met Glu Leu Tyr Glu Trp Lys Gln Val Asp Ile Asn
    370                 375                 380

Asp Glu Ala Gln Lys Glu Arg Val Ser Gln Met Ile Glu Asp His Glu
385                 390                 395                 400

Pro Phe Glu Gly Gln Ala Leu Leu Asp Ala Lys Cys Phe Lys
                405                 410

<210>  18
<211>  1907
<212>  PRT
<213>  Capsicum annuum

<400>  18

Met Ala Pro Ser Lys Asn Gly Arg Gly Lys Thr Lys Gly Asp Lys Lys
1               5                   10                  15

Lys Lys Glu Glu Lys Val Leu Pro Val Val Met Asp Ile Thr Ile Asn
            20                  25                  30

Leu Pro Asp Glu Thr Gln Val Ile Leu Lys Gly Ile Ser Thr Asp Arg
        35                  40                  45

Ile Ile Asp Val Arg Arg Leu Leu Ser Val Asn Thr Thr Thr Cys Asn
    50                  55                  60

Ile Thr Asn Phe Ser Leu Ser His Glu Leu Arg Gly Pro Arg Leu Lys
65                  70                  75                  80

Glu Thr Val Asp Val Ser Ala Leu Lys Pro Cys Leu Leu Thr Leu Ile
                85                  90                  95

Glu Glu Asp Tyr Asp Glu Glu Ser Ala Thr Ala His Val Ile Arg Leu
            100                 105                 110

Leu Asp Ile Val Ala Cys Thr Thr Ser Phe Gly Pro Ser Gly Thr Asn
        115                 120                 125

Gly Lys Asp Leu Lys Thr Glu Ser Ser Lys Asn Ala Arg Gly Ala Gln
    130                 135                 140

Asp Ser Lys Gly Ala Lys Lys Ser Ser Lys Val Gln Ala Asn Asp Lys
145                 150                 155                 160

Ser Ser Ser Pro Pro Gln Thr Pro Thr Pro Ala Ala Ala Ser Gln Gln

                165                 170                 175

Ala Gly Lys Asp Ala Ala Ser Val Asp Val Asp Gly Glu Met Ser Asn
            180                 185                 190

Thr Cys Pro Lys Ile Gly Ser Phe Tyr Glu Phe Phe Ser Leu Ser His
        195                 200                 205

Leu Thr Pro Pro Leu Gln Phe Ile Arg Arg Ala Thr Arg Gln Gln Asp
    210                 215                 220

Gly Glu Val Leu Pro Asp Asp His Leu Phe Thr Leu Glu Val Lys Leu
225                 230                 235                 240

Cys Asn Gly Lys Leu Val Ile Val Glu Ala Cys Lys Lys Gly Phe Tyr
                245                 250                 255

Asn Phe Gly Lys Gln Gly Ile Leu Cys His Asn Leu Val Asp Leu Leu
            260                 265                 270

Arg Gln Val Ser Arg Ala Phe Asp Asn Ala Tyr Asp Glu Leu Met Lys
        275                 280                 285

Ala Phe Ser Glu Arg Asn Lys Phe Gly Asn Leu Pro Tyr Gly Phe Arg
    290                 295                 300

Ala Asn Thr Trp Leu Ile Pro Pro Val Ala Ala Gln Leu Pro Ala Ile
305                 310                 315                 320

Phe Pro Pro Leu Pro Val Glu Asp Glu Asn Trp Gly Ala Asn Gly Gly
                325                 330                 335

Gly Leu Gly Arg Asp Gly Lys Phe Asp Leu Leu Pro Tyr Ala Asn Glu
            340                 345                 350

Phe Leu Asn Val Ala Ser Met Pro Cys Lys Thr Thr Glu Glu Arg Gln
        355                 360                 365

Ile Arg Asp Arg Lys Ala Phe Leu Leu His Ser Leu Phe Val Asp Val
    370                 375                 380

Ala Ile Leu Arg Ala Ile Ser Ala Val Lys His Val Met Glu Lys Val
385                 390                 395                 400

Lys Pro Ala His Cys Asn Ser Asn Gly Glu Ile Ile Leu Thr Glu Ser
                405                 410                 415

Val Gly Asp Leu Ser Ile Phe Val Thr Lys Asp Ala Ser Asn Ala Ser
            420                 425                 430

Ser Lys Val Asp Thr Lys Ile Asp Gly Phe Gln Ala Thr Gly Ile Ala
        435                 440                 445

Met Lys Asn Leu Met Glu Arg Asn Leu Leu Lys Gly Ile Thr Ala Asp
    450                 455                 460

Glu Asn Thr Ala Ala His Asp Val Ala Thr Leu Gly Val Leu Asn Val
465                 470                 475                 480

Arg His Cys Gly Tyr Ile Ala Thr Val Lys Val Gln Gly Lys Glu Asn
                485                 490                 495

Asp Lys Val Gly Ser Pro Leu Gln Ser Met Glu Leu Pro Asp Gln Pro
            500                 505                 510

Asp Gly Gly Ala Asn Ala Leu Asn Ile Asn Ser Leu Arg Leu Leu Leu
        515                 520                 525

His Lys Lys Val Val Asn Lys Val Met His Ser Lys Pro Ser Asp Ser
    530                 535                 540

Glu Glu Thr Asn Cys Ser Gln Ala Phe Val Lys Arg Ile Leu Glu Glu
545                 550                 555                 560

Ser Leu Thr Lys Leu Glu Glu Glu Lys Ile Glu Gly Asp Ser Phe Ile
                565                 570                 575

Arg Trp Glu Leu Gly Ala Cys Trp Ile Gln His Leu Gln Asp Gln Lys
            580                 585                 590

Lys Ser Glu Lys Asp Lys Lys Asn Pro Val Glu Lys Thr Lys Asn Glu
        595                 600                 605

Met Lys Val Glu Gly Leu Gly Ile Pro Leu Lys Ser Leu Lys Asn Arg
    610                 615                 620

Lys Lys Ser Ser Asp Gly Ala Asn Thr Glu Ser Gln Ser Asp Thr Phe
625                 630                 635                 640

Lys Ser Ile Ala Asp Gly Val Gln Arg Gly Ser Glu Lys Thr Val Leu
                645                 650                 655

Gln Ser Gly Glu Ser Gln Phe Glu Thr Asp Thr Asp Gln Asn Gln Val
            660                 665                 670

Val Leu Lys Ala Leu Leu Ser Asp Ala Gly Phe Thr Arg Leu Lys Glu
        675                 680                 685

Ser Glu Thr Gly Leu His Leu Lys Ser Leu Glu Glu Leu Ile Val Leu
    690                 695                 700

Ser Gln Lys Tyr Tyr Asn Glu Val Ala Leu Pro Lys Leu Val Ala Asp
705                 710                 715                 720

Phe Gly Ser Leu Glu Leu Ser Pro Val Asp Gly Arg Thr Leu Thr Asp
                725                 730                 735

Phe Met His Thr Arg Gly Leu Arg Met Arg Ser Leu Gly Gln Val Val
            740                 745                 750

Lys Leu Ser Glu Lys Leu Ser His Val Gln Ser Leu Cys Ile His Glu
        755                 760                 765

Met Ile Val Arg Ala Phe Lys His Ile Leu Gln Ala Val Ile Ala Ser
    770                 775                 780

Val Val Asp Ile Glu Asp Met Ala Ala Ile Ile Ala Ala Ala Leu Asn
785                 790                 795                 800

Met Met Leu Gly Val Pro Glu Asn Asp Asp Ala Asn Glu Leu Gly Val
                805                 810                 815

Asp Ser Leu Ile Trp Ile Trp Leu Glu Leu Phe Leu Lys Lys Arg Tyr
            820                 825                 830

Glu Trp Asp Val Gly Ser Leu Asn Tyr Lys Asp Val Arg Lys Phe Ala
        835                 840                 845

Ile Leu Arg Gly Leu Cys His Lys Val Gly Ile Glu Leu Val Pro Arg
    850                 855                 860

Asp Tyr Asp Met Ser Ser Pro Ser Pro Phe Gln Lys Val Asp Ile Val
865                 870                 875                 880

Ser Leu Val Pro Val His Lys Gln Ala Ala Cys Ser Ser Ala Asp Gly
                885                 890                 895

Arg Gln Leu Leu Glu Ser Ser Lys Thr Ala Leu Asp Lys Gly Lys Leu
            900                 905                 910

Glu Asp Ala Val Ser Tyr Gly Thr Lys Ala Leu Ala Lys Leu Val Ala
        915                 920                 925

Val Cys Gly Pro Tyr His Arg Met Thr Ala Gly Ala Tyr Ser Leu Leu
    930                 935                 940

Ala Val Val Leu Tyr His Thr Gly Asp Phe Asn Gln Ala Thr Ile Tyr
945                 950                 955                 960

Gln Gln Lys Ala Leu Asp Ile Asn Glu Arg Glu Leu Gly Leu Asp His
                965                 970                 975

Pro Asp Thr Met Lys Ser Tyr Gly Asp Leu Ala Val Phe Tyr Tyr Arg
            980                 985                 990

Leu Gln His Thr Glu Leu Ala Leu  Lys Tyr Val Lys Arg  Ala Leu Tyr
        995                 1000                 1005

Leu Leu  His Leu Thr Cys Gly  Pro Ser His Pro Asn  Thr Ala Ala
    1010                 1015                 1020

Thr Tyr  Ile Asn Val Ala Met  Met Glu Glu Gly Leu  Gly Asn Val
    1025                 1030                 1035

His Val  Ala Leu Arg Tyr Leu  His Lys Ala Leu Lys  Cys Asn Gln
    1040                 1045                 1050

Lys Leu  Leu Gly Pro Asp His  Ile Gln Thr Ala Ala  Ser Tyr His
    1055                 1060                 1065

Ala Ile  Ala Ile Ala Leu Ser  Leu Met Glu Ala Tyr  Pro Leu Ser
    1070                 1075                 1080

Val Gln  His Glu Gln Thr Thr  Leu Gln Ile Leu Arg  Ala Lys Leu
    1085                 1090                 1095

Gly Pro  Asp Asp Leu Arg Thr  Gln Asp Ala Ala Ala  Trp Leu Glu
    1100                 1105                 1110

Tyr Phe  Glu Ser Lys Ala Phe  Glu Gln Gln Glu Ala  Ala Arg Asn
    1115                 1120                 1125

Gly Thr  Lys Lys Pro Asp Ala  Ser Ile Ala Ser Lys  Gly His Leu
    1130                 1135                 1140

Ser Val  Ser Asp Leu Leu Asp  Tyr Ile Asn Pro Ser  Pro Asp Ala
    1145                 1150                 1155

Lys Gly  Arg Asp Val Gly Ser  Lys Arg Lys Gly Phe  Val Ser Lys

1160                1165                1170

Ala Leu Phe Ser Gln Val Lys Gly Lys Ser Glu Gln Ser Asn Val
    1175                1180                1185

Ser Ile Pro Asn Ser Asp Thr Pro Lys Asp Val Pro Lys Glu Glu
    1190                1195                1200

Thr Asp Glu Lys Lys Gln Leu Ile Glu Asp His Thr Asp Pro Lys
    1205                1210                1215

Met His Met Glu Pro Val Asp Thr Val Ile Glu Ser Asn His Asn
    1220                1225                1230

Gly Asp Gly Arg Ile Ala Glu Asn Lys Pro Ile Gln Ser Gly Pro
    1235                1240                1245

Leu Leu Lys Glu Thr Leu Ile Glu Lys Ser Thr Val His Glu Val
    1250                1255                1260

Leu Ser Glu Pro Ser Ala Glu Ala Glu Asp Gly Trp Gln Pro Val
    1265                1270                1275

Gln Arg Pro Arg Ser Gly Gly Phe Tyr Gly Arg Arg Arg Arg Gln
    1280                1285                1290

Arg Arg Gln Thr Ile Ser Lys Val Ile Gly Tyr Gln Lys Lys Asp
    1295                1300                1305

Pro Val Ser Asp Val Asp His Ala Lys Leu Lys Asn Asn Tyr Gln
    1310                1315                1320

Ala Ser Lys Tyr Tyr Val Leu Lys Lys Arg Thr Ser Pro Gly Ser
    1325                1330                1335

Tyr Ala Asp Tyr Tyr Ile Ala Lys Ser Gln Thr Pro Gly Thr Lys
    1340                1345                1350

Leu Gly Arg Arg Val Ile Lys Ala Val Ala Tyr Arg Val Lys Ser
    1355                1360                1365

Val Ser Pro Ser Val Arg Asp Ala Val Pro Glu Ile Ser Thr Thr
    1370                1375                1380

Gly Gly Asp Leu Thr Thr Ser Ser Glu Gln Val Gln Val Ser Val
    1385                1390                1395

Thr Lys Glu Val Gly Ser Val Ala Lys Arg Ser Ser Ile Val Asn
    1400                1405                1410

Leu Gly Lys Ser Pro Ser Tyr Lys Glu Val Ala Leu Ala Pro Pro
    1415                1420                1425

Gly Thr Ile Ser Met Leu Gln Glu Arg Val Ser Glu Gly Glu Ile
    1430                1435                1440

Pro Asp Asn Gln Asp Ile Thr Lys Leu Glu Glu Glu Ser Asn Gly
    1445                1450                1455

Ala Glu Thr Asn Ser Lys Thr Met Lys Ser Asp Ala Glu Ser Val
    1460                1465                1470

Glu Lys Glu Asn Ile Gln Asp Leu Val Ala Asp Ser Ala Asp His
    1475                1480                1485

Val Lys Ser Glu Thr Val Ala Thr Asp Lys Lys Glu Glu Ile Gln
    1490                1495                1500

Met Ser Asp Leu Asp Gly Gly Glu Ile Ser Asp Val Ile Ser Ala
    1505                1510                1515

Asn Thr Ser Ile Gln Pro Gly His Val Asp Val Ser Pro Val Glu
    1520                1525                1530

Gln Val Ser Val Glu Thr His Asn Val Pro Pro Ser Asp Asn Ser
    1535                1540                1545

Pro Lys Val Asp Pro Cys Glu Lys Asp Ser Ser Ser His Leu Asn
    1550                1555                1560

Pro Thr Cys Ile Ser Asn Met Thr Leu Gln Asp Met Asp His Leu
    1565                1570                1575

Lys Val Lys Ser Ala Ser Ser His Thr Ser Asp Ala Ser Arg Glu
    1580                1585                1590

Leu Ser Arg Lys Leu Ser Ala Ser Ala Thr Pro Phe Ser Pro Ser
    1595                1600                1605

Pro Ala Val Pro Arg Val Ala Pro Leu Pro Met Asn Ile Asn Leu
    1610                1615                1620

Pro Ser Pro Pro Gly Ile Arg Pro Pro Val Gly Pro Trp Pro Val
    1625                1630                1635

Asn Met Ser Leu His Gln Gly Pro Pro Thr Ile Leu Pro Asn Pro
    1640                1645                1650

Met Cys Ser Ser Pro His His Leu Tyr Pro Ser Pro Pro His Thr
    1655                1660                1665

Pro Asn Met Met His Pro Leu Arg Phe Ile Tyr Pro Pro Tyr Ser
    1670                1675                1680

Gln Pro Gln Thr Ile Pro Pro Ser Thr Phe Pro Met Asn Ser Ser
    1685                1690                1695

Thr Phe His Pro Asn His Tyr Ala Trp Gln Cys Asn Ile Ala Pro
    1700                1705                1710

Asn Ala Ser Glu Tyr Val Pro Ala Thr Val Trp Pro Gly Cys His
    1715                1720                1725

Pro Val Glu Phe Ser Ile Ser Pro Pro Val Ile Glu Ser Ile Thr
    1730                1735                1740

Asp Ser Ile Ser Ala Ala Lys Glu Leu Ser Asp Asn Pro Glu Ser
    1745                1750                1755

Ile Ser Leu Thr Thr Ser Leu Pro Val Asp Leu Asn Thr Gly Asp
    1760                1765                1770

Glu Val Lys Glu Asp Glu Asn Leu Pro Val Ser Glu Met Val Glu
    1775                1780                1785

Ser Leu Ala Ala Val Gly Ser Glu Lys Glu Arg Ala Ser Asn Thr
    1790                1795                1800

Ser Asp Ser His Phe Val Thr Phe Ser Ser Asp Gln Ser Lys Glu
    1805                1810                1815

Gly Ser Gly Ser Asn Glu Lys Ala Gly Ser Cys Ser Asp Asn Tyr
    1820                1825                1830

Val Gln Arg Asn Leu Val Glu Thr Asp Asp Glu Lys Thr Phe Asn
    1835                1840                1845

Ile Leu Val Arg Gly Arg Arg Asn Arg Lys Gln Thr Leu Arg Met
    1850                1855                1860

Pro Ile Ser Leu Leu Lys Arg Pro Tyr Ser Ser Gln Pro Phe Lys
    1865                1870                1875

Ala Val Tyr Ser Arg Val Ile Arg Glu Thr Glu Val Pro Arg Ser
    1880                1885                1890

Thr Ser Phe Asp Pro His Glu His Cys Ile Thr Thr Ala Thr
    1895                1900                1905

<210> 19
<211> 263
<212> PRT
<213> Capsicum annuum

<400> 19

Met Gly Asp Leu His Ser Ile Ala Tyr Asn Asn Asn Asn Leu His Ser
1               5                   10                  15

Leu Ser Ser Leu Gly Cys Asp Phe His Gly Leu Glu Val Phe Asn Asn
            20                  25                  30

Asp Ile Ser Pro Phe Met Glu Ser Thr Pro Pro Phe Leu Ser Asn Leu
        35                  40                  45

Asp Cys Asp Leu Ser Ser Gly Tyr Leu Gln Asp Ala Leu Phe Lys Phe
    50                  55                  60

Asn Ser Lys Arg Arg Arg Leu Leu Leu Phe Asp Asp Asp Asp Glu Glu
65                  70                  75                  80

Lys Glu Asn Tyr Gln Ile Asn Asp Ser Asn Asn Ser Ile Lys Asn Leu
                85                  90                  95

Trp Ser Ser Thr Met Asp Gln Gln Phe Ser Glu Asp Tyr Asp Cys Phe
            100                 105                 110

Ser Gln Ile Thr Lys Cys Asp Ser Phe Ser Gly Asp Pro Met Ser Lys
        115                 120                 125

Met Ser Glu Glu Tyr Ser Lys Glu Glu Ala Ile Ser Glu Asn Tyr Tyr
    130                 135                 140

Tyr Tyr Ser Ser Ser Pro Thr Ser Ser Ser His His Asn Asn Ile Asn
145                 150                 155                 160

Gln Lys Gln Leu Pro Gln Gln Tyr Gly Gly Val Asp Gln Lys Arg Ser
                165                 170                 175

Lys Lys Arg Met Val Gly Lys Ile Val Cys Pro Phe Gly Leu Val Lys
            180                 185                 190

Pro Gly Gly Gln Glu Gly Asp Val Thr Leu Asn Asp Ile Asn Glu Arg
        195                 200                 205

Ile Leu Met Arg Pro Thr Arg Pro Val Arg His Pro Val Gly Asp Phe
    210                 215                 220

Ala Cys Arg Pro Thr Val Ser Pro Ala Gly Pro Gly Leu Ser Gly Lys
225                 230                 235                 240

Lys Val Val Ala Leu Thr Lys Ile His Thr Gln Gly Arg Gly Thr Ile
                245                 250                 255

Thr Ile Ile Arg Thr Arg Gly
            260

<210>  20
<211>  13452
<212>  DNA
<213>  Artificial Sequence

<220>
<223>  pJL462 plasmid

<400>  20
gagatggtca cagcttgtct gtaagcggat gccgggagca gacaagcccg tcagggcgcg      60

tcagcgggtg ttggcgggtg tcggggcgca gccatgaccc agtcacgtag cgatagcgga     120

gtgtatactg gcttaactat gcggcatcag agcagattgt actgagagtg caccatatgc     180

ggtgtgaaat accgcacaga tgcgtaagga gaaaataccg catcaggcgc tcttccgctt     240

cctcgctcac tgactcgctg cgctcggtcg ttcggctgcg gcgagcggta tcagctcact     300

caaaggcggt aatacggtta tccacagaat caggggataa cgcaggaaag aacatgtgag     360

caaaaggcca gcaaaaggcc aggaaccgta aaaaggccgc gttgctggcg tttttccata     420

ggctccgccc ccctgacgag catcacaaaa atcgacgctc aagtcagagg tggcgaaacc     480

cgacaggact ataaagatac caggcgtttc cccctggaag ctccctcgtg cgctctcctg     540

ttccgaccct gccgcttacc ggatacctgt ccgcctttct cccttcggga agcgtggcgc     600

tttctcatag ctcacgctgt aggtatctca gttcggtgta ggtcgttcgc tccaagctgg     660

gctgtgtgca cgaacccccc gttcagcccg accgctgcgc cttatccggt aactatcgtc     720

ttgagtccaa cccggtaaga cacgacttat cgccactggc agcagccact ggtaacagga     780

ttagcagagc gaggtatgta ggcggtgcta cagagttctt gaagtggtgg cctaactacg     840

gctacactag aaggacagta tttggtatct gcgctctgct gaagccagtt accttcggaa     900

aaagagttgg tagctcttga tccggcaaac aaaccaccgc tggtagcggt ggtttttttg     960

tttgcaagca gcagattacg cgcagaaaaa aaggatctca agaagatcct ttgatctttt 1020

ctacggggtc tgacgctcag tggaacgaaa actcacgtta agggattttg gtcatgcatg 1080

atatatctcc caatttgtgt agggcttatt atgcacgctt aaaaataata aaagcagact 1140

tgacctgata gtttggctgt gagcaattat gtgcttagtg catctaatcg cttgagttaa 1200

cgccggcgaa gcggcgtcgg cttgaacgaa tttctagcta gacaaggtac taaaacaatt 1260

catccagtaa aatataatat tttattttct cccaatcagg cttgatcccc agtaagtcaa 1320

aaaatagctc gacatactgt tcttccccga tatcctccct gatcgaccgg acgcagaagg 1380

caatgtcata ccacttgtcc gccctgccgc ttctcccaag atcaataaag ccacttactt 1440

tgccatcttt cacaaagatg ttgctgtctc ccaggtcgcc gtgggaaaag acaagttcct 1500

cttcgggctt ttccgtcttt aaaaaatcat acagctcgcg cggatcttta aatggagtgt 1560

cttcttccca gttttcgcaa tccacatcgg ccagatcgtt attcagtaag taatccaatt 1620

cggctaagcg gctgtctaag ctattcgtat agggacaatc cgatatgtcg atggagtgaa 1680

agagcctgat gcactccgca tacagctcga taatcttttc agggctttgt tcatcttcat 1740

actcttccga gcaaaggacg ccatcggcct cactcatgag cagattgctc cagccatcat 1800

gccgttcaaa gtgcaggacc tttggaacag gcagctttcc ttccagccat agcatcatgt 1860

ccttttcccg ttccacatca taggtggtcc ctttataccg gctgtccgtc atttttaaat 1920

ataggttttc attttctccc accagcttat ataccttagc aggagacatt ccttccgtat 1980

cttttacgca gcggtatttt tcgatcagtt ttttcaattc cggtgatatt ctcattttag 2040

ccataacaag aagccatgaa aaccgccact gcgccgttac caccgctgcg ttcggtcaag 2100

gttctggacc agttgcgtga cggcagttac gctacttgca ttacagctta cgaaccgaac 2160

gaggcttatg tccactgggt tcgtgcccga attgatcaca ggcagcaacg ctctgtcatc 2220

gttacaatca acatgctacc ctccgcgaga tcatccgtgt ttcaaacccg gcagcttagt 2280

tgccgttctt ccgaatagca tcggtaacat gagcaaagtc tgccgcctta caacggctct 2340

cccgctgacg ccgtcccgga ctgatgggct gcctgtatcg agtggtgatt ttgtgccgag 2400

ctgccggtcg gggagctgtt ggctggctgg tggcaggata tattgtggtg taaacaaatt 2460

gacgcttaga caacttaata acacattgcg gacgttttta atgtactgaa ttaacgccga 2520

attgaattat cagcttgcat gcagctctcc catatggtcg actagaggcc tctagggccg 2580

ttttacgttt ggaactgaca gaaccgcaac gttgaaggag ccactcagcc gcgggtttct 2640

ggagtttaat gagctaagca catacgtcag aaaccattat tgcgcgttca aaagtcgcct 2700

aaggtcacta tcagctagca aatatttctt gtcaaaaatg ctccactgac gttccataaa 2760

ttcccctcgg tatccaatta gagtctcata ttcactctca actcgatcga ggcatgattg 2820

aacaagatgg attgcacgca ggttctccgg ccgcttgggt ggagaggcta ttcggctatg 2880

actgggcaca acagacaatc ggctgctctg atgccgccgt gttccggctg tcagcgcagg 2940

ggcgcccggt tctttttgtc aagaccgacc tgtccggtgc cctgaatgaa ctccaagacg 3000

aggcagcgcg gctatcgtgg ctggccacga cgggcgttcc ttgcgcagct gtgctcgacg 3060

ttgtcactga agcgggaagg gactggctgc tattgggcga agtgccgggg caggatctcc 3120

tgtcatctca ccttgctcct gccgagaaag tatccatcat ggctgatgca atgcggcggc 3180

tgcatacgct tgatccggct acctgcccat tcgaccacca agcgaaacat cgcatcgagc 3240

gagcacgtac tcggatggaa gccggtcttg tcgatcagga tgatctggac gaagagcatc 3300

aggggctcgc gccagccgaa ctgttcgcca ggctcaaggc gcggatgccc gacggcgagg 3360

atctcgtcgt gacccacggc gatgcctgct tgccgaatat catggtggaa aatggccgct 3420

tttctggatt catcgactgt ggccggctgg gtgtggcgga ccgctatcag gacatagcgt 3480

tggctacccg tgatattgct gaagagcttg gcggcgaatg ggctgaccgc ttcctcgtgc 3540

tttacggtat cgccgctccc gattcgcagc gcatcgcctt ctatcgcctt cttgacgagt 3600

tcttctgagc gggactctgg ggttcggact ctagctagag tcaagcagat cgttcaaaca 3660

tttggcaata aagtttctta agattgaatc ctgttgccgg tcttgcgatg attatcatat 3720

aatttctgtt gaattacgtt aagcatgtaa taattaacat gtaatgcatg acgttattta 3780

tgagatgggt ttttatgatt agagtcccgc aattatacat ttaatacgcg atagaaaaca 3840

aaatatagcg cgcaaactag gataaattat cgcgcgcggt gtcatctatg ttactagatc 3900

gaccggttgg tacgaattcc tgcaggtcaa catggtggag cacgacacac ttgtctactc 3960

caaaaatatc aaagatacag tctcagaaga ccaaagggca attgagactt ttcaacaaag 4020

ggaaatatcg ggaaacctcc tcggattcca ttgcccagct atctgtcact tcatcaaaag 4080

gacagtagaa aaggaaggtg gcacctacaa atgccatcat tgcgataaag gaaaggctat 4140

cgttcaagat gcctctgccg acagtggtcc caaagatgga cccccaccca cgaggagcat 4200

cgtggaaaaa gaagacgttc caaccacgtc ttcaaagcaa gtggattgat gtgataacat 4260

ggtggagcac gacactctcg tctactccaa gaatatcaaa gatacagtct cagaagacca 4320

aagggctatt gagacttttc aacaaagggt aatatcggga aacctcctcg gattccattg 4380

cccagctatc tgtcacttca tcaaaaggac agtagaaaag gaaggtggca cctacaaatg 4440

ccatcattgc gataaaggaa aggctatcgt tcaagatgcc tctgccgaca gtggtcccaa 4500

agatggaccc ccacccacga ggagcatcgt ggaaaaagaa gacgttccaa ccacgtcttc 4560

aaagcaagtg gattgatgtg atatctccac tgacgtaagg gatgacgcac aatcccacta 4620

tccttcgcaa gaccttcctc tatataagga agttcatttc atttggagag gacctcgaga 4680

attctcaaca caacatatac aaaacaaacg aatctcaagc aatcaagcat tctacttcta 4740

ttgcagcaat ttaaatcatt tcttttaaag caaaagcaat tttctgaaaa ttttcaccat 4800

ttacgaacga tagttaatta accatgagca agctcgagaa gtttaccaac tgctacagcc 4860

tctctaagac cctcaggttc aaggctatcc ctgtgggaaa gacccaagag aatatcgaca 4920

acaagaggct cctcgtcgag gatgagaaga gagctgaaga ttacaagggc gtgaagaagc 4980

tcctcgacag gtactacctc agcttcatca acgatgtgct ccacagcatc aagctcaaga 5040

acctcaacaa ctacatcagc ctcttccgta agaaaaccag gaccgagaaa gagaacaaag 5100

agcttgagaa cctcgagatc aacctccgta aagagatcgc caaggctttc aagggaaacg 5160

agggatacaa gagcctcttc aagaaggata ttatcgagac aatcctgcct gagttcctgg 5220

acgataagga tgagatcgct ctcgtgaaca gcttcaacgg attcactact gccttcaccg 5280

gattcttcag aaacagggaa aacatgttca gcgaagaggc caagagcacc tctatcgctt 5340

tcagatgcat caacgagaac ctcacgcgtt acatcagcaa catggacatc ttcgagaagg 5400

tggacgccat cttcgataag cacgaggtgc aagaaatcaa agagaagatc ctcaacagcg 5460

actacgacgt cgaggacttt tttgaagggg agttcttcaa cttcgttctc acccaagagg 5520

gcatcgacgt gtacaacgct attatcggag gattcgtgac cgagtctggg gagaagatta 5580

agggactcaa cgagtacatc aacctgtaca accagaaaac gaagcagaag ctcccgaagt 5640

tcaagccgct ctacaagcag gttctctctg atcgtgagag cctctcattt tacggtgagg 5700

gttacacctc tgacgaggaa gtgcttgagg ttttccgtaa caccctcaac aagaacagcg 5760

agatcttctc gtccatcaag aagttggaga agcttttcaa gaacttcgac gagtacagca 5820

gcgctgggat cttcgttaag aacggacctg ctatcagcac catcagcaag gatattttcg 5880

gcgagtggaa cgtgatcagg gacaagtgga atgctgagta cgatgacatc cacctcaaga 5940

agaaggctgt cgtcactgag aagtacgagg atgacaggcg taagtcgttc aagaagatcg 6000

gctctttcag cctcgagcag cttcaagaat acgctgatgc tgatctcagc gtggtcgaga 6060

agctcaaaga gatcatcatc cagaaggtcg acgagatcta caaggtgtac gggtcctctg 6120

agaagttgtt cgatgctgat ttcgtcctcg agaagagtct gaagaagaac gacgctgtcg 6180

tcgcgatcat gaaggatttg ctcgacagcg tgaagtcctt cgagaactat atcaaggcct 6240

tcttcggaga gggcaaagag actaataggg acgagtcttt ctacggggat ttcgtgctcg 6300

cttacgatat cctcctcaag gtggaccata tctacgacgc catcagaaac tacgtgaccc 6360

agaagcctta cagcaaggac aagttcaagt tgtactttca gaacccgcag ttcatgggcg 6420

gatgggacaa agacaaagag acagattaca gggccaccat cctcaggtac gggtctaagt 6480

actacctggc catcatggac aagaaatacg ccaagtgcct ccaaaagatc gacaaggatg 6540

acgtgaacgg gaactatgag aagatcaact acaagctcct tccgggaccg aacaagatgc 6600

ttcctaaggt gttcttcagc aagaaatgga tggcctacta caacccgtct gaggacatcc 6660

agaaaatcta caagaacggg accttcaaga aaggcgacat gttcaacctc aacgactgcc 6720

acaagctcat cgatttcttc aaggacagca tctcgcgtta cccgaagtgg tctaacgctt 6780

acgactttaa cttcagcgag acagaaaagt acaaggatat cgccgggttc taccgtgagg 6840

ttgaggaaca gggttacaag gttagcttcg agagcgcctc caagaaagag gttgacaagt 6900

tggtcgaaga gggcaagctc tacatgttcc agatctataa caaggacttc tccgacaaga 6960

gccacggaac tcctaacctc catacgatgt acttcaagct gcttttcgac gagaacaacc 7020

acgggcagat cagactttct ggtggtgctg aactcttcat gcgtagggcc tcactcaaga 7080

aagaagagtt ggttgttcac ccggccaact ctccaatcgc taacaagaat cctgacaacc 7140

cgaaaaagac caccacgctg tcttacgacg tctacaagga caaaaggttc agcgaggacc 7200

agtacgagct tcatatcccg atcgctatca acaagtgccc gaagaacatc ttcaagatca 7260

ataccgaggt gagggtgctg ctcaagcacg atgataaccc ttacgtgatc ggaatcgatc 7320

gtggtgagag aaacctcctc tacatcgttg tggtggacgg aaagggaaac atcgtcgagc 7380

agtacagcct gaacgagatt atcaacaatt tcaacggcat caggatcaag accgactacc 7440

actcactcct cgataagaaa gaaaaagagc gtttcgaggc caggcagaac tggacttcta 7500

tcgaaaacat caaagagttg aaggccggct acatctctca ggtggtgcat aagatctgcg 7560

agctggtgga aaagtacgat gctgtgatcg ctcttgagga cctcaactct gggttcaaga 7620

acagtagagt gaaggttgag aagcaggtct accaaaagtt cgagaagatg ctcatcgaca 7680

agctcaacta catggtggac aaaaagagca acccttgcgc taccggtggt gctcttaagg 7740

gataccagat cacgaacaag ttcgagtcct tcaagagcat gagcacccag aacggcttca 7800

tcttctatat ccctgcttgg ctcaccagca agatcgatcc ttctactggt ttcgtgaacc 7860

tgctcaagac caagtacacc tcgatcgccg acagcaagaa gttcatctcg tctttcgaca 7920

ggatcatgta cgtgccggaa gaggatcttt tcgagttcgc tctcgactat aagaacttca 7980

gcaggaccga cgccgactac attaagaagt ggaagctcta ctcctacggg aaccgtatca 8040

ggatcttccg aaatccgaag aaaaacaacg tgttcgactg ggaagaagtg tgcctcacct 8100

ctgcctacaa agaactgttc aacaagtacg gcatcaacta ccagcagggt gatatcaggg 8160

ctcttttgtg cgagcagagc gacaaggcat tctacagctc attcatggcc ctcatgtctc 8220

tcatgctcca gatgaggaac tctatcaccg gaaggaccga tgtggacttc cttatctctc 8280

cggtcaagaa ctctgacggg atcttctacg acagccgtaa ctatgaggct caagagaacg 8340

ctatcctgcc gaagaatgct gatgcaaacg gggcttacaa cattgcgaga aaggttctct 8400

gggctatcgg gcagtttaag aaagcggaag atgagaagct ggacaaggtg aagatcgcca 8460

tctccaacaa agagtggctt gagtacgctc agacctccgt taagcacaag aggcctgctg 8520

ctactaagaa agctggtcag gctaagaaga agaaatgagg atcccgggcc cgtcgactgc 8580

agaggcctgc atgcatcgaa tttccccgat cgttcaaaca tttggcaata aagtttctta 8640

agattgaatc ctgttgccgg tcttgcgatg attatcatat aatttctgtt gaattacgtt 8700

aagcatgtaa taattaacat gtaatgcatg acgttattta tgagatgggt ttttatgatt 8760

agagtcccgc aattatacat ttaatacgcg atagaaaaca aaatatagcg cgcaaactag 8820

gataaattat cgcgcgcggt gtcatctatg ttactagatc gggaagcttc ggaagacatc 8880

ttgtgagacc gaggtctcag gtagtctaga gtcgacggta tcgacttttt ttttgtttat 8940

cagcttacat tttcttgaac cgtagctttc gtttcttctt ttttaacttt ccattcggag 9000

tttttgtatc ttgtttcata gtttgtccca ggattagaat gattaggcat cgaaccttca 9060

agaatttgat tgaataaaac atcttcattc ttaagatatg aagataatct tcaaaaggcc 9120

cctgggaatc tgaaagaaga gaagcaggcc catttatatg ggaaagaaca atagtatttc 9180

ttatataggc ccatttaagt tgaaaacaat cttcaaaagt cccacatcgc ttagataaga 9240

aaacgaagct gagtttatat acagctagag tcgaagtagt gattgaaatt actgatgagt 9300

ccgtgaggac gaaacgagta agctcgtcta atttctacta agtgtagatg gagacgttaa 9360

tggatacgtc tcggccggca tggtcccagc ctcctcgctg gcgccggctg ggcaacatgc 9420

ttcggcatgg cgaatgggac ttttttttct agaggtaata tggtttaaac tatcagtgtt 9480

tgacaggata tattggcggg taaacctaag agaaaagagc gtttattaga ataacggata 9540

tttaaaaggg cgtgaaaagg tttatccgtt cgtccatttg tatgtgcatg ccaaccacag 9600

ggttcccctc gggatcaaag tactttgatc caacccctcc gctgctatag tgcagtcggc 9660

ttctgacgtt cagtgcagcc gtcttctgaa aacgacatgt cgcacaagtc ctaagttacg 9720

cgacaggctg ccgccctgcc cttttcctgg cgttttcttg tcgcgtgttt tagtcgcata 9780

aagtagaata cttgcgacta gaaccggaga cattacgcca tgaacaagag cgccgccgct 9840

ggcctgctgg gctatgcccg cgtcagcacc gacgaccagg acttgaccaa ccaacgggcc 9900

gaactgcacg cggccggctg caccaagctg ttttccgaga agatcaccgg caccaggcgc 9960

gaccgcccgg agctggccag gatgcttgac cacctacgcc ctggcgacgt tgtgacagtg 10020

accaggctag accgcctggc ccgcagcacc cgcgacctac tggacattgc cgagcgcatc 10080

caggaggccg gcgcgggcct gcgtagcctg gcagagccgt gggccgacac caccacgccg 10140

gccggccgca tggtgttgac cgtgttcgcc ggcattgccg agttcgagcg ttccctaatc 10200

atcgaccgca cccggagcgg gcgcgaggcc gccaaggccc gaggcgtgaa gtttggcccc 10260

cgccctaccc tcaccccggc acagatcgcg cacgcccgcg agctgatcga ccaggaaggc 10320

cgcaccgtga aagaggcggc tgcactgctt ggcgtgcatc gctcgaccct gtaccgcgca 10380

cttgagcgca gcgaggaagt gacgcccacc gaggccaggc ggcgcggtgc cttccgtgag 10440

gacgcattga ccgaggccga cgccctggcg gccgccgaga atgaacgcca agaggaacaa 10500

gcatgaaacc gcaccaggac ggccaggacg aaccgttttt cattaccgaa gagatcgagg 10560

cggagatgat cgcggccggg tacgtgttcg agccgcccgc gcaagtctca accgtgcggc 10620

tgcatgaaat cctggccggt ttgtctgatg ccaagctggc ggcctggccg gccagcttgg 10680

ccgctgaaga aaccgagcgc cgccgtctaa aaaggtgatg tgtatttgag taaaacagct 10740

tgcgtcatgc ggtcgctgcg tatatgatgc gatgagtaaa taaacaaata cgcaagggga 10800

acgcatgaag gttatcgctg tacttaacca gaaaggcggg tcaggcaaga cgaccatcgc 10860

aacccatcta gcccgcgccc tgcaactcgc cggggccgat gttctgttag tcgattccga 10920

tccccagggc agtgcccgcg attgggcggc cgtgcgggaa gatcaaccgc taaccgttgt 10980

cggcatcgac cgcccgacga ttgaccgcga cgtgaaggcc atcggccggc gcgacttcgt 11040

agtgatcgac ggagcgcccc aggcggcgga cttggctgtg tccgcgatca aggcagccga 11100

cttcgtgctg attccggtgc agccaagccc ttacgacata tgggccaccg ccgacctggt 11160

ggagctggtt aagcagcgca ttgaggtcac ggatggaagg ctacaagcgg cctttgtcgt 11220

gtcgcgggcg atcaaaggca cgcgcatcgg cggtgaggtt gccgaggcgc tggccgggta 11280

cgagctgccc attcttgagt cccgtatcac gcagcgcgtg agctacccag gcactgccgc 11340

cgccggcaca accgttcttg aatcagaacc cgagggcgac gctgcccgcg aggtccaggc 11400

gctggccgct gaaattaaat caaaactcat ttgagttaat gaggtaaaga gaaaatgagc 11460

aaaagcacaa acacgctaag tgccggccgt ccgagcgcac gcagcagcaa ggctgcaacg 11520

ttggccagcc tggcagacac gccagccatg aagcgggtca actttcagtt gccggcggag 11580

gatcacacca agctgaagat gtacgcggta cgccaaggca agaccattac cgagctgcta 11640

tctgaataca tcgcgcagct accagagtaa atgagcaaat gaataaatga gtagatgaat 11700

tttagcggct aaaggaggcg gcatggaaaa tcaagaacaa ccaggcaccg acgccgtgga 11760

atgccccatg tgtggaggaa cgggcggttg gccaggcgta agcggctggg ttgtctgccg 11820

gccctgcaat ggcactggaa cccccaagcc cgaggaatcg gcgtgacggt cgcaaaccat 11880

ccggcccggt acaaatcggc gcggcgctgg gtgatgacct ggtggagaag ttgaaggccg 11940

cgcaggccgc ccagcggcaa cgcatcgagg cagaagcacg ccccggtgaa tcgtggcaag 12000

cggccgctga tcgaatccgc aaagaatccc ggcaaccgcc ggcagccggt gcgccgtcga 12060

ttaggaagcc gcccaagggc gacgagcaac cagatttttt cgttccgatg ctctatgacg 12120

tgggcacccg cgatagtcgc agcatcatgg acgtggccgt tttccgtctg tcgaagcgtg 12180

accgacgagc tggcgaggtg atccgctacg agcttccaga cgggcacgta gaggtttccg 12240

cagggccggc cggcatggcc agtgtgtggg attacgacct ggtactgatg gcggtttccc 12300

atctaaccga atccatgaac cgataccggg aagggaaggg agacaagccc ggccgcgtgt 12360

tccgtccaca cgttgcggac gtactcaagt tctgccggcg agccgatggc ggaaagcaga 12420

aagacgacct ggtagaaacc tgcattcggt taaacaccac gcacgttgcc atgcagcgta 12480

cgaagaaggc caagaacggc cgcctggtga cggtatccga gggtgaagcc ttgattagcc 12540

gctacaagat cgtaaagagc gaaaccgggc ggccggagta catcgagatc gagctagctg 12600

attggatgta ccgcgagatc acagaaggca agaacccgga cgtgctgacg gttcaccccg 12660

attacttttt gatcgatccc ggcatcggcc gttttctcta ccgcctggca cgccgcgccg 12720

caggcaaggc agaagccaga tggttgttca agacgatcta cgaacgcagt ggcagcgccg 12780

gagagttcaa gaagttctgt ttcaccgtgc gcaagctgat cgggtcaaat gacctgccgg 12840

agtacgattt gaaggaggag gcggggcagg ctggcccgat cctagtcatg cgctaccgca 12900

acctgatcga gggcgaagca tccgccggtt cctaatgtac ggagcagatg ctagggcaaa 12960

ttgccctagc aggggaaaaa ggtcgaaaag gtcactttcc tgtggatagc acgtacattg 13020

ggaacccaaa gccgtacatt gggaaccgga acccgtacat tgggaaccca aagccgtaca 13080

ttgggaaccg gtcacacatg taagtgactg atataaaaga gaaaaaaggc gatttttccg 13140

cctaaaactc tttaaaactt attaaaactc ttaaaacccg cctggcctgt gcataactgt 13200

ctggccagcg cacagccgaa gagctgcaaa aagcgcctac ccttcggtcg ctgcgctccc 13260

tacgccccgc cgcttcgcgt cggcctatcg cggccgctgg ccgctcaaaa atggctggcc 13320

tacggccagg caatctacca gggcgcggac aagccgcgcc gtcgccactc gaccgccggc 13380

gcccacatca aggcaccctg cctcgcgcgt ttcggtgatg acggtgaaaa cctctgacac 13440

atgcagctcc cg 13452

<210> 21
<211> 28
<212> DNA
<213> Artificial Sequence

<220>
<223> DnaJ domain-containing protein Oligo for guide Forward

<400> 21
agatggcaga gatgtaaaca tcaaatgg 28

<210> 22
<211> 28
<212> DNA
<213> Artificial Sequence

<220>
<223> DnaJ domain-containing protein Oligo for guide Reversed

<400> 22
cggcccattt gatgtttaca tctctgcc 28

<210>  23
<211>  28
<212>  DNA
<213>  Artificial Sequence

<220>
<223>  XRI1-like protein Oligo for guide Forward

<400>  23
agattagtta tggagagcac tccaccag                                          28


<210>  24
<211>  28
<212>  DNA
<213>  Artificial Sequence

<220>
<223>  XRI1-like protein Oligo for guide Reversed

<400>  24
cggcctggtg gagtgctctc cataacta                                          28


<210>  25
<211>  28
<212>  DNA
<213>  Artificial Sequence

<220>
<223>  XRI1-like protein Oligo for guide Forward

<400>  25
agatgccaga taaccaagtg tgatagcg                                          28


<210>  26
<211>  28
<212>  DNA
<213>  Artificial Sequence

<220>
<223>  XRI1-like protein Oligo for guide Reversed

<400>  26
cggccgctat cacacttggt tatctggc                                          28


<210>  27
<211>  28
<212>  DNA
<213>  Artificial Sequence

<220>
<223>  DnaA initiator-associating diaA protein Oligo for guide Forward

<400>  27
agatgcctta ctacctggtc ctgctggg                                          28


<210>  28

<211>  28
<212>  DNA
<213>  Artificial Sequence

<220>
<223>  DnaA initiator-associating diaA protein Oligo for guide Reversed

<400>  28
cggccccagc aggaccaggt agtaaggc                                          28


<210>  29
<211>  28
<212>  DNA
<213>  Artificial Sequence

<220>
<223>  DnaA initiator-associating diaA protein Oligo for guide Forward

<400>  29
agatgttgct agactcatgg gtttggag                                          28


<210>  30
<211>  28
<212>  DNA
<213>  Artificial Sequence

<220>
<223>  DnaA initiator-associating diaA protein Oligo for guide Reversed

<400>  30
cggcctccaa acccatgagt ctagcaac                                          28


<210>  31
<211>  28
<212>  DNA
<213>  Artificial Sequence

<220>
<223>  TAF-2 protein Oligo for guide Forward

<400>  31
agatacatgg cagtagctgg tcgactcg                                          28


<210>  32
<211>  28
<212>  DNA
<213>  Artificial Sequence

<220>
<223>  TAF-2 protein Oligo for guide Reversed

<400>  32
cggccgagtc gaccagctac tgccatgt                                          28


<210>  33
<211>  28
<212>  DNA
<213>  Artificial Sequence

<220>
<223>  TAF-2 protein Oligo for guide Forward

<400>  33
agatacccaa agagtcgacc agctactg                                          28


<210>  34
<211>  28
<212>  DNA
<213>  Artificial Sequence

<220>
<223>  TAF-2 protein Oligo for guide Reversed

<400>  34
cggccagtag ctggtcgact ctttgggt                                          28


<210>  35
<211>  28
<212>  DNA
<213>  Artificial Sequence

<220>
<223>  Phosphoethanolamine N-methyltransferase protein Oligo for guide
       Forward

<400>  35
agatcctgaa tcagtggact tgatattg                                          28


<210>  36
<211>  28
<212>  DNA
<213>  Artificial Sequence

<220>
<223>  Phosphoethanolamine N-methyltransferase protein Oligo for guide
       Reversed

<400>  36
cggccaatat caagtccact gattcagg                                          28


<210>  37
<211>  28
<212>  DNA
<213>  Artificial Sequence

<220>
<223>  Phosphoethanolamine N-methyltransferase protein Oligo for guide
       Forward

<400>  37
agatactggg gacttagccg agaaggcg                                          28


<210>  38
<211>  28
<212>  DNA
<213>  Artificial Sequence

<220>
<223>  Phosphoethanolamine N-methyltransferase protein Oligo for guide
      Reversed

<400>  38
cggccgcctt ctcggctaag tccccagt                                          28


<210>  39
<211>  28
<212>  DNA
<213>  Artificial Sequence

<220>
<223>  eEF1B protein Oligo for guide Forward

<400>  39
agatacctca cccaatatct tgacgaag                                          28


<210>  40
<211>  28
<212>  DNA
<213>  Artificial Sequence

<220>
<223>  eEF1B protein Oligo for guide Reversed

<400>  40
cggccttcgt caagatattg ggtgaggt                                          28


<210>  41
<211>  28
<212>  DNA
<213>  Artificial Sequence

<220>
<223>  eEF1B protein Oligo for guide Forward

<400>  41
agatatgcag ctactgcacc ttcctcag                                          28


<210>  42
<211>  28
<212>  DNA
<213>  Artificial Sequence

<220>
<223>  eEF1B protein Oligo for guide Reversed

<400>  42
cggcctgagg aaggtgcagt agctgcat                                          28


<210>  43
<211>  28
<212>  DNA
<213>  Artificial Sequence

<220>

<223>  Tetratricopeptide repeat (TPR)-like superfamily protein Oligo for
       guide Forward

<400>  43
agatccacta gtccccgacg gcccaaag                                          28


<210>  44
<211>  28
<212>  DNA
<213>  Artificial Sequence

<220>
<223>  Tetratricopeptide repeat (TPR)-like superfamily protein Oligo for
       guide Reversed

<400>  44
cggcctttgg gccgtcgggg actagtgg                                          28


<210>  45
<211>  28
<212>  DNA
<213>  Artificial Sequence

<220>
<223>  Adenylyl cyclase-associated protein Oligo for guide Forward

<400>  45
agatggtcca ggattgagaa aggtgacg                                          28


<210>  46
<211>  28
<212>  DNA
<213>  Artificial Sequence

<220>
<223>  Adenylyl cyclase-associated protein Oligo for guide Reversed

<400>  46
cggccgtcac ctttctcaat cctggacc                                          28


<210>  47
<211>  28
<212>  DNA
<213>  Artificial Sequence

<220>
<223>  Adenylyl cyclase-associated protein Oligo for guide Forward

<400>  47
agatgtggtg cagatacagc tactttcg                                          28


<210>  48
<211>  28
<212>  DNA
<213>  Artificial Sequence

<220>
<223>  Adenylyl cyclase-associated protein Oligo for guide Reversed

<400>  48
cggccgaaag tagctgtatc tgcaccac                                          28


<210>  49
<211>  21
<212>  DNA
<213>  Artificial Sequence

<220>
<223>  U6pro-F Oligo Forward

<400>  49
aggcatcgaa ccttcaagaa t                                                 21


<210>  50
<211>  2889
<212>  DNA
<213>  Artificial Sequence

<220>
<223>  DnaA initiator-associating diaA CDS

<400>  50
atgaatgata gtttagccat aacagcatca tcattggcca ttactgagaa aaagcctcaa      60

aggcctgggg gttgtgtggg tattttcttt caactctttg attggaaccg tagatttgcc     120

aagaagaagt tgtttccaaa gaaactgctt tcaccagctc gcttgaaaca agcttcaaag     180

aagtttggag gggatgaaaa gcagccaaag catcgtttga ttgctaatga gaatagtggg     240

ggttttccaa ttgcaaagag caacggaatg agcaatactc gttgtgaaag caagcgcgaa     300

atgaaagcac caagtttggt tgctagactc atgggtttgg aatcaatgcc agcaggacca     360

ggtagtaagg ccaaaaaggc ttcagcttct gaaactggga gctatgtggc ggagaaactt     420

gatgctcggc ctggcggatc tgataaagaa gatatggatt gtgagaaggc tgaaataaag     480

cgtgaattga ggccccaaaa gcttcagaag ataggggtga gtgagagacg tccagtcagc      540

agatttagtg ctgaagcatt gcagttgagg actgtattgt caaggccaag gaaacatcag     600

cctaaattga cttctccagt aaaaagtcct agaaacgtct ctggtagaaa tgcatctagg     660

ttgattgggg ctgcaactag gattttggaa ccgggtttgc aaaagagtag ggcgaaatgt     720

gctcttactt atcctaaata tttttctcct ttggaagata aagcagattt agcactgcat     780

catttggaag tccctaatcc ttgtgtggat tcaaagactt cggaagtgag agcatctgtg     840

ccttcatgta aaaattgtgg ttatatgctt catagtaaga atggtacacc aaatggggaa     900

gaacacccat cgtctgtctc gtcacctgtc tctagttact ctcaaccttc ttgtcaaggt     960

ccaggaagaa atatgctaag gttgcccatt atcaacagca gagatcaatt agagagagtt    1020

tttgagggtt cgtcttcaga cgccaatgct gaaatagatg atgtatcata ctgtgcagag    1080

ctcatattgg gcaagcgacc tattagcaga agtcgaatag cgatgcatgg cgcctgccaa    1140

gggagcaatg tgaaaaagga tgcatcatct gtcactcatg tgcttaacca gaagcaaaat 1200

caaacatctc agaatagaga gaggggattt atgaagtcta agcaaagtag tttgcagagc 1260

aacagagttt tagcagctgc agaatctacg attaatacca aaagttttgt tgcacagaat 1320

agaaggttag gtgcatctac ccgattgaga atgccagcca cagccgatgg ctgcaagttt 1380

gaaactgaaa gaaaacctta ttcaaggaga agtgattccc tctctccggt gcggaagaag 1440

agattgatga atgtctccag acaaggtgaa agttctagtt ttgtgaatgc gaatttggga 1500

agagaatcaa gcccttattc tgacaaaaca agtagaaaag atgttttccc aatttcttcc 1560

gttaatagtc actccactaa accgaagttg ccatgtttgc gagagagtgg tgcaaccaat 1620

aatagttctg agggaagtaa tgttgtttcg tttactttta gatctgcaat gaagcagaaa 1680

gctggcattc atgcagaagt cacaaaaagg aagtctcaaa atagctcaag ttttgatgct 1740

actccaggga gatccttctt tactggaaat gatgaaacag catgcttgca gaaatccttt 1800

cctttgaaag gagacatttt aggagcactt ttggagcaaa agctaaagga attgactagt 1860

gaagaagagt tcgcagaagg agatgctgca cctagaaaaa gcactgcaac gatccttcag 1920

gagctaataa ctgctttgaa tgatgagacc caatttcatc tggatagttt gccttctaaa 1980

cctaacagaa aagaagattt gtatgatgat cgtgaagtgt cttcaagaaa tacttccatg 2040

aatttccagg ccataccaga ttcagcaacc gatttagttg ggaattcact tgataatgat 2100

caccttagtc caggatgtgt tcttgaagct accttttcaa ctgacagcta cctctccagc 2160

agtcccaata gtagctcaaa ggacaaagtg cttgccgaat ctgtggactc tatttatgac 2220

gaaccactct ttccagaacc tgatagagat ctttcggatt gtgcgacctc attattcacc 2280

aggaggagtt gcagagcact gatcacagac catgttaaca acatttctgg agtgctaagc 2340

aagattaatc agttaaaagg aagcaaactc ggttatgcaa atgaggtcat tttgaatact 2400

gaactcatac ttggaacctc acctgaacaa caggctttac ctgtagacga tgggttatca 2460

gtgagccatt ttctcctcaa tgaacttgaa atgctatcaa gtcttctatg gatgactttt 2520

ggtcagttgc ttggctgtaa cgaccccaaa cagatgaatc agcttaaagg atttgccttt 2580

gattgtctat tagagtatct agactcaaaa tttggtcgat actctgattc tggtttcaga 2640

atctggtcta aactaccatc aagcatgact aaagagattc tgattgctga tattattgaa 2700

gaggtcaaag agtggacgga atttgttggg ttgatccccg acgagctaat agaatgggat 2760

atgagccatt ctttgggtaa atggacggat tttgagattg aagaatttga atgtggcact 2820

gaagttgata ggcatattct ccaagtatta gtagatgaag ttgttttgga cctctacagt 2880

tctagttaa 2889

<210> 51

<211>  1668
<212>  DNA
<213>  Artificial Sequence

<220>
<223>  TAF-2 CDS

<400>  51
atggctggtg agaagattat agaaaaacct gaagctgttg ctccgggact gaaatctgat      60

ccctctaaca aactgattga gaaggatttg gtttcaaaaa aagatggaaa agctgctgac     120

tcggtggctt ctttgggagc tgtgaccagc atcaagtgtg aaaatgatca accacctgct     180

gcagagcaag gatataatgg gacttataat cagttagatg agcaggctta ctttaatgcg     240

ggtgttcaat cagacaatgg ttcccttctc tactatatgc ctggctataa tccttacagt     300

gctggatttg tgggaggcga tgggaagcag ccctatcctt cgtccggata tctccagcaa     360

cctgtctcat atggttcaga ttccatgcca tgttatacat ggggctcgcc atactgtgct     420

gatatcacca atagtgctgc tccaaaatcc gggaacgtta aatcaacttt tggacgaaat     480

ggttcagtca agtcgaatgg ttttaattcc acaaaaacaa atagttcttt ctcaagcaaa     540

aactcgaccg tactctttaa cccaaagagt cgaccagcta ctgccatgtc aaatcctcca     600

aagtctttcc atcaagctca gccttttaat ccagtgaaca agtttcagtc cgatgtccag     660

tctggaggcc tgatgaaggg gtttcatctg gttggggact acccatcata cacgagtcag     720

aatcaaggtt ttttcatgcc ttatgatccc atcaactgtc aaacaaacag tagaatgtgg     780

aatggaaact acagagctaa accacgggga aatttcacca gaaatggtgt gtttgaggca     840

actaatgagt tacctcgtgg tccccgtgcc aacggtagaa gtgtcccttc aaaaccatct     900

gctgaggagg atcagcttgt gccagctgtt cagagggaga agtataacaa ggaggatttt     960

aagactcagt atgacaatgc aaaattttac attatcaagt catacagtga agatgatatc    1020

cacaagtgtg tcaaatatga tgtgtggtca agtactccaa atggaaacaa gaaattggac    1080

actgcctttg ttgaatctga ggctaaagca agtggaactg gttcaagctg tccagtgttc    1140

ctcttctttt ctgtaaatgg aagtggacag tttttaggtg tagctgagat ggttgggcag    1200

gttgacttca acagaaacat ggacttttgg cagcttgata agtggagtgg gttcttccca    1260

ctgaagtggc acatagttaa agatgtacca aatacacagt tcaggcacat catccttgaa    1320

aacaatgata atagacctgt aacctatagc agagatacac aggagattgg attgaaggaa    1380

ggtttggaaa tgcttaacat acttaagaat tactcagaga agacatctat attggatgat    1440

ttcaatttct atgagaagcg tgagaaagtg ctcaaggcta aaaggagttc aaaaccagtt    1500

attcaagctg atgcatacga gaaagctgat tctcttaagc aattcaaagg aggagacaag    1560

gtactcgagg aggagctgaa gaccaactct actgatccaa ctgcaccttt ggtttctctc    1620

accaaaaacc tatcgattaa ttcaaggcca ttcaaaagta gtgtctga                1668

<210>  52
<211>  1416
<212>  DNA
<213>  Artificial Sequence

<220>
<223>  Adenylyl cyclase-associated CDS

<400>  52
atggatgaga agttgataca gcgattggag tccgccgtgt caaggctgga ggcgctttca      60

gtcggcggcg gtgtttcact tggagatgga gaagctgcgg cggctttaga tccatccatt      120

attgcatttg atgatctgag gtcacggttc gtcggtagag tttgcactgc tgcagagaag      180

attggtggac aggttttgga tgtcactaag attgttgagg aggcttttga agctcagaga      240

gaacttctta tcaaaatcaa ggaaactcag aaacctagca tgtctggttt ggctgaattt      300

cttaagcctc tgaatgatgt gatagtgaaa ggtactaaaa tgacagaagg acggcgatct      360

gatttgttca accacctgaa ggctgctgtt gatagtctaa cagccctggc atggatcgca      420

tacactggaa aagattgtgg tatgagtatg cctattgcac atgtggaaga aagctggcag      480

atggcagagt tttacaataa caaggtcctt gtggagttca gaaacaaaga cccagatcat      540

gttgaatggg caaaagcttt gaaagagatt tatctccctg gcttgcggga ttacgttaag      600

agtcactacc cattgggccc cgcgtggagt gctacaggga aagtagctgt atctgcacca      660

ccaaaagctc ccgcaccaag tcgtcccatg cctcctccac tgccaccagc ttctttattc      720

agctcggaat ctccccaggc ttcatcatca cgccccaaga aagggatgtc agctgttttt      780

gatgaaatta attcggggaa gccagtcacc ggtggattga gaaaggtgac agatgatatg      840

aagacaaaga atcgtgcaga tagaaccggt gtcattagtg caggagaaaa ggaacgtcgc      900

gttagctcac catctttctc caaagctgga cctccaaaac tggagcttca gatgggtcgt      960

aaatgggtgg ttgagaatca aataggaaga aagaatctag tcattgatga ttgtgatgca      1020

aagcaatctg tctatatttt tgggtgcaaa gattcagtcc tgcagatcca agggaaagtc      1080

aacaacataa caattgataa gtgtactaaa atgggagttg tattctcgga tgttgtggca      1140

gcttgtgagg ttgtcaactg taatggtgtg gaggttcaat gtcagggttc agcaccgact      1200

atttcagttg ataatacaac tggatgccaa ttatacttga gcaaagattc tttggggact      1260

tccattacaa cagccaagtc aagtgaaatt aatgttttgg ttcctggtgc aggggctgat      1320

agtgattggg gggagcacgc tttgccacag cagttttctc atgtatataa gagtggccaa      1380

tttgtgacta cacccgtctc acattcagga ggttaa      1416

<210>  53
<211>  1485
<212>  DNA

<213>  Artificial Sequence

<220>
<223>  Phosphoethanolamine N-methyltransferase CDS

<400>  53
atggctgcca ttgctccaga ggtaggagaa gagcgtgaga ttcaaaagag ttattggatg      60

gagcaaacta gtgaactcaa tttggaggca atgatgcgtg attctgcagc atctgatctt     120

gacaaagagg ataggcctga ggtgctttca ctgctccctt catatgaagg taaatccgtg     180

ttggaattgg gtgctggtat tggccgtttc actggggact tagccgagaa ggctggggag     240

cttgtagcag tggactttat tgaagaagtg actaagaaga atgaaagtat caacgggcac     300

cacaagaatg tcaagtttgt gtgtgctgat gtgacttcac cagatttgac ggttttacct     360

gaatcagtgg acttgatatt ttctaactgg ctactgatgt atctttccaa cgaagaggtt     420

caggaccttg cggagagaat gatcaaatgg ttgaaagttg gtggctatat attttttaga     480

gagacatgct tccatcaatc aggagaccac aagggaaaga acaacccaac ccactatcga     540

gagcctcgat tttatacaaa ggttttccaa gaatgtactt taaaggatga tgctggaaaa     600

tcatatgaac tttctcttat tggttataag tgcattgaaa cttatgtaaa aaacaaaaag     660

aatcaaaatg agatttgctg gatatggcag aaggttcatt ccgaggatga caggggattc     720

cagcatttct tggacactgt tcagtacaag tctaatggta tactgagata tgaacgtgtg     780

ttcggggcag gctttgtcag cacaggagga attgacacca ccaaagaatt cgttgccatg     840

atggatcttc agcctggcga aaaggtcctt gacgttggct gtggcattgg aggaggtgac     900

ttttacatgg ctgagaagta tgatgttcat gttgtaggca ttgatctttc agttaacatg     960

atcacattgg cttttgaacg tgctattggg cgcaaatgcg cggttgaatt tgaggttgct    1020

gattgcacgg tgaaaacata tcctgaagaa tcattcgatg tgatatacag ccgggacact    1080

atcctgcaca tccaggacaa gcctgcatta ttcagatctt tctataagtg gctgaagcct    1140

ggaggcagag tcctcataag cgattactgc aaaaaggcag gaccagcttc cgaagacttt    1200

gcagcgtata tcaaacaaag aggctacgat ttacatgatg ttgaagcata tggacagatg    1260

cttagagatg ctggtttcaa cgaagttatt gctgatgatc gaactgagca gttcatgcaa    1320

gttctccaga aagagttgga tactgtggag aatgaaagag agttatttat ccaagaattt    1380

tctgaacaag actacaatga tatagttgga ggttggaagg ccaagctagt tagaagttca    1440

tctggtgagc agagatgggg tttgttcttt gccaagaaaa attga                    1485


<210>  54
<211>  1527
<212>  DNA
<213>  Artificial Sequence

<220>

<223>  DnaJ domain-containing protein CDS

<400>  54
atggagtgta ataaagatga agccttcaga gcaaaaaaaa ttgcagagcg gaagtttgaa      60

cagaaggatt atgctggtgc aaagaaattt gccttgaaag ctcaggtttt gtatccaggg     120

cttgacgatt taacccagat gttaacaaca tttgatgttt acatctctgc cgaaaacaaa     180

gtaagtgggg aagtggattg gtatggggta cttggtgtga gcccgtcaag tgatgatgaa     240

acggtaaaga aacagtacag gaagctagct cttgtcctcc accctgataa aaataaatct     300

attggtgctg aaggtgcatt tcaacttctc tctgaggcct ggagcttgtt atctgataaa     360

tccaagaggt tggcatataa ccagagaagg agttcaaaag gtccccagca gaaacaacag     420

aaagttccag tgccctcagg tggtccatca actttctgga ccatttgcca ccgttgcaag     480

atgcattacg agtatttgaa gatatatctc aaccacactc ttctttgccc caactgtcat     540

gaatctacta gtcagtggaa aagagaacgg agattgaagg gagtaaagaa agtaccagat     600

cctgattttc acaatttcga tcaggatagg agcgaaagtt gttttgaaga caatgaagtc     660

tgggcttcat atgatgctga tgatggcatg ccccgtttct atgctctgat taacaaagtc     720

atctcaaggg aaccatttaa agtgaggctc agttggctta attcaaaaac gaacactgaa     780

tttggtccaa tggagtgggt agcttcaggt ttctataaaa cttctggtga gtttaggatt     840

ggcagatatg aaacgggtaa gtcggtgaat tccttctccc acaaggttcg atggtcaaag     900

ggccctcgtg gaactgttct aatatatccc caaaaggggg atgtttgggc cctcttcaga     960

aattggtctg ctgactggaa tcagaataca cctgatgatg ttatacacaa gtatgacatg    1020

gtcctagttc ttgatgatta caatgaggag caaggaattt cagtagctcc tctcatcaaa    1080

gttgctggtt ttaagacagt ctttcgtcca gacttgaatc cggagaagat gattagaata    1140

accagagaag agatgtttcg tttctctcat caggtcccaa gccacttgct tacaggtgaa    1200

gaaggtcaaa atgctccaaa gggttgccag gagcttgatc cagctgctac tccattggag    1260

cttcttcaga cagtaacaga aactaatgag gtgccagcca tgcagaacga caaggaggct    1320

aatggaggtt catcacagaa agttcaggaa acaaaaacta gtgaggctgc tgatcatact    1380

ctcaaatcta gggaaggcgg gatggtagaa agtgaaggag agattagtct tggttcagaa    1440

cagatggttg agaaggttac tatggagcaa acacaggtta atcctgtaaa gactgacaag    1500

gctactaagt ttggaactga gacgtag                                        1527

<210>  55
<211>  1242
<212>  DNA
<213>  Artificial Sequence

<220>
<223>  Elongation factor 1-gamma 1 (eEF1B) CDS

<400> 55
atggctctga ttttgcattc aacagacaac aacaaaaatg cctcaaaggc actcattgca 60

gctgagtaca caggtgttaa ggttgatctt gcaaaggact tccagatggg tgtatccaac 120

aagacacctg agtttcttga gatgaatccc attggaaagg ttcctgtgct tcaaacacct 180

gatggacctg tttttgagag caatgccatt gcgcgttatg ttaccaaaac aaagcccaac 240

aatcctcttt ttggctcttc attgattgaa tatgctcaaa ttgaacaatg gaatgatttt 300

tctgccactg aaattgatgc aaacattgca cgatggctgt acccacgcct tggctatgct 360

gtttacatct ctcaagctga ggaaggtgca gtagctgcat taaagagagc gcttggtgct 420

ttgaacaccc atcttgcatc taacacatac ttggttggac attttatcac actggctgac 480

atcatcatgg tctgtaactt gagcattggt tttaggatga tattgaccaa gagctttacc 540

aaggaatttc cacatgtaga gagatatttc tggactgttg ttaatcagcc taatttcgtc 600

aagatattgg gtgaggtgaa acaagctgaa tctatccctg ctgtgcaatc caagcctgca 660

caacctgaaa aactcaaggc taaggaggag ccaaagaagg aggttaagaa ggaagaacca 720

agtcctgtag aggaggaagc agcacccaag cctaaggcaa agaatcctct tgatttattg 780

cctccaagta agatgattct ggatgattgg aagaggcttt actccaacac caagactaac 840

ttccgtgagg ttgctgttaa aggtttctgg gacatgtatg accccgaagg atattctctc 900

tggttttgtg attacaagta caatgatgag aacacagttt cgtttgtaac cttgaacaag 960

gttggtggtt ttctgcagag gatggagctg gtacgcaagt atgcttttgg taagatgttg 1020

attgttggtt ctgaggcccc attcaaggtg cagggcttgt ggcttttccg tgggaaggaa 1080

attcccaagt ttgttatgga ggaagtgtat gacatggaac tctatgaatg gaaggaagta 1140

gacatcaatg atgaggccca gaaggagcga gttagccaaa tgattgaaga tcacgagcct 1200

tttgagggac aggctttgtt ggacgccaag tgcttcaagt aa 1242

<210> 56
<211> 5775
<212> DNA
<213> Artificial Sequence

<220>
<223> Tetratricopeptide repeat (TPR)-like superfamily CDS

<400> 56
atggcaccca acaagaatgg tcgtggcaaa acaaagggag acaagaagaa gaaagaagag 60

aaggttctcc cagttgtaat ggatataacg ataaaccttc cggaggagac tcaagttatt 120

ttaaagggaa tatctacgga tagaattatc gatgttcgtc gattattatc tgtcaataca 180

acaacttgta atgtcactaa tttctcactg tctcatgagt taaggggtcc acgtttaaaa 240

gaaacagtgg acgtttccgc actgaagccc tgcatcctga ctcttatcga agaggaatac 300

gatgaagaaa gcgcaacggc gcatgttaga aggctgttgg acatcgtcgc ttgtacaacg 360

agttttgggc cgtcggggac tagtggtaaa gagttgaaaa ctgactcttg caagaatgcg 420

cgaggtgtgc aggataacaa gaatgctaag aaatccaaca aggttcgtgg gaatgataag 480

tcatcgtcgc cgccacaaac gccaactccg gtggcgcaac agctgggtaa agatgcggga 540

tcggaagaag ttgatggaga gatgagcaat acttgcccta agattggaag cttctatgag 600

ttcttctcgc tttctcatct cacgcctcct cttcagtgta atgcgccctc tctctctcac 660

attgtgtctc tcttctcgtg tttcttgtta aaattaagac tcatcttgat tcttgcagtc 720

ataagaagag caacaagaca acaagatgat gaagttttgc cagatgatca tcttttctct 780

cttgaagtga aactttgtaa tggaaagctg gttattgttg aagcttgcaa gaaaggattt 840

tacaactttg gaaagcaggg gattctttgt cacaatcttg ttgatttgtt gagacaactc 900

agtagagcat ttgacaatgc atacgatgat ctcatgaaag cattcttgga gcgtaataag 960

tttgggaatc ttccatacgg attcagagcc aacacatggc ttataccacc tgtggcagca 1020

cagttgccag ctatttttcc acctctacct gtggaggatg atacctgggg agcaaatgga 1080

ggtggtctag gccgagatgg aaaatttgat tctttacctt ttgccaatga atttttgaat 1140

gttgcatcca tggcttgtaa gacaacagag gagaggcaga tcagagacag gaaggctttt 1200

attcttcata gtttatttgt tgatgtcgcc attttacgag ccatttcagc tgtaaagcat 1260

gtcatggaga aagttaaacc agctcattgt gatttgaatg gagaaatcat ttataatgag 1320

acagttgggg acttgagcat atttgttacc aaagattctt caaatgctag ctgcaaagta 1380

gataccaaaa ttgatggatt tcaagcaact ggaatagcta tgaagaatct gatggaaaga 1440

aatttactga aggggataac tgctgatgaa aatactgctg cccatgatat tgctacttta 1500

ggtgttctga atgtaagaca ttgtggttat attgcaactg taaaagttca aggaaaagaa 1560

aatgacaaag tgggcagccc actgcaaagc atggaacttg ctgatcagcc tgatggtggt 1620

gcaaatgccc tcaatatcaa cagtttacgc ttgctccttc acaagaaagt ggacaacaag 1680

gtaatgcatt caaaaccttc agaaactgaa gagcctaatt gctctcaggc atttgtaagg 1740

agaatactag aagagagtct taccaaactt gaagaagaga aaatagaagg tgactctttc 1800

atcagatggg aacttggtgc atgctggata cagcacttac aagatcagaa aaaatcagaa 1860

aaggacaaga aaccctccgc tgagaagaaa aaaaatgaga tgaaggttga gggacttgga 1920

atacctctta agtcccttaa gaacagaaag aagagcacag atggaactaa catggaatcc 1980

cagtcagaaa gcttcaaatc tgccgcagat ggtgttggag ggggatcaga aaaacctgtc 2040

ctgcagtctg gggagtctca gtttgagaca gatacagatc aaaatcaggt cgtcctcaag 2100

gcattgttgt ctgatgctgg ctttacaagg ttgaaggagt cagagactgg acttcacctt 2160

aagtctttgg aagagctgat tgatctgtca cagaagtact ataatgaagt tgccctgcca 2220

aagctggtgg ctgattttgg ctctttagaa ctctcaccag tagatggtcg aaccttaact 2280

gatttcatgc atacccgagg tctacgtatg cgttctcttg gacaagtagt taaactttct 2340

gagaagttat cacatgtgca atctctttgt atacacgaga tgatagtccg agcttttaaa 2400

catattcttc aagcagctat tgcatcagtt gttgacattg aagatatagc tgcgataatt 2460

gctgctgcct tgaatatgat gcttggggta cctgaaaatg atgattcaaa tgagtacggt 2520

gttgattctt tgatctggag atggctgaaa ttatttttga agaagagata cgaatgggat 2580

gttggcagcc tgaactacaa agatatgagg aaatttgcta tcctccgtgg tttatgccat 2640

aaggtgggaa ttgaactggt tccaagagat tatgatatga gttcagcaag tccttttcag 2700

aaagtagaca ttgtcagcct agtaccagtg cataagcaag ctgcttgctc ttctgcagac 2760

ggaagacagc ttttggaatc atccaaaaca gctctggata agggaaaact tgaagatgcg 2820

gtcagctatg ggactaaggc tcttgccaag ctggtcgcag tatgtggtcc ataccatcga 2880

atgacagctg gagcttatag ccttcttgct gttgttctgt atcacaccgg tgattttaat 2940

caggccacaa tctatcagca aaaggccttg gacataaatg aaagagagtt gggccttgat 3000

cacccagaca ctatgaaaag ttatggtgat cttgcagttt tctattaccg acttcaacac 3060

acagagttgg ctctcaagta tgtaaaacga gctctttatt tgttgcatct cacatgtggc 3120

ccctcccatc caaatactgc ggcgacatat ataaatgtgg ctatgatgga ggaagggctc 3180

ggtaatgtgc atgttgctct cagatatctc cataaagctt tgaagtgtaa ccaaaagtta 3240

ctcggtccag accatattca gacagctgca agttatcatg ctatagcaat tgccctctct 3300

ttgatggaag cttatccttt gagcgttcag catgagcaaa caaccttgca gatacttcga 3360

gcaaagcttg gtccagatga tcttcgcaca caggatgctg ctgcatggct tgagtatttt 3420

gagtcgaagg cttttgaaca gcaagaagct gctcgaaatg gaactaagaa gcctgatgca 3480

tcaatagcca gcaagggtca tttgagtgtg tcagatttgc tcgactacat taatccaagt 3540

ccagatgcca aagggagaga tgttggatca aaaagaagag gttttgtctc aaaggctctt 3600

atttctcagg tgaagggaaa atctgatcaa aacaatgttg ccataccaaa ctctgacact 3660

tttaaagatg ttccaaaaga agagacagat gaaaagaaac aaattgtcga agatcacact 3720

gatcccaaga tgaatatgga acctgttgac actgtaattg aatcccatca caatggagat 3780

ggaggaatta ctgagaacaa acccatccaa tctggaccat tgttaaagga aacttcaatt 3840

gagaagtcta tggtccgtga agtcttatct gaaccttctg ctgaagcaga agatggatgg 3900

cagccagtgc agagaccaag gtcaggtggt ttctatggac gaagacgaag gcagaggcgt 3960

caaaccatca gcaaggttat tggttaccag aaaaaggacc caatttctga tgttgatcat 4020

gctaaattga agaataacta tcaagctagt aaatattatg tcttaaagaa acggacatca 4080

ccaggaagct atgcagatta ttacttagca aaaagtcagg cttctggtac caaacttggt 4140

cgaagagtca taaaagctgt agcctaccgt gtgaagtctg tgtcatcttc tgtcagagat 4200

gctgttcctg agatctccac aactggagga gatttgttaa atacttcatc agagcaggtc 4260

caagtttctg caacaaagga ggttggatca ctatcaaaga gaagttcaat agtaaaccta 4320

ggaaaatctc cttcctataa ggaagtagca cttgccccgc caggtaccat ttctatgttg 4380

caggagagag tttctgaaga tgaaattcct gataacccag acgttatgaa acttgaaaag 4440

gagagcaatg gagcagaaga aaattccaaa ataatgggaa gagatgcaga atccatggag 4500

aaagagaaca ttcaggatct agttgcaaat tcttctgatc atgtaaaaag tgaaacagta 4560

gatactgaca gtaaagaaga aattcaaatg agtgatctca aaggtggtga aatttcagat 4620

ctgatatctg caaatgcatc tattcaaccc ggccatgttg atgttagtcc aatggaacag 4680

ggtagtgtca agactcataa tgtccctact tctgacaatt ctcccaaagc ggatccctgt 4740

gaaaaggact catcaagcaa tttaaatcct ggcgttatct caaatatgac cttgcaagat 4800

atggatcatc tgaaggtaaa atctgcgtca tctcatgcaa gtgatgccag tcgagaattg 4860

tccagaaagc tatctgcatc agcagcacca ttcagccctt ccccagctgt tccccgcggt 4920

accccattac ctatgaacat taatctccct tctcctcctg gaacacgacc accgattggt 4980

ccttggtcag tgaccatgtc tcttcatcaa ggaccaccga ccatcttgcc tagtccaatg 5040

tgctcctccc ctcatcacct gtacccttca cctccacaca ccccaaacat gatgcaccca 5100

ttgcgcttta tctaccctcc gtattctcaa ccccagacgt tacctccaaa tacattcccc 5160

atgagtagca gcactttcca tccgaatcat tatgcttggc aatgcaatat agctcctaat 5220

gcatcagagt atgttcctgc cacagtttgg cctggctgcc acccagtcga gttttctatc 5280

tcaccacctg tgattgagcc tataactgac tcaatttcct ctgctaagga gatatctgat 5340

aatcctgaaa atattacttt gacaacaagc ttactagttg atctcaacac cggggatgaa 5400

gtcaaggaag atgtaaatct tccagcatca gaaacagtgg agaacatagc tgcagttgta 5460

ccagaaaaag agagagcaag caacactcca gattcacatt ttgttacctc gtctagtgat 5520

caatcaaaag agggcagtgg atcaaatcat gtgcaaagaa atcttacgga gacggataat 5580

gagaagacct tcaacatttt ggtacggggc cggaggaatc gcaaacaaac tctaagaatg 5640

cctataagtt tgctcaagag accatattct tctcagccct tcaaagctgt atatagcagg 5700

gtaataaggg agactgaggt tcccagctct accagctttg atccacatga acatggcata 5760

acgactgcta cttga 5775

<210> 57
<211> 753
<212> DNA

<213>  Artificial Sequence

<220>
<223>  XRI1-like CDS

<400>  57
atgggtgacc ttcactcaat tgcttacaat agcttctctt ccctaggttg tgacttccat      60

ggcctagaag ttctcaacaa tgatatgtct ccatttatgg agagcactcc accatttttg      120

tcaaattttg attgtgagtt gtcaagtgga tatctacaag atgctttatt caaattcaac      180

tcaaaaagaa ggcgtttatt tttgtttaat gatgatgatg acaaggagaa ttatcaaaat      240

aaagattcaa ttaagaattt gtggagctcc acaattgacc aacaattttc tgaggattat      300

gattctttta gccagataac caagtgtgat agcttttctg gggatccaat gagcaaaatg      360

agtgaagaat attccaaaag aacagaggag gaagcaatct cagaaaatta ttattattct      420

tctaattctt caccaacatc atcatctcat cataataaac aacctctaca atatggagga      480

ggtgataaaa agagaagaat gtgtggaaaa attgtgtatc catttgggct agtgaagccc      540

ggagggcaag aaggagacgt gacattaaat gacattaacg agaggattct aatgaggccg      600

acccggcccg taaggcatcc ggttggggac tttgcatgtc gttcgaccgt atgccctgct      660

ggaccaggat tatccggcaa gacggtggtt gcactcacca aaattcatac tcaagggagg      720

gggacaatca caattataag gactcgaggg tga                                  753

<210>  58
<211>  2910
<212>  DNA
<213>  Artificial Sequence

<220>
<223>  DnaA initiator-associating dia A CDS

<400>  58
atgaatgtta gttcagctag agaaatgaat gatagtttag ccataacagc atcatcattg      60

gccattactg agaaaaaacc tcaaaggcct ggtggttgtg tgggcatttt ctttcagctc      120

tttgattgga accgtagatt tgccaagaag aagttgtttc caaagaaact gctttcacca      180

gatcgcttga aacaagcttc aaaaaagttt ggaggggatg agaagcagcc aaagattcgt      240

ttgattgcta atgagaacag tgggggtttt ccaaatgtaa agaacaatgg aatgaccaat      300

acatgttgtg aaagcaagcg caaaatgaaa gctccaagtt tggttgctag gctcatgggt      360

ttggaatcga tgccagcagc atcaggcagt aagcccaaaa aggcttcagg ttctgaaatt      420

gggagcaatg tggcagagaa actcggtggt caacctggcg gatctgataa agaagatatg      480

gatttcgaga aggccgaaat aaagcgtgaa ttgaggcctc aaaagcttca gaagataggg      540

cttagcgaga gacgttcagt ttgcagattt agtgctgaag cattgcagtt gaggactgta      600

ttgtcaaggc caaggaaaca tcagcctaaa ttggtttctc cagtaaaaag tcctagaaat      660

gtctctggta gaaatgcatc taggttgatt ggcgctgcaa ctaggatttt agaaccgggt 720

ttgcaaaaga gtagagtgaa atgtgctctc acttatccta ctagatattt ttcccctttg 780

gaagataaag cagatttagc acctcatcat ttggaaggcc ccaatccttg tgtggattca 840

aaaactttga aaggaacatc tgtgccttca tgtaaaaatt gtggttattt tcttcatagt 900

aagaacggta taccaaatgt ggaagaacac tcatcgtctg tctcgtcacc tatctctagt 960

tactctggac ctccttgtca cggtccagga agaaatattc caaagttgcc cattttcagt 1020

agcagggatc aactagagag agtttctgag ggttcgtcct cagacgccac tgctgaaata 1080

gatgatgtat catactgtgc agaactcata ttgggcaaaa gatctattag cagaagtcga 1140

atggagatgc atggcacccg ccaagggaac aatgtgaaga aggatgcatc acctgtcact 1200

catgtgctta atcagaaaca aaatcaaaca tctcagaata gagagagggg ccttatgaag 1260

tctaagccaa gtagtttgca gagcaacaga attttagcag ctgcagaatc tatgaataat 1320

accaaaaatt ttgttgcaca gaacagaagg ctaggtgcat ctacccgatt gaggatgcca 1380

gccacagccg atggctgcaa gtttgaaacg gaaagaaaac cttattcgag tagaagtgat 1440

tccttctctc cggtgcggaa gaagagattg aataatgtct ctagacaagg agacagttct 1500

agttttgtga atgcgaatat gggaggagaa tcaagtccct cttctgacaa aacaagtaga 1560

aaagatgttg cttttccaat ttgttctgtt gatagtcact ccactaaacc aaagttgcca 1620

tgtttgcgag agggtgttac aaccaataat agttctgagg gatgtaacgt tgtttcattt 1680

acatttaaat ctgcaatgaa gcagaaagct ggcactcatg cagaagtcac aaaaaggaag 1740

tctcagaatt actcaagtgt tgatgctact cgggggagat ccttctttaa aggaaatgat 1800

gaagcaccat gtttgcataa atcctttcct ttgaaagggg acattttagg agcacttctg 1860

gagcaaaagc taaaggaatt gactagtgaa gaagagttcg gagaaggggg cgctgcacct 1920

agaaaaagca ctgcaacgat ccttcaggag ctaataacgg ctttaaatga agagaaacaa 1980

tttcatctgg acagttcgcc cgttagacct agcaggaaag aagatttgtg tgatgatggt 2040

gatgtatctt ctagaaatac ttgcatgaat ttccaggcca taccagattc agcagcaaca 2100

gatttagttg ggaattcact tgatcaccat agtccgggat gtgttcttga agctgccttt 2160

tcaactgaca gctacctctc cagcagtccc aatagtagct caaaggacaa agtgcttgct 2220

ggatcagtgg actctattga tgatgaacca ctctttccag aacctgatag agatctttcg 2280

gattgtgaga cctcattatc caccaggagt tgcagagcac tgatcactga ccattttaac 2340

aacgtttctg gattgctaag caagattgat cagttaaaag gaagcaaact cagttatgca 2400

aaagaggtca ttttgaatac tgaactcata tttagaacta cacctcaaca gcaggctcta 2460

cctgtagagg atgggttctc tgtgagccat tttctcctca atgaacttga aatgctatcg 2520

agtcttctgt ggatgacttt cagtcagctg cttggctgta acgacccaaa acaaatgaat 2580

cagcttaaag gattcgcctt tgattgtcta ttagagtacc tagactcaaa atttgctcga 2640

tactctgatt ctggtttcag aacctggacc aaactaccat caagtatgac caaagagatt 2700

ctgattgctg atattattga agaggtcaaa gagtggacag aatttgttgg tttgatccca 2760

gacgagctaa tagaatggga tatgagccat tctttgggta aatggaccga tttcgagatt 2820

gaagaatttg aatgtggcac tgaagttgct aggcatattc tccaagaatt agtagatgaa 2880

gttgttttag acctctacag ttttagctaa 2910

<210> 59
<211> 1710
<212> DNA
<213> Artificial Sequence

<220>
<223> TAF-2 CDS

<400> 59
atggctggag agaagattat agaaaaatct gaagcggttg ctcctgggct gaaatctgat 60

tcctctacca aactgaccga gaaggatttg gtttcaaaaa atgatggaaa agtatctgac 120

tcggtgtctt ctttgggagc tgtgactggc atcaagggtg aaattgatca accaccagtt 180

gcagagcaag gtgcttatta tccacctact agctattgcg attactacta cccaggatat 240

aatgggacct ataatcagtt agatgatcaa gcatacttta attcgggcgt tcagtcagaa 300

aatggttccc ttctttatta catgcctggt tataatcctt atggtgctgg atatatcgga 360

ggtgacggaa agcagcccta tctatcgtct ggatatcttc agcaacctgt ctcatatggt 420

tcggattcca tgccatgtta ttcatggggt ggttcgacgt actgtgcaga tatcaccaat 480

agtgctgctc ccaaatccgg caatgtcaaa tcaacttttg gacgaaatgg ttcggtgaga 540

tcaaacggtt ttaatccaac aaaaacgaat ggttctttct cgagcaaaaa ctcgaccatg 600

ctttttaacc caaagaatcg accgtctact gccatatcaa atccaccaaa gtctatccat 660

caagctcagc cttttaatcc tgcgaacaag tctgatgtcc agtccggagg cctgacaaag 720

gggtatcata tggttgggga cttcccatca tacagcagtc aaaatcaagg gtttttcatg 780

ccttatgatc ccattcacta tcaaacaaac agtagattgt ggaatgggaa ctataggggt 840

aaaccacggg gaaatttcac cagaaatggt gtgtttgagg catcgaatga gttacctcgt 900

ggtccccgtg ccaacagcag gagcgcccct tcaaaaccat ctgctgagga ggatcagctt 960

gagccaacaa ttcagaggga gaagtataac aaggaggatt ttaagactca gtatgataat 1020

gcaaagtttt acattatcaa gtcgtacagt gaagatgata ttcacaagtg tgtcaaatat 1080

gctgtatggt caagtactcc taatggaaac aagaaattgg acactgcgtt ccttgatgct 1140

gagggtaaag caagtggaac tgattcaagc tgtccagtgt tcctcttctt ttcagtaaat 1200

ggaagtggac agtttttagg tgtagctgag atggttgggc aggttgactt caacagaaac 1260

atggactttt ggcagcttga taagtggagt gggttcttcc cagtgaagtg gcacatagtt 1320

aaagatgtcc ctaacacaca gttcaggcac atcatcctca aaaacaatga taatagacct 1380

gtaacctata gcagagatac tcaggagatt ggtttgaagg aaggtttgga aatgcttaac 1440

atacttaaga actactcgga gaagacatct atattggatg atttcaattt ctatgagaag 1500

cgtgagaaag tgctcaaggc taaaaggagt tcaaaaccag ctattcaagc tgatgtattt 1560

gagaaagctg attctgttag gcaattcaaa ggaggagata aggtacgcga agaggagcta 1620

aagagcaact cgactgatcc aactgcatct ctcgtttctc ttgccaaaaa tctatcgatt 1680

aattcgaggc cattcaaaag tagtgtctga 1710

<210> 60
<211> 1428
<212> DNA
<213> Artificial Sequence

<220>
<223> Adenylyl cyclase-associated CDS

<400> 60
atggaggaga aattggtaca gcgattggaa tcggcggtgg cgcggctgga ggcgttatca 60

gccaccggct tccgttccgg cggttccgtc gtggcaggtg gcgatggtac ggcggtttta 120

gatccgtcga ttattgcatt tgacgatcta cggtcgcagt ttctttcgaa ggttttgagt 180

gctgcagaga agatcggagg acaagttttg gatgttagta agattgttga ggaggctttt 240

gaagctcaga gagaacttct cattaaaatc aaggagacta agaaaccaga caactcaggt 300

ttggttgaat ttctcaaacc attgaatgat gtgatcgtga aagctacaaa aatgactgaa 360

ggacgccggt ctgatttctt caaccacctg aaatcggctg ttgatagtct atcggctcta 420

gcatggattg cctacattgg aaaagactgc ggaatgagca tgcctattgc attcgtggaa 480

gaaagttggc agatggctga attttataat aacaagattc ttgtggagtt taaaaacaaa 540

gacccaaatc atgttgaatg ggcaaaagct ttgaaagaac tttacctccc tggattgaga 600

gattatgtta agagtcacta tccattaggt cctgtgtgga gcgctacagg gaaacctgct 660

gtatctgcac cttcgaaagc tccttcacca agtgctcctg ctcctccagt tccccctcca 720

gcttctctct tcagctctga atctcctcag gcttcatcat cgcgccccaa gcaagggatg 780

tctgctgtct ttgatgaaat taattcggga aagccagtga cttctggatt gagaaaagta 840

acagatgaca tgaagacaaa gaaccgtttc gacagaaccg gcgttgttaa tgctggtgat 900

aaggaagctc gcatgagctc accccctgtc tctaaaactg gacctccaaa attggagctt 960

cagatgggcc gtaaatgggt ggttgagaat caagttggac aaaagaacct agttattgat 1020

gattgcgata caaagcaatc agtatatgtc tatgggtgca aagattccgt tctgcagatc 1080

aaaggaaaag tcaataacct cacaattgac aaatgcacta agatgggaat tgtattcgcg 1140

ggtgttgtgg cagcttgtga ggttgtcaat tgcaacagtg tagaggtgca atgtcagggc 1200

tcggctccta caatatcaat cgacaataca aatggatgcc agttatactt gaacaaagat 1260

tctttggagg gttctattac cacagctaag tcaagtgaaa tcaatgtttt ggttcctggt 1320

gcagggcctg atgatgattg gggtgagcat gctttgccac agcagtatgc tcatgaatac 1380

aaggacggac actttgtgac tactcctgtc tcccactctg gagcttaa 1428

<210> 61
<211> 1419
<212> DNA
<213> Artificial Sequence

<220>
<223> Adenylyl cyclase-associated CDS

<400> 61
atggatgaga aattgataca gagattggag tccgccgtaa tacggctgga ggcgctgtca 60

gtcggtggcg gagcttcagt tggagatggt gtagctgcgg cagctttaga tccatctatt 120

attgcctttg acgatctgag gtcacagttc gtaggcagag tccttggtgc tgcagagaag 180

attggtggac aagttttgga cgtgactaag attgttgaag aggcttttga agctcagaga 240

gaacttctta tcaaaatcaa ggaaactcag aaacctagca tgtctggttt gcctgaattt 300

ctgaaacctc tgaatgatgt gattctgaaa gctacaaaaa tgacagaagg atggcgactt 360

gatttcttca accacttgaa gtctgctgtt gatagtctaa tagccctggc atggattgct 420

tacactggaa aagattgtgg tatgagtatg cctattgcac acgtggaaga aagttggcag 480

atggctgaat tttacaataa caaggtcctt gtggagttca gaaacaaaga cccaaatcat 540

attgaatggg caaaagcttt gaaagagctt tatctccctg gcttgtggga ttatgttaag 600

agtcactatc cactgggccc tgtatggagt gctacaggta aagttgctgt atctgcacca 660

tcaaaagctc ctttgccaac aagtcgtccc atgccgcctc ccctgccgcc agcttctcta 720

ttcagctctg gatctttcca ggcttcatca tcacgcccga agaaagggat ggcagctgtt 780

tttgatgaaa ttaattcggg gaagccagtt accaccggat taagaaaggt gaccgatgat 840

atgaagacaa agaaccgagc agacagaacc ggtgtcatta gtgccggaga aaaggaaggt 900

cgccttagct caccctcttt ctctaaagct ggacctccaa aactggagct tcagatgggc 960

cgtaaatgga tggttgagaa tcaaatagga agaaagaatc tagtcattga tgattgcgat 1020

gcaaaacaat ctgtctatat ttttgggtgc aaagattcag ttctgcagat ccaaggaaaa 1080

gtcaacaaca taacaattga taagtgcact aaaatgggag ttgtattcac ggatgtcgtg 1140

gcagcttgtg aggttgtcaa ctgcaatggt gtggagatgc aatgtcaggg ttcagcacca 1200

acaatttcag ttgataatac aactggatgt caattatact tgggcaaaga ttcttcggag 1260

acttccatta caacagctaa gtcaagtgaa atcaatgttt tggttcctgg tgcagggcct 1320

gatagtgatt ggggtgagca cgctttgccg cagcagtttt ctcatgtata taagaatggc 1380

cagtttgtga ctacatctgt ctcccattca ggaggttaa 1419

<210> 62
<211> 1470
<212> DNA
<213> Artificial Sequence

<220>
<223> PMT CDS

<400> 62
atggctccag gaataggaca agagcgtgag attcaaaaga gttattggat cgagaaaact 60

gctggactta aattggaggc aatgatgcat gtgcatgatc ttgaatcatc tgatcttgat 120

aaagaggata ggcatgaggt gctttcactg ctcccgtcat atgaagggaa atccgtgttg 180

gaattgggtg ctggtattgg tcgtttcaca ggagacttag ccaagaaggc tggggagctc 240

gtagcagtgg attttattga aggagtgatt aaggagaatg aaagcatcag cgggcaccac 300

aagaatgtca agtttaagtg tgctgatgtg acttcaccag atttgacgtt ttcacctgaa 360

tctgtggact tgatattctc taactggcta ctgatgtatc tttctgatga agaggtgcag 420

gaccttgcgg agagaatgat gacatggttg aaagttggtg gatatatatt ttttagagag 480

acatgcttcc atcagtccgg agacaacaag ggaaagaaca acccaactca ctatcgggaa 540

cctagattct atacaaaggt tttccaagaa tgtaatgctg gaaaatcata tgaactttct 600

ctcgtcggtt ataagtgcat ggaaacttat gtaagaaaca aaaggaatca aaatgagatt 660

tgctggatat ggcagaaggt tcgttctgat gatgacaggg gattccagca tttcttggac 720

actgttcagt acaagtctaa tggcatactg cgatatgaac gtgtctttgg gaaagacttt 780

gtgagcacag gtggaatcga taccaccaaa gaattcgttg ccatgttgga tcttcagcct 840

ggcgaaaaag tccttgatgt tggctgtggc atcggtggtg gtgactttta tatggctgag 900

aagtatgatg ttcatgttct cggcattgat ctctcagtta acatgatctc attgtctttt 960

gaacgtgcta ttggtcgcaa atgcgcggtt gaatttgagg ttgctgattg cacagtgaaa 1020

acatatcctg aaggatcatt cgacgtgata tacagccggg acactatcct gcacatccag 1080

gataaaccag cattattcca atctttctac aagtggctga agcccggagg cagagtcctc 1140

ataagtgact attgcaaaaa ggcagggcca gcatcagaag aatttgcagc gtacatcaaa 1200

caaagaggtt acgatttaca tgatgttgaa gcatatggac agatgcttag agatgctggt 1260

ttcaatgaag ttattgctga ggatcgaact gagttgttca tgaaagttct ccagaaagag 1320

ttggatattg tggagaagga aagagagtca tttatccagg agttttctga acaagactat 1380

aatgatatag ttggaggttg gaaggccaag ctagtgagga gttcatctgg tgaacagaga 1440

tggggtttat tcattgccaa gaaaaattga 1470

<210> 63
<211> 1476
<212> DNA
<213> Artificial Sequence

<220>
<223> PMT CDS

<400> 63
atggatgttg cttcaggaca agagcgtgag gttcagaaga gttattggat tgagcatact 60

gctgaactta ctgttgaggc catgatgctc gactctaaag cagccgatct agacaaagag 120

gagaggcctg aggtgctctc tctgcttccg gcatatgaag ggaaaactgt gttggaactg 180

ggtgctggta ttggccgttt cactggtgaa ttagccaaga aagctggaca gcttatagca 240

ttggacttta ttgaaggcgc aattaagaag aatgaaagca taaatgggtg ccataagaat 300

gtcaagttca tgtgtgctga tgtgacttct ccagatttga atttttcacc ggaatcggtg 360

gacttgatat tttctaattg gctgctgatg tatctttctg atgaggaggt tcaggatctt 420

gctaagagaa tggttaaatg gttgaaagtc ggtggttaca tatttttcag agagtcgtgc 480

ttccatcaat caggagacca taagcgaaag aacaacccaa cccactatcg agatcctagc 540

ttttatacaa aggttttcag agaatgtcat gtaaatgctg gtgatggaaa atcatttgaa 600

ctttctcttg gcggttgcaa gtgcatcggt gcttatgtga ggaacaaaaa gaatcaaaat 660

cagatttgct ggtcgtggca gaaggttggt tctgagaatg acatggaatt tcagcgcttc 720

ttggatactg ttcagtacaa gtgtagtggc atactacgct atgagcgagt ctttggacaa 780

ggttttgtga gcacaggagg acttgaaact accaaagaat ttgtagccat gttggatctt 840

cagcctggcc agaaagtcct tgatgttggc tgtggcattg gaggaggtga cttctacatg 900

gcagaaaagt acgatgttca cgtcgttggc attgacctgt ctatcaacat gatatcattt 960

gctcttgagc gtgctattgg tctcaagtgt gctgttgaat ttgaggttgc tgattgcacc 1020

aagaaaacat atcctgttgg tacatttgat gtcatataca gtcgggatac tatccttcac 1080

atccaggaca aacctgcact cttcagatct ttctacaagt ggctgaaacc aggaggcaaa 1140

gtccttataa gtgattactg caaaagagcc ggaccagcat cagaagaatt tgcaggttac 1200

attaagcaaa ggggatatga tttacatgat gttgaagcat atgggcagat gctcagagat 1260

gcaggttttc atgatgttgt ggccgaggat cgtactgaac agttcattgg ggttctgcaa 1320

aaggagttgg atactgtgga gaagggaaga gagtcattta tccatgagtt ctctgaacaa 1380

gactataatg aaatagttgg aggttggaaa gccaagctaa tcaggagttc atctggtgag 1440

cagaggtggg gcttgttcgt tgccaagaag aactga                                1476

<210>  64
<211>  816
<212>  DNA
<213>  Artificial Sequence

<220>
<223>  PMT CDS

<400>  64
atgctatgct ggctatggca gaaagttaat tctgaggatg acaggggatt ccagcgtttc      60

ctggacaacg cgcaatacaa gtgtagtggc atactgcgat atgaacgtgt ctttggagaa     120

ggttatgtga gcacaggagg aattgatacc acaaaagaat ttgtctccat gttggatctt     180

caacctggcc aaaaagtcct tgatgttggc tgtggaatag gtggaggtga cttttacatg     240

gctgagaatt atgatgttca tgttgttccc attgacctct caattaacat gatatctttc     300

gctcttgagc gtgcaattgg tctcaaatgt gccgtcaaat ttgaggttgc tgattgtaca     360

aagaaaacgt atcctgatgg cacatttgat gtgatataca gtcgagacac tatccttcat     420

atccaagaca aacttgcgtt attcagatct ttctacaggt ggctgaaacc aggaggcaaa     480

gtcctcataa gcgactactg caaaagacca ggacctgcat cggaagattt ttcaggctat     540

attaagcgaa ggggttatga tttacattat gttgaagcat atggcctgat gctcagagag     600

gcgggtttcg atgaagttat tgccaaggat cgaactgaac agttcattaa tgttctccgg     660

aaggagttgt atactgtgga gaaggaacga gattcatttt tccaggaatt ctcagaacaa     720

ggctacaatg aaatagttgg aggttggaac gccaagctat tgaggagttc atctggtgaa     780

cagaggtggg gattgttcat tgccaagaag aagtga                                816

<210>  65
<211>  2193
<212>  DNA
<213>  Artificial Sequence

<220>
<223>  DnaJ domain-containing CDS

<400>  65
atggagtgca ataaagacga ggccctcaga gcaaaaataa ttgcagagcg gaagtttgaa      60

caaaaggatt atgctggtgc aaagaaattt gccttgaaag ctcaggcttt gtatccgggg     120

cttgacgctc tagcccagat gttaacaaca cttgatatct acatcgctgc tgaaaataaa     180

ataagtgggg aagtggattg gtatggggta cttggtgtga gcccgtcaac tgatgatgaa     240

acagtgagga aacagtacag gaagctagct ctcattctcc accccgataa aaacaaatct     300

gttggtgctg aaggtgcatt taaacttctc tctgaggcct ggagcttgtt gagtgataaa     360

tccaagaggt tggcatataa ccagaggagg agttcaaaag gattccagca gaaagctcca 420

gtacactcgg gtggtccgtc agcacctcca cgaaatgggt ttcataattt tactagtaag 480

acttcgaaga ctcagaaaaa tgcttctcgg gtgccctctt catcagttaa ttcttcagcc 540

aaccaaagaa gtgatacttt ctggaccatt tgccaccgat gcaagatgca ttatgagtat 600

ttgaaaattt atcttaacca cactcttctg tgccccaact gtcatgaagc ttttatggcc 660

acagaaacag ccccaccgtt caatcatgca aaatcatcca attctacgcg tcagtggcat 720

cagaatgctg ggaatcatgc acctaatgga aatcaattcc caataggaag aaatgctagt 780

gctggaaaaa catcaggatc agcaccagca agttctaata ctgcgaatta ttcgaacttt 840

cagcaggact cattttccag aatggctggt gttggtagct cggatccttc cattgcagca 900

aaagctgcaa atgttgttca gcaggcacat gagaaaatga agagagaacg tgatgattca 960

cagactagac agcctcctct taaacagaga aagcttgatg acaatggaat ccgttatgga 1020

tccaatatac ctcactatgc agaaagggct tatggtttct cttctagcaa gtttaacagt 1080

acaagggagc tgacaccact tgaaaaccgg aatatgctga tgggaaaagc tcggaaagag 1140

atactcagta agttgaatga atggagatcg cagccgcaag atgcacaaaa aaataaggtg 1200

aaagagggca aaaaagaaaa gcagagggct gctaatgttc ttggccaaaa tgtgaatggg 1260

aatgtcgagt catcagctat gaagggggta aagaaagatg ttaatgcttc agctaatgat 1320

gcaaaacaag aggatctact gccacaatca atgaatgtac cagatcctga ttttcacaat 1380

tttgaccagg ataggagcga aagttgtttc ggagacaatg aagtctgggc tgcatatgat 1440

gctgatgatg ggatgccccg tttctacgct ctgattagca aagtcgtctc aagggagcca 1500

tttaaagtga ggcttagttg gcttaattca aagacaaaca gtgaatttgg tccaatggaa 1560

tgggtagctt caggtttcta caagactagt ggtgaattta ggattggcag atacgaaacg 1620

agtaagtctg tgaattcctt ctcccacaag gttcggtggt caaagggccc tcgtggatct 1680

gttctcatat ttcccatgaa gggggaggtt tgggccctct acagacattg gtctcctgac 1740

tggaatcaga atacacctga tgatgtcata cacaagtatg acatggtcct agttcttgat 1800

gactacaatg aggagcaagg aatttcagta gctcctctcg tcaaagttgc tggttttaaa 1860

acggtctttc gtccagattt gaattcggag aaggtgatga gaatcaccag agaagagatg 1920

tttcgtttct ctcatcgggt cccaagccac ttgcttacag gtgaagaagc tcaaaatgct 1980

ccaaagggtt gccaggagct tgatccagct gctaccccat tggagcttct tcagacatta 2040

ccagaaacta atggggtgtc agccatgcag aatgacaaag attttaatgg actttcacca 2100

cagaatgttc aggagactaa aactagagag actgctgatc atactctcaa ctccagtgaa 2160

gacgggatgg taaacggtga agaagctcca tga 2193

<210>  66
<211>  1245
<212>  DNA
<213>  Artificial Sequence

<220>
<223>  Elongation factor 1-gamma 1 (eEF1B)

<400>  66
atggctttga ttttacattc aacaagtaac aacaaaaatg ccgcaaaggg actcattgcg      60

gctgagtaca caggtgtgaa agttgaactt gcaaaggatt tccagatggg tgtgtcaaac     120

aagacacctg aatttctcga gatgaatcca attggaaagg ttcctgtgct tcaaacacct     180

gatggacctg tttttgagag caatgctatt gcacgttatg ttaccaaaac gaagcccgat     240

aatcttcttt ttggctcttc attgattgaa tatgctcaaa ttgaacaatg gaatgatttt     300

tctgccactg aagttgatgc aaacattgca cgttggctgt acccacgcct tggctatgcc     360

gtatacattc ctccagctga ggaagctgcg gtagctgcat taaagagagc tcttggtgct     420

ttgaacaccc atctcgcatc caacacatac ttggttggac attcaatcac attggctgac     480

attattatgg tctgcaactt gagcattggt tttggggtga taatgaccaa gagctttacc     540

aaagaattcc cacatgtaga gagatacttc tggactgtgg ttaatcagcc aaatttctgc     600

aagatattgg gtgaggttaa acaagctgaa tctatcccag ctccgccatc caagaagccc     660

gcaccggcga cagaaaagcc caaggctaag gaggagccaa agaaagaggt taaggaagaa     720

cctaagcctg cagaggagga agcagcaccc aagcctaagc caaagaatcc tcttgatctc     780

ttgcctccaa gtccgatgat tctggatgat tggaagaggc tttactccaa caccaagacc     840

aacttccgtg aggttgctgt taaaggtttc tgggacatgt atgaccctga aggatattct     900

ctctggtttt gcgattacaa gtacaatgat gagaacacag tttcgtttgt aaccttgaac     960

aaggttggtg gttttctgca aaggatggat ctggtgcgca agtatgcttt tggtaagatg    1020

ttgattgttg gttctgagcc cccattcaag gtgcaaggct tgtggctttt ccgtggaaag    1080

gaaattccca agttcgttat ggatgaagtc tacgacatgg agctctatga atggaagcaa    1140

gtagacatca acgatgaagc ccagaaggaa cgtgttagcc aaatgattga agaccatgag    1200

ccttttgagg gacaggctct gttggatgca aagtgcttca agtaa                    1245

<210>  67
<211>  5724
<212>  DNA
<213>  Artificial Sequence

<220>
<223>  TPR-like superfamily CDS

<400>  67
   atggcaccaa gcaagaatgg tcgtggcaaa actaagggag acaagaagaa gaaagaagag      60
   aaggttcttc ccgttgtaat ggatataacg ataaaccttc cggatgagac tcaagttatt 120
   ttaaagggaa tatctacgga tagaattatt gacgttcgtc gattactatc tgtgaacaca 180
   acaacttgta atattaccaa tttctcactg tctcatgagt taaggggtcc acgtttaaaa 240
   gaaacagtgg acgtttccgc actgaagccc tgcctcctga ctctcataga agaggactac 300
   gatgaagaaa gcgccacggc gcatgttata aggctgctgg atatcgttgc atgtacgacg 360
   agctttgggc cgtcggggac taatggcaag gacttgaaaa ctgagtctag taagaatgcg 420
   cggggtgcgc aggatagtaa gggcgccaaa aaatcaagca aggttcaggc aaatgataag 480
   tcatcgtcgc cgccacaaac gccaactccg gcggcggcgt ctcaacaggc gggtaaagat 540
   gcagcgtcag tggatgttga tggagagatg agcaatacgt gccctaagat tggaagcttc 600
   tatgagttct tctcgctttc tcatctcact cctccgcttc agttcataag aagagcgaca 660
   agacagcaag atggtgaagt tttgccagat gatcatcttt tcactcttga agtgaaactt 720
   tgtaatggaa agctggttat agttgaagct tgcaagaaag gattttataa ctttgggaag 780
   caggggattc tttgtcacaa tcttgttgat ttgttgagac aagtcagtag agcatttgac 840
   aatgcttacg atgagctcat gaaagcattc tcagagcgta ataagtttgg gaatcttccc 900
   tacggattca gagccaacac atggcttata ccacctgtgg cagcacaatt gccagctatc 960
   tttccacctc tacctgtgga ggatgaaaac tggggagcaa atggaggtgg gcttggccga 1020
   gatggcaaat ttgatttgtt accttatgcc aatgaatttt tgaatgttgc atccatgcct 1080
   tgtaagacaa cagaggagag gcagatacga gacaggaagg cttttcttct tcatagttta 1140
   tttgttgatg tcgccatttt acgagccatt tcagctgtaa agcatgtcat ggagaaagtt 1200
   aaaccagctc attgtaattc gaatggagaa atcattttga ctgagtcagt tggggatttg 1260
   agcatatttg ttaccaaaga tgcttcaaat gctagcagca aagtagatac caaaattgat 1320
   ggatttcaag caactggaat agctatgaag aatctgatgg aaagaaattt gctgaagggg 1380
   ataactgctg atgaaaatac tgctgcccat gatgttgcta ctttaggtgt tttgaatgta 1440
   aggcattgtg gttatattgc aactgtaaaa gttcaaggaa aagaaaatga caaagtgggc 1500
   agcccactgc aaagcatgga acttcctgat cagcctgatg gtggtgcaaa tgccctcaat 1560
   atcaacagtt tgcgtttact ccttcacaag aaagtggtca acaaggtaat gcattcaaaa 1620
   ccttcagatt ctgaagagac aaactgctct caggcatttg taaagagaat actagaagag 1680
   agtcttacca aacttgaaga agagaaaata gaaggtgact ctttcatcag atgggaactt 1740
   ggtgcatgct ggatacaaca cttacaagat cagaagaaat cagaaaagga caaaaagaac 1800
   cctgttgaga agacaaaaaa tgagatgaag gttgagggac ttggaatacc actcaagtcc 1860
   cttaagaaca gaaagaagag ctcagatggt gctaacacgg aatcccagtc tgacaccttc 1920
   aaatctatcg cagatggtgt tcaaagggga tcagaaaaaa ctgtcctgca gtctggggag 1980
   tcgcagtttg agactgatac ggatcaaaat caggtcgtcc tcaaggcatt gttgtctgat 2040
   gctggtttta ctaggttgaa agagtcagag actggacttc accttaagtc tttggaagag 2100
   ctgattgttc tgtcacagaa gtattacaac gaagttgccc tgccaaagct ggtggctgat 2160
   tttggctctt tggaactctc accggtagat ggtcgaacct taactgattt catgcataca 2220
   cggggtctac gtatgcgttc tcttggacaa gtagttaaac tttctgagaa gttgtcacat 2280
   gtgcaatctc tttgtataca tgagatgata gttcgagctt ttaaacatat tctgcaagca 2340
   gttattgcat cggttgttga tattgaggat atggctgcaa taattgctgc tgctttgaat 2400
   atgatgcttg ggtacctgaa aaatgatgat gcaaatgagt taggtgttga ttctttgatc 2460
   tggatatggc tggaattatt tttgaagaag cgatatgaat gggatgttgg cagcctaaac 2520
   tacaaagatg tgaggaaatt tgcgatcctc cgtggtttat gccataaggt gggaattgaa 2580
   ctggttccaa gagattatga tatgagttcc ccaagtcctt ttcagaaagt agacattgtc 2640
   agcctagtac cagtgcataa gcaagctgct tgctcttctg cagacggaag gcagcttttg 2700
   gagtcgtcca aaacagctct ggataaggga aaacttgaag atgctgtcag ttatgggact 2760
   aaggctcttg ccaagctggt cgcagtatgt ggtccatatc atcgaatgac tgctggagct 2820
   tatagtcttc ttgctgttgt tctgtatcac accggtgatt ttaatcaggc cacaatttat 2880
   cagcagaagg ccttggacat aaatgagaga gagttgggcc tcgatcaccc agacacaatg 2940
   aaaagttatg gtgatcttgc agttttctat taccgacttc aacacacaga gttggctctc 3000
   aagtatgtaa aacgagctct ttatttgttg catctcacat gtggcccctc gcatccaaac 3060
   actgcagcaa catatataaa tgtggctatg atggaggaag ggctcggtaa tgtgcatgtt 3120
   gccctcagat atctccataa agctttgaag tgtaaccaaa agttacttgg tccagaccat 3180
   attcagacag ctgcaagtta tcatgctata gcaattgcac tctctttgat ggaagcttat 3240
   cctttgagtg ttcagcatga gcaaacaacc ttgcagatac ttcgagcaaa gcttggtcca 3300
   gatgatcttc gcacacagga tgctgctgca tggctcgagt attttgagtc aaaggcgttt 3360
   gaacaacaag aagctgctcg aaatggaact aagaagcctg atgcatcaat agctagcaag 3420
   ggccatttga gtgtgtcaga tttgctcgac tacatcaatc caagtcctga tgccaaaggg 3480
   agagatgttg gatcaaaaag aaaaggtttt gtctcaaagg ctcttttttc tcaggtgaag 3540
   ggaaaatctg agcaaagcaa tgtttccata ccaaactctg acactcctaa agatgtcccc 3600
   aaagaagaaa cagatgagaa gaaacaactt atcgaagatc atacagatcc caagatgcat 3660
   atggaacccg ttgacacagt aattgaatcc aatcataatg gagatggaag aattgctgag 3720
   aacaaaccca tccaatctgg acccttgctg aaggaaactt taattgagaa gtctacagtc 3780
   catgaagtgt tatctgaacc ttctgctgaa gcagaagatg gatggcagcc agtgcagaga 3840
   ccaaggtcag gtggtttcta tggacgaaga cgaaggcaga ggcgtcaaac catcagcaaa 3900
   gtcattggtt accagaaaaa ggacccagtt tctgatgttg atcatgctaa attgaagaat 3960
   aactatcaag ctagtaaata ttatgtctta aagaaacgga catcaccagg aagttatgca 4020
   gattattaca tagcaaaaag tcaaactcct ggtaccaaac ttggtcgaag agtcataaaa 4080
   gctgtagcct accgcgtaaa gtctgtatca ccttctgtca gagatgctgt tcctgagatt 4140
   tccacaactg gaggagattt aactacttca tcagagcagg tccaagtttc tgtaacaaaa 4200
   gaggttggat cagtagcaaa gagaagttct atagtaaacc ttggaaaatc tccttcctat 4260
   aaagaagtag cacttgcccc gccaggtacc atctctatgt tgcaggagag agtttctgaa 4320
   ggtgaaattc ctgataatca agacattaca aaacttgaag aggagagcaa cggagcagaa 4380
   acaaattcca aaacaatgaa aagtgatgca gaatccgtgg agaaagagaa cattcaggat 4440
   cttgttgcag attctgctga tcatgttaaa agtgaaacag tagctactga caaaaaagaa 4500
   gaaattcaaa tgagtgatct tgacggtggt gaaatttcag atgtgatatc tgcaaataca 4560
   tctattcaac ccggccatgt tgatgttagt ccagtggaac aggttagtgt cgagactcat 4620
   aatgtccctc cttctgacaa ttctcccaaa gtggatccct gtgaaaagga ctcatcaagc 4680
   catttaaatc caacctgtat ctcgaatatg accttgcaag atatggatca tctgaaggta 4740
   aaatctgcat catctcatac aagtgatgca agtcgagaat tgtctagaaa gctatccgca 4800
   tcagcaacac cattcagccc ttccccagcc gttccccgcg tagcaccatt acctatgaac 4860
   attaatctcc cttctcctcc tggaatacga ccacctgttg gcccttggcc agtgaacatg 4920
   tctcttcatc aaggaccacc gaccatcttg cccaatccaa tgtgctcctc ccctcaccac 4980
   ctgtaccctt cacctccaca caccccaaac atgatgcacc cattgcgctt tatctaccct 5040
   ccgtattctc aaccccagac gatacctcca agtactttcc ccatgaatag cagcactttc 5100
   catccgaatc attatgcttg gcaatgcaac atagctccta atgcatcaga gtatgttcct 5160
   gccacagttt ggcctggctg ccacccagtc gagttttcta tctcaccacc tgtaattgag 5220
   tctataactg actcaatatc tgctgctaag gagctatctg acaatcctga aagcattagt 5280
   ttgacaacaa gcttaccagt agatctgaac accggagatg aagtcaaaga agatgaaaat 5340
   cttccagtat cggaaatggt ggagagctta gctgctgttg gatcagaaaa agagagagca 5400
   agcaacactt cagattcaca ttttgttacc ttctctagtg atcaatcaaa agagggaagt 5460
   ggatcaaatg agaaggctgg aagttgcagt gacaattatg tgcaaagaaa tcttgtggag 5520
   acggatgatg agaagacctt caacattttg gtacggggcc ggaggaaccg caaacaaact 5580
   ctaagaatgc caataagttt gctcaagaga ccatattctt ctcagccctt caaagctgta 5640
   tatagtaggg ttataaggga gactgaggtt cccaggtcta ccagctttga tccacatgaa 5700
   cattgcataa cgactgctac ttga 5724

<210> 68
<211> 789
<212> DNA
<213> Artificial Sequence

<220>
<223> XRI1-like CDS

<400> 68
atgggtgacc ttcactcaat tgcttacaac aacaataatc tccatagctt atcttcccta 60

ggttgtgact tccatggcct tgaagttttc aacaatgaca tatctccatt tatggagagc 120

actccaccat ttttatcaaa tcttgattgt gacttatcaa gtggatatct acaagatgca 180

ttattcaaat tcaactcaaa aagaagacgt ttattattat ttgatgatga tgatgaagag 240

aaggagaatt atcaaattaa tgattcaaat aattccatta agaatttgtg gagttccaca 300

atggaccaac aattttctga ggattatgat tgttttagcc aaataaccaa gtgtgatagc 360

ttttctggtg atcccatgag caaaatgagt gaagaatatt caaaggagga agcaatatca 420

gaaaattatt attattattc atcttcacca acatcatcat ctcatcataa taatattaat 480

caaaaacaat taccccaaca atatggagga gttgatcaaa agagaagcaa gaaaaggatg 540

gttggaaaaa ttgtgtgtcc atttgggcta gttaagccag gagggcaaga aggtgatgtg 600

acattaaatg acatcaatga gaggattttg atgaggccaa cccggcccgt taggcatccg 660

gtcggggact ttgcatgtcg cccgaccgta tcaccagccg gaccagggtt gtccggtaag 720

aaagtagtcg cgctcactaa aattcatact caagggagag ggacaatcac gattataagg 780

actcgaggg 789


<210> 69
<211> 801
<212> DNA
<213> Artificial Sequence

<220>
<223> ToBRFV movement protein CDS

<400> 69
atggctcttg ttaagggtaa agtcaatatt aatgagttca tagacttgtc aaaatcagaa 60

aaatttcttc cgtctatgtt cacacctgtt aagagtgtca tgatctccaa ggttgataag 120

atattggttc atgaagatga atctttgtcc gaagtcaatt tactcaaagg tgtaaaactc 180

attgatggtg gctatgtaca tcttgctggt cttgtggtga caggtgaatg gaatttgcca 240

gataattgtc gtggtggtgt cagtgtctgt ttggtcgata agagaatgga gagagcggac 300

gaggcaactc ttgcttcata ctataccgca gcggctaaga aaaggtttca gttcaaagtc 360

gttccaaatt acaacatcac taccaaggac gcagaaaagg cagtttggca agtactagtt 420

aatattagaa atgttaaaat tgctgcgggt tactgtccgc tgtcattaga atttgtgtca 480

gtgtgtattg tttataaaaa tattataaaa ctcggtttga gagagaaaat tacgagcgtc 540

acggatggag ggcccatgga actatcagaa gaagttgttg atgagttcat ggaagaagtc 600

ccgatgtctg taaggcttgc aaaatttcgt tcgaagaccg gaaaaaagtt tagtagtaaa 660

agtgagaata atagtggtaa taataggccg aaaccagaca aaaaccaaag gaaggaaaag 720

ggtttaaaag ttagggttga gaaggataat ttaattgata atgaattgga gacttacgtc 780

gccgattcag attcgtatta a 801